# EUROPÄISCHE PATENTSCHRIFT

(11) **EP 3 864 015 B1**
(45) Veröffentlichungstag und Bekanntmachung des Hinweises auf die Patenterteilung: **10.05.2023**
(21) Anmeldenummer: 19780272.1
(22) Anmeldetag: 09.10.2019
(51) Int. Cl.: C07D 471/04, C07D 487/04

(54) **VERFAHREN ZUR HERSTELLUNG VON IMIDAZOLDERIVATEN**
PROCESS FOR THE PREPARATION OF IMIDAZOLE DERIVATEIVES
PROCÉDÉ DE PRÉPARATION DE DÉRIVÉS D'IMIDAZOLES

(30) Priorität: 11.10.2018 EP 18199885
(43) Veröffentlichungstag der Anmeldung: 18.08.2021
(73) Patentinhaber: Bayer Aktiengesellschaft, 51373 Leverkusen (DE)
(72) Erfinder: WILLOT, Matthieu, 40215 Düsseldorf (DE); FISCHER, Rüdiger, 50259 Pulheim (DE); HAGER, Dominik, 40789 Monheim (DE); HOFFMEISTER, Laura, 40593 Düsseldorf (DE); MOSRIN, Marc, 40789 Monheim am Rhein (DE); WILCKE, David, 40237 Düsseldorf (DE)
(74) Vertreter: BIP Patents
(86) Internationale Anmeldenummer: PCT/EP2019/077304
(87) Internationale Veröffentlichungsnummer: WO 2020/074558

(56) Entgegenhaltungen:
- WO-A1-2018/033448
- WO-A1-2018/130437
- WO-A1-2018/130443
- MARC MOSRIN ET AL: "TMPZnCl?LiCl: A New Active Selective Base for the Directed Zincation of Sensitive Aromatics and Heteroaromatics", ORGANIC LETTERS, Bd. 11, Nr. 8, 16. April 2009 (2009-04-16) , Seiten 1837-1840, XP055019579, ISSN: 1523-7060, DOI: 10.1021/ol900342a
- CHRISTOS I. STATHAKIS ET AL: "TMPZnOPiv-LiCl: A New Base for the Preparation of Air-Stable Solid Zinc Pivalates of Sensitive Aromatics and Heteroaromatics", ORGANIC LETTERS, Bd. 15, Nr. 6, 15. März 2013 (2013-03-15), Seiten 1302-1305, XP055522219, US ISSN: 1523-7060, DOI: 10.1021/ol400242r
- MARK L. HLAVINKA ET AL: "Zn(tmp) 2 : A Versatile Base for the Selective Functionalization of C-H Bonds", ORGANOMETALLICS, Bd. 26, Nr. 17, 1. August 2007 (2007-08-01), Seiten 4105-4108, XP055522203, US ISSN: 0276-7333, DOI: 10.1021/om700475t

## Beschreibung

Die vorliegende Erfindung betrifft ein Verfahren zur Herstellung von Imidazolderivaten der Formel (II) 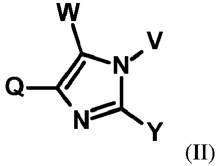 ausgehend von Verbindungen Q-H über Zwischenstufen der Formel (IIIa) oder (IIIb) 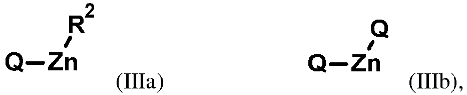 in denen die in den Formeln (II), (IIIa) und (IIIb) angegebenen Strukturelemente die nachstehend angegebenen Bedeutungen haben. Ferner betrifft die Erfindung derartige Imidazolderivate und Zwischenstufen.

Imidazolderivate der Formel (II) haben große technische Bedeutung für die pharmazeutische und die agrochemische Industrie und sind eine wichtige Zwischenstufe unter anderem bei der Herstellung von Verbindungen, die beispielsweise als Pestizide wirksam sind.

In der Literatur ist bekannt, dass Verbindungen der Formel (II) beispielsweise in einem ersten Schritt durch Kondensation von Imidazol-4-carbonsäurederivaten mit ortho-substituierten Bis(amin)-, Aminalkohol- oder Aminthiol-(hetero)arylderivaten in Gegenwart einer Säure (vgl. WO 2007/042544 oder Tetrahedron Letters 2012, 53, 5691-5694) hergestellt werden können. In der Literatur ist weiterhin bekannt, dass Verbindungen der Formel (II) in einer Cyclisierungsreaktion aus Imidazolcarbonsäure-Derivaten mit ortho-Diaminen oder ortho-Aminoalkoholen erhalten werden können sowie durch eine Cyclisierungsreaktion von α-Halogenketonen mit aromatischen Aminen (vgl. WO 2018/130443 oder WO 2018/130437). Die bisher im Stand der Technik beschriebenen chemischen Syntheseverfahren von derartigen Imidazolderivaten bedienen sich aber sehr häufig Methoden, die aus industrieller Sicht wirtschaftlich nicht zu realisieren sind und/oder andere Nachteile aufweisen.

Nachteilig sind die geringen chemischen Ausbeuten, die Durchführung bei sehr hohen Temperaturen (ca. 150 °C bis 250 °C) sowie die mögliche schwierige Regio- und Chemo-Selektivität der Kondensation, insbesondere bei Imidazopyridin- und Imidazopyridazinderivaten. Die Herstellung ist daher sehr teuer und nicht für großtechnische kommerzielle Verfahren geeignet. Außerdem sind entsprechende Verbindungen kaum kommerziell erhältlich. Dies gilt insbesondere für 5-Alkylsulfanyl-1H-imidazol-4-carbonsäurederivate.

Im Hinblick auf die vorstehend geschilderten Nachteile besteht dringend Bedarf für ein vereinfachtes, technisch und ökonomisch durchführbares Verfahren zur Herstellung von Imidazolderivaten, insbesondere von Imidazolderivaten der Formel (II). Die mit diesem angestrebten Verfahren erhältlichen Imidazolderivate sollen dabei vorzugsweise mit guter Ausbeute, hoher Reinheit und in ökonomischer Weise erhalten werden.

In der Literatur ist bekannt, dass halogenierte Pyridinderivate unter Verwendung einer Zinkmetallorganischen Base dargestellt werden können (vgl. WO 2018/033448).

Überraschenderweise wurde gefunden, dass in einem Verfahren unter Verwendung einer Zink-metallorganischen Base Imidazolderivate der Formel (II) vorteilhaft hergestellt werden können.

Gegenstand der vorliegenden Erfindung ist demnach ein Verfahren zur Herstellung von Verbindungen der Formel (II) 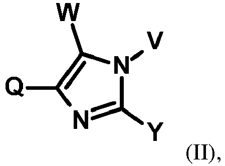
in welcher (Ausgestaltung 1)
   - Q: für ein Strukturelement 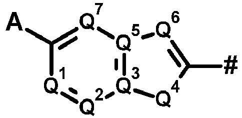
steht, wobei das Zeichen # die Bindung zum Rest des Moleküls anzeigt und
   - Q¹: für N oder CR⁶ steht,
   - Q²: für N oder CR⁶ steht,
   - Q³: für N oder C steht,
   - Q⁴: für O, S, N, CR⁶ oder NR⁷ steht,
   - Q⁵: für N oder C steht,
   - Q⁶: für N oder CH steht und
   - Q⁷: für N oder CH steht,
wobei maximal fünf der Variablen Q¹, Q², Q³, Q⁴, Q⁵, Q⁶ und Q⁷ gleichzeitig für Stickstoff stehen und Q³ und Q⁵ nicht gleichzeitig für N stehen und
   - R⁶: für Wasserstoff, (C₁-C₄)Alkyl, (C₁-C₄)Halogenalkyl, (C₁-C₄)Cyanoalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
   - R⁷: für (C₁-C₄)Alkyl, (C₁-C₄)Halogenalkyl, (C₁-C₄)Cyanoalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht und
   - A: für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonyl steht, oder
   - A: für -O-CF₂-O- steht und gemeinsam mit Q¹ und dem Kohlenstoffatom, an das es gebunden ist, einen fünfgliedrigen Ring bildet, wobei Q¹ für Kohlenstoff steht,
   - W: für Halogen oder S(O)ₙR⁸ steht, wobei
   - R⁸: für (C₁-C₆)Alkyl, (C₁-C₆)Cyanoalkyl, (C₁-C₆)Alkoxy(C₁-C₆)alkyl, (C₁-C₆)Halogenalkyl, (C₂-C₆)Alkenyl, (C₂-C₆)Halogenalkenyl, (C₂-C₆)Alkinyl, (C₂-C₆)Halogenalkinyl oder (C₃-C₈)Cycloalkyl steht und
   - n: für 0, 1 oder 2 steht,
   - V: für (C₁-C₆)-Alkyl, (C₁-C₆)Halogenalkyl, (C₁-C₆)Cyanoalkyl, (C₁-C₆)Alkoxy-(C₁-C₆)alkyl, (C₁-C₆)Halogenalkoxy-(C₁-C₆)alkyl, (C₂-C₆)Alkenyl, (C₂-C₆)Alkenyloxy-(C₁-C₆)alkyl, (C₂-C₆)Halogenalkenyloxy-(C₁-C₆)alkyl, (C₂-C₆)Halogenalkenyl, (C₂-C₆)Cyanoalkenyl, (C₂-C₆)Alkinyl, (C₂-C₆)Alkinyloxy-(C₁-C₆)alkyl, (C₂-C₆)Halogenalkinyloxy-(C₁-C₆)alkyl, (C₂-C₆)Halogenalkinyl, (C₂-C₆)Cyanoalkinyl, (C₃-C₈)Cycloalkyl, (C₃-C₈)Cycloalkyl-(C₃-C₈)Cycloalkyl, (C₁-C₆)Alkyl-(C₃-C₈)Cycloalkyl, Halogen(C₃-C₈)cycloalkyl, Cyano(C₃-C₈)cycloalkyl, (C₁-C₆)Alkylthio-(C₁-C₆)alkyl, (C₁-C₆)Halogenalkylthio-(C₁-C₆)alkyl, (C₁-C₆)Alkylsulfinyl-(C₁-C₆)alkyl, (C₁-C₆)Halogenalkylsulfinyl-(C₁-C₆)alkyl, (C₁-C₆)Alkylsulfonyl-(C₁-C₆)alkyl, (C₁-C₆)Halogenalkylsulfonyl-(C₁-C₆)alkyl, (C₁-C₆)Alkylcarbonyl-(C₁-C₆)alkyl, (C₁-C₆)Halogenalkylcarbonyl-(C₁-C₆)alkyl, (C₁-C₆)Alkoxycarbonyl-(C₁-C₆)alkyl oder (C₁-C₆)Halogenalkoxycarbonyl-(C₁-C₆)alkyl steht und
   - Y: für Wasserstoff, Halogen, (C₁-C₄)Alkyl, (C₁-C₄)Halogenalkyl, (C₁-C₄)Alkoxy, (C₁-C₄)Halogenalkoxy, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, SCN, (C₁-C₄)Alkylcarbonyl, (C₁-C₄)Halogenalkylcarbonyl, (C₁-C₄)Alkoxycarbonyl, (C₁-C₄)Halogenalkoxycarbonyl, Aminocarbonyl, (C₁-C₄)Alkylaminocarbonyl, Di-(C₁-C₄)alkyl-aminocarbonyl, (C₁-C₄)Halogenalkylaminocarbonyl, (C₃-C₆)Cycloalkylaminocarbonyl, Aminothiocarbonyl, (C₁-C₄)Alkylaminothiocarbonyl, Di-(C₁-C₄)alkylaminothiocarbonyl, (C₁-C₄)Halogenalkylaminothiocarbonyl, (C₃-C₆)Cycloalkylaminothiocarbonyl, Amino, (C₁-C₄)Alkylamino, (C₁-C₄)Halogenalkylamino, Di-(C₁-C₄)Alkylamino, (C₃-C₆)Cycloalkylamino, (C₁-C₄)Alkylsulfonylamino, (C₁-C₄)Alkylcarbonylamino, (C₁-C₄)Halogenalkylcarbonylamino, (C₁-C₄)Alkylcarbonyl-(C₁-C₄)alkyl-amino, (C₁-C₄)Halogenalkylcarbonyl-(C₁-C₄)alkyl-amino, (C₃-C₆)Cycloalkylcarbonylamino, (C₃-C₆)Cycloalkylcarbonyl-(C₁-C₄)alkyl-amino, (C₁-C₄)Alkylthiocarbonylamino, (C₁-C₄)Halogenalkylthiocarbonylamino, (C₁-C₄)Alkylthiocarbonyl-(C₁-C₄)alkyl-amino, (C₁-C₄)Halogenalkylthiocarbonyl-(C₁-C₄)alkyl-amino, (C₃-C₆)Cycloalkylthiocarbonylamino, (C₃-C₆)Cycloalkylthiocarbonyl-(C₁-C₄)alkyl-amino, (C₂-C₄)Alkenyl, (C₂-C₄)Halogenalkenyl, (C₂-C₄)Cyanoalkenyl, (C₃-C₆)Cycloalkyl-(C₂)alkenyl, (C₂-C₄)Alkinyl oder (C₂-C₄)Halogenalkinyl steht,
   oder für jeweils gegebenenfalls einfach oder mehrfach, gleich oder verschieden substituiertes (C₃-C₆)Cycloalkyl oder (C₅-C₆)Cycloalkenyl steht, wobei als Substituenten jeweils in Frage kommen: (C₃-C₆)Cycloalkyl, (C₁-C₄)Alkyl, (C₁-C₄)Halogenalkyl, (C₁-C₄)Alkoxy, (C₁-C₄)Halogenalkoxy, Aminocarbonyl, Aminothiocarbonyl, Halogen oder Cyano,
   oder für jeweils gegebenenfalls einfach oder mehrfach, gleich oder verschieden substituiertes Aryl oder Hetaryl steht, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, SF₅, Tri-(C₁-C₄)alkylsilyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Halogenalkyl-(C₃-C₆)cycloalkyl, Halogen-(C₃-C₆)cycloalkyl, Cyano-(C₃-C₆)cycloalkyl, (C₁-C₄)Alkyl, (C₁-C₄)Halogenalkyl, (C₁-C₄)Cyanoalkyl, (C₁-C₄)Hydroxyalkyl, Hydroxycarbonyl-(C₁-C₄)-alkoxy, (C₁-C₄)Alkoxycarbonyl-(C₁-C₄)alkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₃-C₆)Cycloalkyl-(C₂)alkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxycarbonyl-(C₁-C₄)alkoxy, (C₁-C₄)Alkoxy-(C₁-C₄)alkoxy, (C₁-C₄)Alkoxyimino, (C₁-C₄)Halogenalkoxyimino, (C₁-C₄)Alkylthio, (C₁-C₄)Halogenalkylthio, (C₁-C₄)Alkoxy-(C₁-C₄)alkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkoxy-(C₁-C₄)alkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkoxy-(C₁-C₄)alkylsulfonyl, (C₁-C₄)Alkylsulfonyl-(C₁-C₄)alkyl, (C₁-C₄)Alkylsulfonyloxy, (C₁-C₄)Halogenalkylsulfonyloxy, (C₁-C₄)Alkylcarbonyl, (C₁-C₄)Halogenalkylcarbonyl, (C₁-C₄)Alkylcarbonyloxy, (C₁-C₄)Alkoxycarbonyl, (C₁-C₄)Halogenalkoxycarbonyl, Aminocarbonyl, (C₁-C₄)Alkylaminocarbonyl, (C₁-C₄)Halogenalkylaminocarbonyl, Di-(C₁-C₄)alkyl-aminocarbonyl, (C₂-C₄)Alkenylaminocarbonyl, Di-(C₂-C₄)-alkenylaminocarbonyl, (C₃-C₆)Cycloalkylaminocarbonyl, (C₁-C₄)Alkylsulfonylamino, (C₁-C₄)Alkylamino, Di-(C₁-C₄)Alkylamino, (C₁-C₄)Halogenalkylamino, (C₃-C₆)Cycloalkylamino, Aminosulfonyl, (C₁-C₄)Alkylaminosulfonyl, Di-(C₁-C₄)alkylaminosulfonyl, (C₁-C₄)Alkylsulfoximino, Aminothiocarbonyl, (C₁-C₄)Alkylaminothiocarbonyl, Di-(C₁-C₄)alkylaminothiocarbonyl, (C₁-C₄)Halogenalkylaminothiocarbonyl, (C₃-C₆)Cycloalkylaminothiocarbonyl, (C₁-C₄)Alkylcarbonylamino, (C₁-C₄)Halogenalkylcarbonylamino, (C₁-C₄)Alkylcarbonyl-(C₁-C₄)alkyl-amino, (C₁-C₄)Halogenalkylcarbonyl-(C₁-C₄)alkyl-amino, (C₃-C₆)Cycloalkylcarbonylamino, (C₃-C₆)Cycloalkylcarbonyl-(C₁-C₄)alkyl-amino, (C₁-C₄)Alkylthiocarbonylamino, (C₁-C₄)Halogenalkylthiocarbonylamino, (C₁-C₄)Alkylthiocarbonyl-(C₁-C₄)alkyl-amino, (C₁-C₄)Halogenalkylthiocarbonyl-(C₁-C₄)alkyl-amino, (C₃-C₆)Cycloalkylthiocarbonylamino, (C₃-C₆)Cycloalkylthiocarbonyl-(C₁-C₄)alkyl-amino, Hetaryl, Oxo-Hetaryl, Halogen-Hetaryl, Halogen-Oxo-Hetaryl, Cyano-Hetaryl, Cyano-Oxo-Hetaryl, (C₁-C₄)Halogenalkyl-Hetaryl oder (C₁-C₄)Halogenalkyl-Oxo-Hetaryl,
dadurch gekennzeichnet, dass in einem ersten Verfahrensschritt a) eine Verbindung Q-H, in welcher Q die oben genannte Bedeutung hat,
mit einer Zink-metallorganischen Base der Struktur (NR³R⁴)-Zn-R² oder (NR³R⁴)₂-Zn, in welcher
R² für Halogen oder -O-Pivaloyl steht und
R³ und R⁴ gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R⁵-Radikale substituiert sein kann und R⁵ ausgewählt ist aus der Gruppe bestehend aus Methyl, Ethyl, n-Propyl und i-Propyl,
zu einer Verbindung der Formel (IIIa) oder der Formel (IIIb) umgesetzt wird, 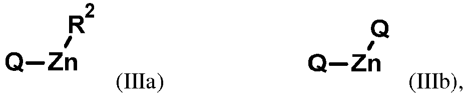
in welcher Q und R² jeweils die oben genannten Bedeutungen haben,
und diese Verbindung der Formel (IIIa) oder (IIIb) in einem zweiten Verfahrensschritt b) mit einer Verbindung der Formel (I) 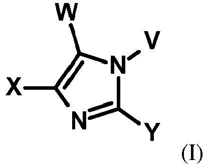
in welcher X für Halogen steht und V, W und Y jeweils die oben genannten Bedeutungen haben, in Gegenwart eines Katalysators zur Verbindung der Formel (II) umgesetzt wird.

Vorzugsweise stehen maximal vier der Variablen Q¹, Q², Q³, Q⁴, Q⁵, Q⁶ und Q⁷ gleichzeitig für Stickstoff. Unter Stickstoff werden hierbei N und/oder NR⁷ verstanden.

Bevorzugte und besonders bevorzugte Bedeutungen der in den vorstehend erwähnten Formeln (I), (II) (IIIa) und (IIIb) des erfindungsgemäßen Verfahrens aufgeführten Reste Q, V, W, R¹, R², X, und Y werden im Folgenden erläutert, wobei die Zink-metallorganische Base weiter unten noch genau beschrieben wird, so dass die bevorzugten Ausgestaltungen der Base dort angegeben sind.

### (Ausgestaltung 2)

- Q: steht bevorzugt für ein Strukturelement aus der Reihe Q1 bis Q14, 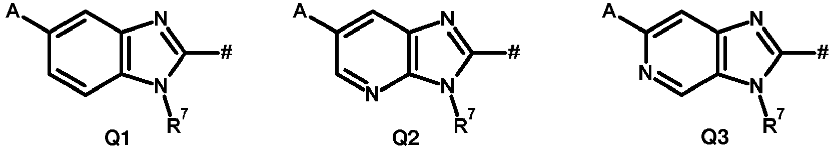 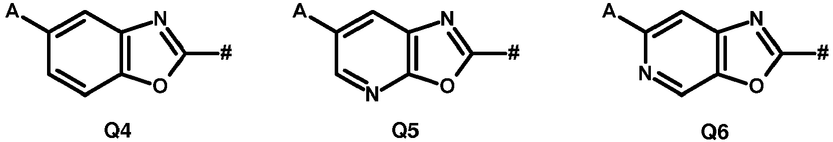 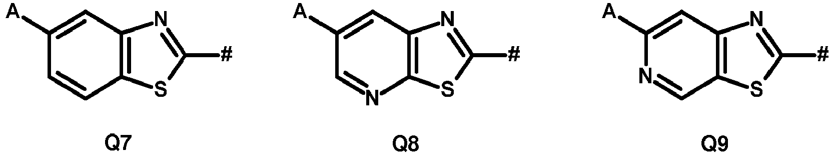 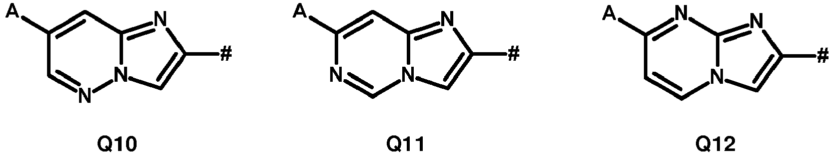 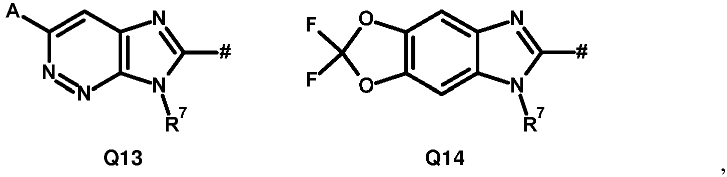 wobei
- R⁷: bevorzugt für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht und
- A: bevorzugt für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl steht,
- W: steht bevorzugt für Halogen oder S(O)ₙR⁸, wobei
- R⁸: bevorzugt für (C₁-C₆)Alkyl, (C₁-C₄)Cyanoalkyl, (C₁-C₄)Alkoxy-(C₁-C₄)alkyl, (C₁-C₆)Halogenalkyl, (C₂-C₄)Alkenyl, (C₂-C₄)Halogenalkenyl, (C₂-C₄)Alkinyl, (C₂-C₄)Halogenalkinyl oder (C₃-C₆)Cycloalkyl steht und
- n: bevorzugt für 0, 1 oder 2 steht,
- R²: steht bevorzugt für Halogen, insbesondere Chlor, Brom oder Iod,
- X: steht bevorzugt für Halogen, insbesondere Brom oder Iod,
- V: steht bevorzugt für (C₁-C₆)-Alkyl, (C₁-C₄)Halogenalkyl, (C₁-C₄)Cyanoalkyl, (C₁-C₄)Alkoxy-(C₁-C₄)alkyl, (C₁-C₄)Halogenalkoxy-(C₁-C₄)alkyl, (C₃-C₆)Cycloalkyl, (C₃-C₆)Cycloalkyl-(C₃-C₆)Cycloalkyl, (C₁-C₄)Alkyl-(C₃-C₆)Cycloalkyl, Halogen(C₃-C₆)cycloalkyl, Cyano(C₃-C₆)cycloalkyl, (C₁-C₄)Alkylthio-(C₁-C₄)alkyl, (C₁-C₄)Halogenalkylthio-(C₁-C₄)alkyl, (C₁-C₄)Alkylsulfinyl-(C₁-C₄)alkyl, (C₁-C₄)Halogenalkylsulfinyl-(C₁-C₄)alkyl, (C₁-C₄)Alkylsulfonyl-(C₁-C₄)alkyl oder (C₁-C₄)Halogenalkylsulfonyl-(C₁-C₄)alkyl und
- Y: steht bevorzugt für Wasserstoff, Halogen, (C₁-C₄)Alkyl, (C₁-C₄)Halogenalkyl, (C₁-C₄)Alkoxy, (C₁-C₄)Halogenalkoxy, Aminocarbonyl, (C₁-C₄)Alkylaminocarbonyl, Di-(C₁-C₄)alkyl-aminocarbonyl, (C₁-C₄)Halogenalkylaminocarbonyl, (C₃-C₆)Cycloalkylaminocarbonyl, Amino, (C₁-C₄)Alkylamino, (C₁-C₄)Halogenalkylamino, Di-(C₁-C₄)Alkylamino, (C₃-C₆)Cycloalkylamino, (C₁-C₄)Alkylsulfonylamino, (C₁-C₄)Alkylcarbonylamino, (C₁-C₄)Halogenalkylcarbonylamino, (C₁-C₄)Alkylcarbonyl-(C₁-C₂)alkyl-amino, (C₁-C₄)Halogenalkylcarbonyl-(C₁-C₂)alkyl-amino, (C₃-C₆)Cycloalkylcarbonylamino, (C₃-C₆)Cycloalkylcarbonyl-(C₁-C₂)alkyl-amino, (C₂-C₄)Alkenyl, (C₂-C₄)Halogenalkenyl, (C₂-C₄)Cyanoalkenyl oder (C₃-C₆)Cycloalkyl-(C₂)alkenyl,
oder für jeweils gegebenenfalls einfach oder mehrfach, gleich oder verschieden substituiertes (C₃-C₆)Cycloalkyl oder (C₅-C₆)Cycloalkenyl, wobei als Substituenten jeweils in Frage kommen: (C₃-C₆)Cycloalkyl, (C₁-C₄)Alkyl, (C₁-C₄)Halogenalkyl, (C₁-C₄)Alkoxy, (C₁-C₄)Halogenalkoxy, Halogen oder Cyano,
oder für jeweils gegebenenfalls einfach oder mehrfach, gleich oder verschieden substituiertes Phenyl, Pyridyl, Pyrimidyl, Pyridazinyl, Thiophenyl, Furanyl, Pyrazolyl, Pyrrolyl, Thiazolyl, Oxazolyl oder Imidazolyl, wobei als Substituenten jeweils in Frage kommen: Cyano, Halogen, Nitro, Acetyl, Hydroxy, Amino, SF₅-, (C₃-C₆)Cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₂)alkyl, (C₂-C₄)Alkenyl, (C₂-C₄)Halogenalkenyl, (C₂-C₄)Cyanoalkenyl, (C₃-C₆)Cycloalkyl-(C₂)alkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₂)alkoxy, (C₁-C₄)Alkoxyimino, (C₁-C₄)Halogenalkoxyimino, (C₁-C₄)Alkylthio, (C₁-C₄)Halogenalkylthio, (C₁-C₄)Alkylthio-(C₁-C₂)alkyl, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Halogenalkylsulfonyloxy, (C₁-C₄)Alkylcarbonyl, (C₁-C₄)Halogenalkylcarbonyl, Aminocarbonyl, (C₁-C₄)Alkylaminocarbonyl, (C₁-C₄)Halogenalkylaminocarbonyl, Di-(C₁-C₄)alkyl-aminocarbonyl, (C₃-C₆)Cycloalkylaminocarbonyl, Aminothiocarbonyl, (C₁-C₄)Alkylaminothiocarbonyl, Di-(C₁-C₄)alkylaminothiocarbonyl, (C₁-C₄)Halogenalkylaminothiocarbonyl, (C₃-C₆)Cycloalkylaminothiocarbonyl, (C₁-C₄)Alkylsulfonylamino, (C₁-C₄)Alkylamino, Di-(C₁-C₄)Alkylamino, (C₁-C₄)Halogenalkylamino, (C₃-C₆)Cycloalkylamino, Aminosulfonyl, (C₁-C₄)Alkylaminosulfonyl, Di-(C₁-C₄)alkyl-aminosulfonyl, (C₁-C₄)Alkylcarbonylamino, (C₁-C₄)Halogenalkylcarbonylamino, (C₁-C₄)Alkylcarbonyl-(C₁-C₂)alkyl-amino, (C₁-C₂)Halogenalkylcarbonyl-(C₁-C₂)alkyl-amino, (C₃-C₆)Cycloalkylcarbonylamino, (C₃-C₆)Cycloalkylcarbonyl-(C₁-C₂)alkyl-amino, (C₁-C₄)Alkylthiocarbonylamino, (C₁-C₄)Halogenalkylthiocarbonylamino, (C₁-C₄)Alkylthiocarbonyl-(C₁-C₂)alkyl-amino, (C₁-C₄)Halogenalkylthiocarbonyl-(C₁-C₂)alkyl-amino, (C₃-C₆)Cycloalkylthiocarbonylamino oder (C₃-C₆)Cycloalkylthiocarbonyl-(C₁-C₂)alkyl-amino.

### (Ausgestaltung 3)

- Q: steht besonders bevorzugt für ein Strukturelement aus der Reihe Q2, Q3, Q4, Q10, Q11, Q13 oder Q14, wobei
- R⁷: besonders bevorzugt für (C₁-C₄)Alkyl oder (C₁-C₄)Alkoxy-(C₁-C₄)alkyl steht und
- A: besonders bevorzugt für Brom, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl steht,
- W: steht besonders bevorzugt für Halogen oder S(O)ₙR⁸, wobei
- R⁸: besonders bevorzugt für Methyl, Ethyl, n-Propyl oder iso-Propyl steht und
- n: besonders bevorzugt für 0, 1 oder 2 steht,
- R²: steht besonders bevorzugt für Chlor,
- X: steht besonders bevorzugt für Brom oder Iod, insbesondere Iod,
- V: steht besonders bevorzugt für Methyl, Ethyl, n-Propyl oder iso-Propyl und
- Y: steht besonders bevorzugt für Wasserstoff, Brom, Iod, Ethenyl, Cyclopropylethenyl, i-Propenyl, Cyclopropylethinyl, Methyl, Ethyl, Isopropyl, Cyclopropylethyl, Methoxycarbonyl, Trifluorethylaminocarbonyl, Aminocarbonyl, Methylaminocarbonyl, Dimethylaminocarbonyl, Ethylaminocarbonyl, Aminothiocarbonyl, Methylaminothiocarbonyl, Dimethylaminothiocarbonyl, oder für jeweils gegebenenfalls einfach oder zweifach, gleich oder verschieden substituiertes Cyclopropyl, Cyclobutyl, Cyclopentyl, Cyclohexyl, Cyclopentenyl oder Cyclohexenyl, wobei als Substituenten jeweils in Frage kommen: Methyl, Ethyl, n-Propyl, i-Propyl, cyclo-Propyl, Difluormethyl, Trifluormethyl, Cyano, Fluor oder Chlor,
oder für jeweils gegebenenfalls einfach, zweifach oder dreifach, gleich oder verschieden substituiertes Phenyl, Pyridin-2-yl, Pyridin-3-yl, Pyridin-4-yl, Pyrimidin-5-yl, Pyridazin-3-yl, Pyridazin-4-yl, Thien-2-yl, Thien-3-yl, 1,3-Thiazol-5-yl, 1H-Imidazol-1-yl, 1H-Imidazol-2-yl, 1H-Imidazol-5-yl, 1H-Pyrazol-1-yl, 1H-Pyrazol-3-yl, 1H-Pyrazol-4-yl, 1H-Pyrazol-5-yl, 1H-Pyrrol-1-yl, 1H-Pyrrol-2-yl, 1H-Pyrrol-3-yl, oder 1-Cyclohexenyl, wobei als Substituenten jeweils in Frage kommen: Cyano, Fluor, Chlor, Methyl, Cyclopropyl, Cyanomethyl, Cyanoisopropyl, Cyanocylopropyl, Trifluormethyl, Trifluorethyl oder Aminocarbonyl.

### (Ausgestaltung 4)

- Q: steht ganz besonders bevorzugt für das Strukturelement Q2, Q3 oder Q13, wobei
- R⁷: ganz besonders bevorzugt für Methyl, Ethyl, n-Propyl oder iso-Propyl steht, insbesondere für Methyl und
- A: ganz besonders bevorzugt für Brom, Trifluormethyl, Pentafluorethyl oder Trifluormethylthio steht,
- W: steht ganz besonders bevorzugt für S(O)ₙR⁸, wobei
- R⁸: ganz besonders bevorzugt für Ethyl steht und
- n: ganz besonders bevorzugt für 0 oder 2 steht,
- R²: steht ganz besonders bevorzugt für Chlor,
- X: steht ganz besonders bevorzugt für Iod,
- V: steht ganz besonders bevorzugt für Methyl und
- Y: steht ganz besonders bevorzugt für Wasserstoff, Brom, Cyclopropyl, para-Chlorphenyl, 5-Chlorthien-2-yl oder 5-Chlor-2-pyridin.

Die vorstehend aufgeführten Restedefinitionen bzw. Erläuterungen gelten sowohl für die Endprodukte und Zwischenprodukte als auch für die Ausgangsprodukte entsprechend. Diese Restedefinitionen können untereinander, also auch zwischen den jeweiligen Vorzugsbereichen, beliebig kombiniert werden.

Erfindungsgemäß bevorzugt werden solche Verbindungen, in welchen eine Kombination der vorstehend als bevorzugt aufgeführten Bedeutungen vorliegt.

Erfindungsgemäß besonders bevorzugt werden solche Verbindungen, in welchen eine Kombination der vorstehend als besonders bevorzugt aufgeführten Bedeutungen vorliegt.

Erfindungsgemäß ganz besonders bevorzugt werden solche Verbindungen, in welchen eine Kombination der vorstehend als ganz besonders bevorzugt aufgeführten Bedeutungen vorliegt.

In einer weiteren bevorzugten Ausführungsform der Erfindung steht Q für Q1 und R⁷, A, W, R², X, V und Y haben die in Ausgestaltung 1 oder die in Ausgestaltung 2 oder die in Ausgestaltung 3 oder die in Ausgestaltung 4 angegebenen Bedeutungen (Ausgestaltung 5).

In einer weiteren bevorzugten Ausführungsform der Erfindung steht Q für Q2 und R⁷, A, W, R², X, V und Y haben die in Ausgestaltung 1 oder die in Ausgestaltung 2 oder die in Ausgestaltung 3 oder die in Ausgestaltung 4 angegebenen Bedeutungen (Ausgestaltung 6).

In einer weiteren bevorzugten Ausführungsform der Erfindung steht Q für Q3 und R⁷, A, W, R², X, V und Y haben die in Ausgestaltung 1 oder die in Ausgestaltung 2 oder die in Ausgestaltung 3 oder die in Ausgestaltung 4 angegebenen Bedeutungen (Ausgestaltung 7).

In einer weiteren bevorzugten Ausführungsform der Erfindung steht Q für Q4 und A, W, R², X, V und Y haben die in Ausgestaltung 1 oder die in Ausgestaltung 2 oder die in Ausgestaltung 3 oder die in Ausgestaltung 4 angegebenen Bedeutungen (Ausgestaltung 8).

In einer weiteren bevorzugten Ausführungsform der Erfindung steht Q für Q5 und A, W, R², X, V und Y haben die in Ausgestaltung 1 oder die in Ausgestaltung 2 oder die in Ausgestaltung 3 oder die in Ausgestaltung 4 angegebenen Bedeutungen (Ausgestaltung 9).

In einer weiteren bevorzugten Ausführungsform der Erfindung steht Q für Q6 und A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 10).

In einer weiteren bevorzugten Ausführungsform der Erfindung steht Q für Q7 und A, W, R², X, V und Y haben die in Ausgestaltung 1 oder die in Ausgestaltung 2 oder die in Ausgestaltung 3 oder die in Ausgestaltung 4 angegebenen Bedeutungen (Ausgestaltung 11).

In einer weiteren bevorzugten Ausführungsform der Erfindung steht Q für Q8 und A, W, R², X, V und Y haben die in Ausgestaltung 1 oder die in Ausgestaltung 2 oder die in Ausgestaltung 3 oder die in Ausgestaltung 4 angegebenen Bedeutungen (Ausgestaltung 12).

In einer weiteren bevorzugten Ausführungsform der Erfindung steht Q für Q9 und A, W, R², X, V und Y haben die in Ausgestaltung 1 oder die in Ausgestaltung 2 oder die in Ausgestaltung 3 oder die in Ausgestaltung 4 angegebenen Bedeutungen (Ausgestaltung 13).

In einer weiteren bevorzugten Ausführungsform der Erfindung steht Q für Q10 und A, W, R², X, V und Y haben die in Ausgestaltung 1 oder die in Ausgestaltung 2 oder die in Ausgestaltung 3 oder die in Ausgestaltung 4 angegebenen Bedeutungen (Ausgestaltung 14).

In einer weiteren bevorzugten Ausführungsform der Erfindung steht Q für Q11 und A, W, R², X, und Y haben die in Ausgestaltung 1 oder die in Ausgestaltung 2 oder die in Ausgestaltung 3 oder die in Ausgestaltung 4 angegebenen Bedeutungen (Ausgestaltung 15).

In einer weiteren bevorzugten Ausführungsform der Erfindung steht Q für Q12 und, A, W, R², X, V und Y haben die in Ausgestaltung 1 oder die in Ausgestaltung 2 oder die in Ausgestaltung 3 oder die in Ausgestaltung 4 angegebenen Bedeutungen (Ausgestaltung 16).

In einer weiteren bevorzugten Ausführungsform der Erfindung steht Q für Q13 und R⁷, A, W, R², X, V und Y haben die in Ausgestaltung 1 oder die in Ausgestaltung 2 oder die in Ausgestaltung 3 oder die in Ausgestaltung 4 angegebenen Bedeutungen (Ausgestaltung 17).

In einer weiteren bevorzugten Ausführungsform der Erfindung steht Q für Q14 und R⁷, W, R², X, V und Y haben die in Ausgestaltung 1 oder die in Ausgestaltung 2 oder die in Ausgestaltung 3 oder die in Ausgestaltung 4 angegebenen Bedeutungen (Ausgestaltung 18).

In einer besonders bevorzugten Ausführungsform der Erfindung steht Q für Q2, Q3, Q4, Q10, Q11, Q13 oder Q14 und R⁷, A, W, R², X, V und Y haben die in Ausgestaltung 1 oder die in Ausgestaltung 2 oder die in Ausgestaltung 4 angegebenen Bedeutungen (Ausgestaltung 19).

In einer ganz besonders bevorzugten Ausführungsform der Erfindung steht Q für Q2, Q3 oder Q13 und R⁷, A, W, R², X, V und Y haben die in Ausgestaltung 1 oder die in Ausgestaltung 2 oder die in Ausgestaltung 3 angegebenen Bedeutungen (Ausgestaltung 20).

In einer weiteren bevorzugten Ausführungsform der Erfindung steht W für S(O)ₙR⁸ und Q, n, R⁷, R⁸, A, R², X, V und Y haben die in Ausgestaltung 1 oder die in Ausgestaltung 2 oder die in Ausgestaltung 3 oder die in Ausgestaltung 4 angegebenen Bedeutungen (Ausgestaltung 21).

Vorteilhafterweise lassen sich die Imidazolderivate der Formel (II) mit dem erfindungsgemäßen Verfahren mit guten Ausbeuten und in hoher Reinheit herstellen. Ein großer Vorteil des erfindungsgemäßen Verfahrens ist dessen Regioselektivität. Aufgrund der sehr guten funktionellen Gruppen-Toleranz von Zink-Reagenzien sind Zinkbasen sehr attraktiv. Insbesondere vorteilhaft ist ferner die Möglichkeit, auch bei deutlich niedrigeren Temperaturen Negishi-Kupplungen durchführen zu können, wobei in erfindungsgemäßen Verfahren auch bei höheren Temperaturen empfindliche funktionelle Gruppen wie Ester oder Fluoratome toleriert werden, ohne die die vorhandene Regioselektivität zu beeinträchtigen. Ferner können Negishi Kreuzkupplungen im Rahmen eines erfindungsgemäßen Verfahrens auch in Gegenwart von ortho-Substituenten am Imidazolgerüst gute Ausbeuten an Zielprodukt ergeben, obwohl solche Kupplungen mit 2-substituierten Imidazolderivaten bislang dafür bekannt sind, niedrige Ausbeuten zu liefern. Somit werden weitere und/oder flexiblere Derivatisierungen von Edukt und Produkt möglich, ohne Synthesenrouten ständig ändern bzw. anzupassen zu müssen.

Das erfindungsgemäße Verfahren kann anhand des folgenden Schemas (I) erläutert werden: 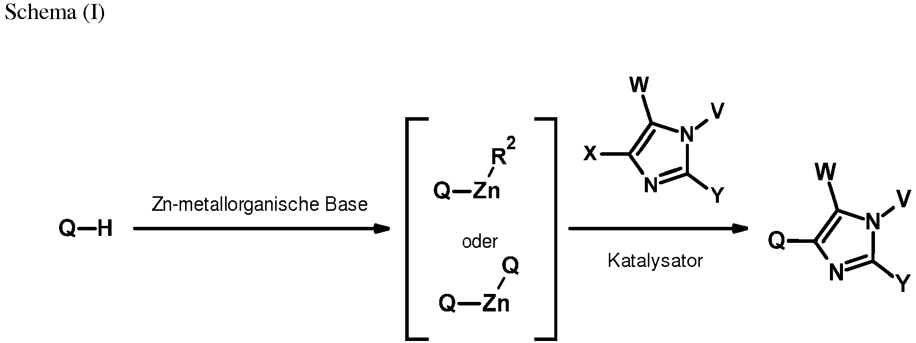 Hierin haben Q, W, R², X, V und Y sowie die innerhalb der jeweiligen Definitionen ggfs. vorhandenen weiteren Strukturelemente jeweils die vorstehend angegebenen Bedeutungen. Die in Klammern angegebenen Verbindungen stellen die Zwischenstufe (Formel IIIa beziehungsweise IIIb) dar, welche mit einer Verbindung der Formel (I) weiter zur Verbindung der Formel (II) umgesetzt wird. Demnach lässt sich das erfindungsgemäße Verfahren in die beiden Verfahrensschritte a) und b) unterteilen, wobei Schritt a) die Umsetzung der Verbindung Q-H zur jeweiligen Zwischenstufe und Schritt b) die weitere Umsetzung der Zwischenstufe zur Verbindung der Formel (II) ist.

### Allgemeine Definitionen

Im Zusammenhang mit der vorliegenden Erfindung umfasst der Begriff Halogen (Hal), soweit nicht anders definiert, solche Elemente, die ausgewählt sind aus der Gruppe bestehend aus Fluor, Chlor, Brom und Iod.

Der Begriff "Halogenide" beschreibt im Zusammenhang mit der vorliegenden Erfindung Verbindungen zwischen Halogenen und Elementen anderer Gruppen des Periodensystems, wobei salzartige Halogenide (ionische Verbindungen (Salze), die aufgrund der großen Elektronegativitätsdifferenz zwischen den beteiligten Elementen aus Anionen und Kationen bestehen und durch elektrostatische Wechselwirkungen zusammengehalten werden) oder kovalente Halogenide (kovalente Verbindungen, bei denen der Elektronegativitätsunterschied nicht so groß ist wie bei den vorstehend genannten ionischen Verbindungen, die Bindungen jedoch eine Ladungspolarität aufweisen) vorliegen können, abhängig von der Art der chemischen Bindung. Erfindungsgemäß besonders bevorzugt sind salzartige Halogenide.

Der Begriff "Pivaloyl" beschreibt im Zusammenhang mit der vorliegenden Erfindung den deprotonierten Rest der Pivalinsäure (X) mit der Summenformel (CH₃)₃CCO₂H. 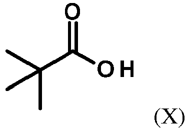 "O-Pivaloyl" bedeutet entsprechend, dass die Bindung des Pivaloylrestes über das deprotonierte Sauerstoffatom der Säuregruppe erfolgt.

Sofern nicht an anderer Stelle anders definiert, wird unter dem Begriff "Alkyl", entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl n-Heptyl, n-Octyl, n-Nonyl, n-Decyl, n-Undecyl und n-Dodecyl. Von diesen Alkylresten sind C₁-C₆-Alkylreste besonders bevorzugt. Insbesondere bevorzugt sind C₁-C₄-Alkylreste.

Sofern nicht an anderer Stelle anders definiert, wird unter dem Begriff "Alkenyl", entweder in Alleinstellung oder aber in Kombination mit weiteren Begriffen, erfindungsgemäß ein linearer oder verzweigter C₂-C₁₂-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden. Bevorzugt hiervon sind C₂-C₆-Alkenylreste und besonders bevorzugt sind C₂-C₄-Alkenylreste.

Sofern nicht an anderer Stelle anders definiert, wird unter dem Begriff "Alkinyl", entweder in Alleinstellung oder aber in Kombination mit weiteren Begriffen, erfindungsgemäß ein linearer oder verzweigter C₂-C₁₂-Alkinylrest, welcher mindestens eine Dreifachbindung aufweist, beispielsweise Ethinyl, 1-Propinyl und Propargyl, verstanden. Bevorzugt hiervon sind C₃-C₆-Alkinylreste und besonders bevorzugt sind C₃-C₄-Alkinylreste. Der Alkinylrest kann dabei auch mindestens eine Doppelbindung aufweisen.

Sofern nicht an anderer Stelle anders definiert, wird unter dem Begriff "Cycloalkyl", entweder in Alleinstellung oder aber in Kombination mit weiteren Begriffen, erfindungsgemäß ein C₃-C₈-Cycloalkylrest verstanden, beispielsweise Cyclopropyl, Cyclobutyl, Cyclopentyl, Cyclohexyl, Cycloheptyl und Cyclooctyl, verstanden. Bevorzugt hiervon sind C₃-C₆-Cycloalkylreste.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Sofern nicht an anderer Stelle anders definiert, wird unter dem Begriff "Aryl" erfindungsgemäß ein aromatischer Rest mit 6 bis 14 Kohlenstoffatomen, vorzugsweise Phenyl, Naphthyl, Anthryl oder Phenanthrenyl, besonders bevorzugt Phenyl, verstanden.

Sofern nicht an anderer Stelle anders definiert, wird unter dem Begriff "Arylalkyl" eine Kombination von erfindungsgemäß definierten Resten "Aryl" und "Alkyl" verstanden, wobei der Rest im Allgemeinen über die Alkylgrupe gebunden wird, Beispiele hierfür sind Benzyl, Phenylethyl oder α-Methylbenzyl, wobei Benzyl besonders bevorzugt ist.

Sofern nicht an anderer Stelle anders definiert, bedeutet "Hetaryl" bzw "heteroaromatischer Ring" eine mono-, bi- oder tricyclische heterocyclische Gruppe aus C-Atomen und mindestens einem Heteroatom, wobei mindestens ein Zyklus aromatisch ist. Bevorzugt enthält die Hetaryl-Gruppe 3, 4, 5, 6, 7 oder 8 C-Atome. Besonders bevorzugt sind dabei monocyclische Gruppen aus 3, 4, 5, 6, 7 oder 8 C-Atomen und mindestens einem Heteroatom.Insbesondere bevorzugt ist die Hetaryl-Gruppe ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl, Imidazopyridinyl und Indolizinyl.

Durch Halogen substituierte Reste, z.B. Halogenalkyl (=Haloalkyl), sind einfach oder mehrfach bis zur maximal möglichen Substituentenzahl halogeniert. Bei mehrfacher Halogenierung können die Halogenatome gleich oder verschieden sein. Soweit nicht anders definiert steht Halogen dabei für Fluor, Chlor, Brom oder Iod, insbesondere für Fluor, Chlor oder Brom. Mit einem oder mehreren Halogenatomen (-Hal) substituierte Alkyl-Gruppen sind beispielsweise ausgewählt aus Trifluormethyl (CF₃), Difluormethyl (CHF₂), CF₃CH₂, ClCH₂ oder CF₃CCl₂.

Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Die Synthese von Verbindungen Q-H als Edukte eines erfindungsgemäßen Verfahrens ist dem Fachmann grundsätzlich bekannt. Beispielsweise können Verbindungen Q-H mit Q = Q1, Q2, Q3, Q13 oder Q14 aus entsprechenden (Het)Aryl-Diaminderivaten durch Ringschluss zur jeweiligen Imidazolverbindung erhalten werden, wie zum Beispiel in WO 2014/100065 oder WO 2015/017610 beschrieben, vorzugsweise unter sauren Bedingungen. Alternativsynthesen sind ebenfalls möglich, sind jedoch komplexer und dadurch im Regelfall wirtschaftlich nachteiliger. Die Verbindungen Q-H mit Q = Q4, Q5 oder Q6 können beispielweise aus entsprechenden (Het)Aryl-Aminoalkoholderivaten durch Ringschluss mit einem Orthoester der Ameisensäure zur jeweiligen Oxazolverbindung erhalten werden, wie zum Beispiel in WO 2018/037223. Die Verbindungen Q-H mit Q = Q7, Q8 oder Q9 können beispielweise aus entsprechenden (Het)Aryl-Aminohalogenderivaten durch Ringschluss mit einem O-Alkylcarbonothioat und anschließende Decarboxylierung zur jeweiligen Thiazolverbindung erhalten werden, wie zum Beispiel in WO 2013/066729. Die Verbindungen Q-H mit Q = Q10, Q11 oder Q12 können beispielweise aus entsprechenden (Het)Aryl-Aminoderivaten durch Ringschluss mit einem Halogenacetaldehyd zur jeweiligen Imidazolverbindung erhalten werden, wie zum Beispiel in WO 2003/099816, WO 2010/083145 oder Organic Process Research & Development 2006, 10, 398-402.

Die Umsetzung der Verbindungen Q-H zu Verbindungen der Formel (IIIa) oder Formel (IIIb) im ersten Verfahrensschritt (Schritt a)) erfolgt in Gegenwart einer Zink-metallorganischen Base der Struktur (NR³R⁴)-Zn-R² oder (NR³R⁴)₂-Zn, in welcher (Ausgestaltung B-1)
R² wie vorstehend (Ausgestaltung 1) definiert ist (daher für Halogen oder -O-Pivaloyl steht),
R³ und R⁴ gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R⁵-Radikale substituiert sein kann und
R⁵ ausgewählt ist aus der Gruppe bestehend aus Methyl, Ethyl, n-Propyl und i-Propyl.

Bevorzugt ist, dass (Ausgestaltung B-2)
R² wie vorstehend als bevorzugt (Ausgestaltung 2) definiert ist (daher für Halogen steht, insbesondere für Chlor, Brom oder Iod),
R³ und R⁴ gemeinsam eine -(CH₂)₅-Gruppe bilden, wobei jede dieser Gruppen optional durch 1, 2, 3 oder 4 R⁵-Radikale substituiert sein kann und
R⁵ ausgewählt ist aus der Gruppe bestehend aus Methyl und Ethyl.

Besonders bevorzugt ist, dass (Ausgestaltung B-3)
R² wie vorstehend als besonders bevorzugt (Ausgestaltung 3) oder als ganz besonders bevorzugt (Ausgestaltung 4) definiert ist (daher für Chlor steht) und
R³ und R⁴ gemeinsam eine -(CH₂)₅-Gruppe bilden, die durch 4 Methylgruppen substituiert ist.

Die vorstehend aufgeführten Restedefinitionen können untereinander, also auch zwischen den jeweiligen Vorzugsbereichen, beliebig kombiniert werden.

In einer ganz besonders bevorzugten Ausgestaltung der erfindungsgemäßen Base ist das Strukturelement (NR³R⁴) Tetramethylpiperidin (TMP) gemäß Formel (IV).

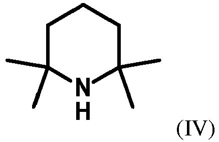

Erfindungsgemäß ganz besonders bevorzugte Zink-metallorganische Basen sind demnach dadurch gekennzeichnet, dass Zink gebunden an TMP vorliegt, insbesondere als Zinkhalogenid und ganz besonders bevorzugt als Zinkchlorid. Derartige Basen weisen folgende Struktur der Formel (V) auf (Ausgestaltung B-4)

(V) (TMP)ₓ ZnCl₂₋ₓ ,

worin x für die Zahl 1 oder 2 steht. Hierunter wiederum bevorzugt sind Basen mit x=1 (Ausgestaltung B-5) gemäß Formel (VI):

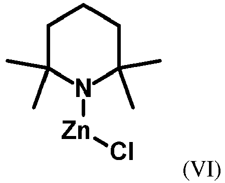

In einer weiteren bevorzugten Ausführungsform des erfindungsgemäßen Verfahrens liegt die Zink-metallorganische Base in Verbindung mit Alkali- oder Erdalkalihalogeniden vor. Dies gilt insbesondere für Basen der Formeln (V) und (VI). Besonders bevorzugte derartige Alkali- oder Erdalkalihalogenide sind Lithiumchlorid und Magnesiumchlorid, ganz besonders bevorzugt ist Lithiumchlorid. Erfindungsgemäß ganz besonders bevorzugte Zink-metallorganische Basen sind demnach TMP ZnCl. LiCl oder (TMP)₂ Zn·2LiCl (Ausgestaltung B-6). Am meisten bevorzugt ist TMP ZnCl. LiCl (VII; Ausgestaltung B-7).

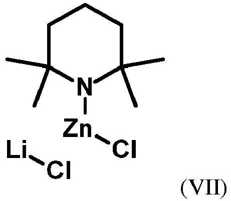

Beispielhaft werden in nachfolgender Tabelle 1 konkrete Kombinationen von Verbindungen der Formeln (I), (II) und (IIIa) bzw. (IIIb) mit erfindungsgemäßen Basen genannt, die in einem erfindungsgemäßen Verfahren zur Anwendung kommen können. Da in einigen Ausgestaltungen das Strukturelement R² sowohl in der erfindungsgemäßen Base als auch in der Verbindung der Formel (IIIa) vorliegt, gilt für R² jeweils die engste Definition.

**Tabelle 1:**

| Nummer | Verbindungen der Formeln (I), (II) und (IIIa) bzw. (IIIb) | Base gemäß |
|---|---|---|
| 1 | Ausgestaltung 1 | Ausgestaltung B-1 |
| 2 | Ausgestaltung 1 | Ausgestaltung B-2 |
| 3 | Ausgestaltung 1 | Ausgestaltung B-3 |
| 4 | Ausgestaltung 1 | Ausgestaltung B-4 |
| 5 | Ausgestaltung 1 | Ausgestaltung B-5 |
| 6 | Ausgestaltung 1 | Ausgestaltung B-6 |
| 7 | Ausgestaltung 1 | Ausgestaltung B-7 |
| 8 | Ausgestaltung 2 | Ausgestaltung B-1 |
| 9 | Ausgestaltung 2 | Ausgestaltung B-2 |
| 10 | Ausgestaltung 2 | Ausgestaltung B-3 |
| 11 | Ausgestaltung 2 | Ausgestaltung B-4 |
| 12 | Ausgestaltung 2 | Ausgestaltung B-5 |
| 13 | Ausgestaltung 2 | Ausgestaltung B-6 |
| 14 | Ausgestaltung 2 | Ausgestaltung B-7 |
| 15 | Ausgestaltung 3 | Ausgestaltung B-1 |
| 16 | Ausgestaltung 3 | Ausgestaltung B-2 |
| 17 | Ausgestaltung 3 | Ausgestaltung B-3 |
| 18 | Ausgestaltung 3 | Ausgestaltung B-4 |
| 19 | Ausgestaltung 3 | Ausgestaltung B-5 |
| 20 | Ausgestaltung 3 | Ausgestaltung B-6 |
| 21 | Ausgestaltung 3 | Ausgestaltung B-7 |
| 22 | Ausgestaltung 4 | Ausgestaltung B-1 |
| 23 | Ausgestaltung 4 | Ausgestaltung B-2 |
| 24 | Ausgestaltung 4 | Ausgestaltung B-3 |
| 25 | Ausgestaltung 4 | Ausgestaltung B-4 |
| 26 | Ausgestaltung 4 | Ausgestaltung B-5 |
| 27 | Ausgestaltung 4 | Ausgestaltung B-6 |
| 28 | Ausgestaltung 4 | Ausgestaltung B-7 |
| 29 | Ausgestaltung 17 | Ausgestaltung B-1 |
| 30 | Ausgestaltung 17 | Ausgestaltung B-2 |
| 31 | Ausgestaltung 17 | Ausgestaltung B-3 |
| 32 | Ausgestaltung 17 | Ausgestaltung B-4 |
| 33 | Ausgestaltung 17 | Ausgestaltung B-5 |
| 34 | Ausgestaltung 17 | Ausgestaltung B-6 |
| 35 | Ausgestaltung 17 | Ausgestaltung B-7 |
| 36 | Ausgestaltung 19 | Ausgestaltung B-1 |
| 37 | Ausgestaltung 19 | Ausgestaltung B-2 |
| 38 | Ausgestaltung 19 | Ausgestaltung B-3 |
| 39 | Ausgestaltung 19 | Ausgestaltung B-4 |
| 40 | Ausgestaltung 19 | Ausgestaltung B-5 |
| 41 | Ausgestaltung 19 | Ausgestaltung B-6 |
| 42 | Ausgestaltung 19 | Ausgestaltung B-7 |
| 43 | Ausgestaltung 20 | Ausgestaltung B-1 |
| 44 | Ausgestaltung 20 | Ausgestaltung B-2 |
| 45 | Ausgestaltung 20 | Ausgestaltung B-3 |
| 46 | Ausgestaltung 20 | Ausgestaltung B-4 |
| 47 | Ausgestaltung 20 | Ausgestaltung B-5 |
| 48 | Ausgestaltung 20 | Ausgestaltung B-6 |
| 49 | Ausgestaltung 20 | Ausgestaltung B-7 |
| 50 | Ausgestaltung 21 | Ausgestaltung B-1 |
| 51 | Ausgestaltung 21 | Ausgestaltung B-2 |
| 52 | Ausgestaltung 21 | Ausgestaltung B-3 |
| 53 | Ausgestaltung 21 | Ausgestaltung B-4 |
| 54 | Ausgestaltung 21 | Ausgestaltung B-5 |
| 55 | Ausgestaltung 21 | Ausgestaltung B-6 |
| 56 | Ausgestaltung 21 | Ausgestaltung B-7 |

Vorzugsweise wird die Zink-metallorganische Base in dem erfindungsgemäßen Verfahren in einer Gesamtmenge von 0,5 bis 5,0 Äquivalenten, vorzugsweise von 0,8 bis 2,0 Äquivalenten, weiter bevorzugt von 1,0 bis 1,5 Äquivalenten und besonders bevorzugt von 1,0 bis 1,2 Äquivalenten, bezogen auf die Verbindung Q-H, eingesetzt. Ein Vorteil des erfindungsgemäßen Verfahrens ist es diesbezüglich, dass die metallorganische Base in nahezu stöchiometrischen Mengen eingesetzt werden kann.

In Abhängigkeit davon, ob in der eingesetzten Zink-metallorganischen Base das Strukturelement (NR³R⁴) einfach oder zweifach vorhanden ist, entstehen Zwischenverbindungen der Formel (IIIa) beziehungsweise der Formel (IIIb) in Verfahrensschritt a).

Die Umsetzung der Verbindungen der Formel (IIIa) beziehungsweise (IIIb) zu Verbindungen der Formel (II) im zweiten Verfahrensschrittschritt b) erfolgt in Gegenwart einer Verbindung der Formel (I) 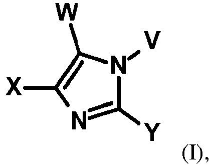 in welcher X, V, W und Y jeweils die oben genannten Bedeutungen haben.

Die Verbindungen der Formel (I) sind bevorzugt so gewählt, dass X die beste Abgangsgruppe im Molekül darstellt. Daher ist X bevorzugt Brom oder Iod und insbesondere Iod. Die Reaktion findet dann während der Negishi Kreuzkupplung nahezu ausschließlich an der 4 Position statt, da X die beste Abgangsgruppe an dem Imidazolgerüst ist. Sie liefert dann regioselektiv das entsprechende Imidazolderivat der Formel (II).

Verbindungen der Formel (I) lassen sich beispielsweise durch selektive Substitution einer entsprechenden Vorläuferverbindung, d.h. einem Imidazolderivat, in dem die Reste W und X für das gleiche Halogen (z.B. Iod) stehen, erhalten. Eine derartige selektive Substitution kann beispielsweise durch Halogen-Metall-Austausch der Vorläuferverbindung in Gegenwart von Lithium- oder Magnesiumbasen und anschließender Umsetzung mit elementarem Halogen (z.B. Brom) oder einem Disulfid erfolgen. Derartiger Halogen-Metall-Austausch ist beispielsweise in Bulletin of the Chemical Society of Japan 2013, 86, 927-939 oder Journal of Organic Chemistry 2000, 65, 4618-4634 beschrieben.

Vorzugsweise wird die Verbindung der Formel (I) in dem erfindungsgemäßen Verfahren in einer Gesamtmenge von 0,5 bis 10 Äquivalenten, vorzugsweise von 0,8 bis 5,0 Äquivalenten, weiter bevorzugt von 1,0 bis 2,5 Äquivalenten und besonders bevorzugt von 1,0 bis 1,5 Äquivalenten oder besonders bevorzugt von 1,5 bis 2,0 Äquivalenten oder besonders bevorzugt von 1,0 bis 2,0 Äquivalenten, bezogen auf die Verbindung Q-H, eingesetzt.

Die Umsetzung in Verfahrensschritt b) erfolgt ferner in Gegenwart eines Katalysators. Vorzugsweise ist der Katalysator eine Palladiumverbindung oder eine Nickelverbindung. Besonders bevorzugt ist der Katalysator eine Palladiumverbindung. Ganz besonders bevorzugt handelt es sich um Tetrakis(triphenylphosphine)palladium(0), abgekürzt Pd(PPh₃)₄, der Formel (IX). 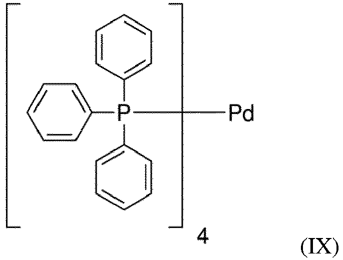 Üblicherweise werden in einem erfindungsgemäßen Verfahren 2,5-25 mol% und vorzugsweise 5-20 mol% Katalysator eingesetzt.

Die erfindungsgemäße Umsetzung der Verbindungen Q-H zu Verbindungen der Formel (IIIa) beziehungsweise (IIIb) und weiter zu Verbindungen der Formel (II) erfolgt vorzugsweise jeweils in Gegenwart eines organischen Lösungsmittels. Als Lösungsmittel kommen prinzipiell alle organischen Lösungsmittel in Frage, die unter den angewendeten Reaktionsbedingungen inert sind und in denen die umzusetzenden Verbindungen eine ausreichende Löslichkeit aufweisen. Als geeignete Lösungsmittel sind insbesondere zu nennen: Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Ethylencarbonat, Propylencarbonat, N,N-Dimethylacetamid, N,N-Dimethylformamid (DMF), N-Methylpyrrolidon (NMP), N-Ethyl-2-pyrrilidon (NEP), N-Butyl-2-pyrrilidon (NBP); N,N'-Dimethylpropylenharnstoff (DMPU), Halogenkohlenwasserstoffe und aromatische Kohlenwasserstoffe, insbesondere Chlorkohlenwasserstoffe wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, insbesondere 1,2-Dichlorbenzol, Chlortoluol, Trichlorbenzol; 4-Methoxybenzol, fluorierte Aliphate und Aromaten wie Trichlortrifluorethan, Benzotrifluorid und 4-Chlorbenzotrifluorid.Es können auch Lösungsmittelgemische, vorzugsweise Gemische aus den vorstehend genannten Lösungsmitteln wie Tetrahydrofuran (THF), 1,4-Dioxan, Diethylether, Diglyme, Methyltertbutylether (MTBE), tert-Amyl-methylether (TAME), 2-Methyl-THF, Toluol, Xylole, Mesitylen, Dimethylformamid (DMF), eingesetzt werden.

Bevorzugte Lösungsmittel sind THF, N,N-Dimethylformamid (DMF), 1,4-Dioxan, Diglyme, Methyltertbutylether (MTBE), tert-Amyl-methylether (TAME), 2-Methyl-THF, Toluol und 4-Methoxybenzol.

Besonders bevorzugte Lösungsmittel sind THF und N,N-Dimethylformamid (DMF), ganz besonders bevorzugt ist THF.

Das Lösungsmittel kann ferner entgast (sauerstoff-frei) sein.

Vorzugsweise wird für beide Verfahrensschritte a) und b) das gleiche Lösungsmittel verwendet. Alternative erfindungsgemäße Ausgestaltungen, in denen für die Verfahrensschritte a) und b) unterschiedliche Lösungsmittel verwendet werden, sind allerdings ebenfalls möglich, wobei die Lösungsmittel dann ebenfalls vorzugsweise aus den vorstehend genannten Lösungsmitteln ausgewählt sind und die jeweiligen als bevorzugt, besonders bevorzugt und ganz besonders bevorzugt angegebenen Lösungsmittel auf den jeweiligen Verfahrensschritt a) oder b) zu beziehen sind.

Die Umsetzung in Verfahrensschritt a) wird im Allgemeinen bei einer Temperatur zwischen 0 °C und 80 °C und zunehmend bevorzugt zwischen 10 °C und 70 °C, zwischen 15 °C und 60 °C, zwischen 20 °C und 50 °C, zwischen 20 °C und 40 °C und ganz besonders bevorzugt zwischen 20 °C und 35 °C, beispielsweise bei Raumtemperatur bzw. 25 °C, durchgeführt.

Schritt a) erfolgt im Allgemeinen in einem Zeitraum von 5 min bis 12 h, bevorzugt von 15 min bis 10 h und besonders bevorzugt von 30 min bis 2 h.

Die Umsetzung in Verfahrensschritt b) wird im Allgemeinen bei einer Temperatur zwischen 40 °C und 90 °C und zunehmend bevorzugt zwischen 50 °C und 85 °C, zwischen 55 °C und 80 °C, zwischen 60 °C und 80 °C und ganz besonders bevorzugt zwischen 65 °C und 75 °C, beispielsweise bei 65 °C, durchgeführt.

Schritt b) erfolgt in dieser Variante der Erfindung im Allgemeinen in einem Zeitraum von 5 min bis 12 h, bevorzugt von 15 min bis 10 h und besonders bevorzugt von 30 min bis 4 h.

Die Reaktion wird üblicherweise bei Normaldruck durchgeführt, kann aber auch bei erhöhtem bzw. vermindertem Druck durchgeführt werden.

Die Isolierung der gewünschten Verbindungen der Formel (II) kann beispielsweise durch wässrige Aufarbeitung in Gegenwart von gesättigten Ammoniumchlorid- oder Natriumthiosulfat-Lösungen und/oder anschließende Chromatographie erfolgen. Solche Verfahren sind dem Fachmann bekannt und schließen auch eine Kristallisation aus einem organischen Lösungsmittel oder Lösungsmittelgemisch ein.

Ein Beispiel einer besonders bevorzugten Ausführungsform des erfindungsgemäßen Verfahrens kann anhand des folgenden Schemas (II) erläutert werden:

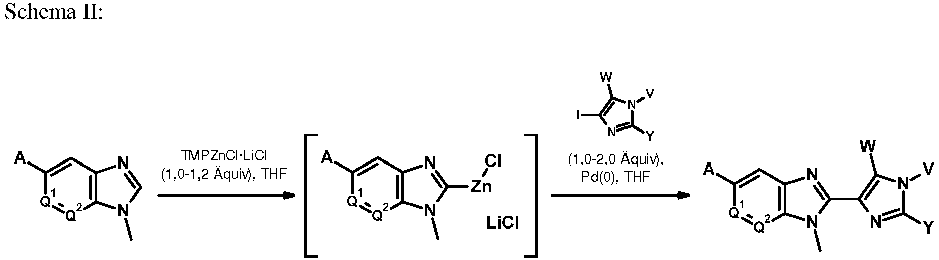

Hierin haben A, Q¹, Q², V, W und Y die vorstehend angegebenen Bedeutungen. Die in Klammern angegebene Verbindung stellt die entsprechende Zwischenstufe der Formel (IIIa) dar, welche weiter zum Produkt, einer Verbindung der Formel (II), umgesetzt wird. Beide Umsetzungen finden in THF als Lösungsmittel statt. "Äquiv" bezeichnet die eingesetzte Menge an Äquivalenten TMPZnCl·LiCl bzw. Verbindung der Formel (I). Pd(0) steht für eine Palladiumverbindung als Katalysator, vorzugsweise Pd(PPh₃)₄.

Die vorliegende Erfindung wird anhand der nachfolgenden Beispiele näher erläutert, wobei die Beispiele nicht in einer die Erfindung einschränkenden Weise zu interpretieren sind.

### Analytische Bestimmungen

Die nachstehend beschriebenen Durchführungen der analytischen Bestimmungen beziehen sich auf alle Angaben im gesamten Dokument, sofern die Durchführung der jeweiligen analytischen Bestimmung an der jeweiligen Textstelle nicht gesondert beschrieben ist.

### Massenspektrometrie

Die Bestimmung von [M+H]⁺ oder M mittels LC-MS unter sauren chromatographischen Bedingungen wurde mit 1 mL Ameisensäure pro Liter Acetonitril und 0,9 mL Ameisensäure pro Liter Millipore-Wasser als Eluenten durchgeführt. Es wurde die Säule Zorbax Eclipse Plus C18 50 mm * 2,1 mm, 1,8 µm verwendet, bei einer Temperatur des Säulenofens von 55 °C.

### Instrumente:

**LC-MS3:** Waters UPLC mit SQD2 Massenspektrometer und SampleManager Probenwechsler. Linearer Gradient 0,0 bis 1,70 Minuten von 10% Acetonitril zu 95% Acetonitril, von 1,70 bis 2,40 Minuten konstant 95% Acetonitril, Fluss 0,85 mL/min.

**LC-MS6 und LC-MS7:** Agilent 1290 LC, Agilent MSD Massenspektrometer, HTS PAL Probenwechsler. Linearer Gradient 0,0 bis 1,80 Minuten von 10% Acetonitril zu 95% Acetonitril, von 1,80 bis 2,50 Minuten konstant 95% Acetonitril, Fluss 1,0 mL/min).

Die Bestimmung von [M+H]⁺ mittels LC-MS unter neutralen chromatographischen Bedingungen wurde mit Acetonitril und Millipore-Wasser mit 79 mg/L Ammoniumcarbonat als Eluenten durchgeführt.

### Instrumente:

**LC-MS4:** Waters IClass Acquity mit QDA Massenspektrometer und FTN Probenwechsler (Säule Waters Acquity 1,7 µm 50 mm ^{∗} 2,1 mm, Säulenofentemperatur 45 °C). Linearer Gradient 0,0 bis 2,10 Minuten von 10% Acetonitril zu 95% Acetonitril, von 2,10 bis 3,00 Minuten konstant 95% Acetonitril, Fluss 0,7 mL/min.

**LC-MS5:** Agilent 1100 LC System mit MSD Massenspektrometer und HTS PAL Probenwechsler (Säule: Zorbax XDB C18 1,8 µm 50 mm ^{∗} 4,6 mm, Säulenofentemperatur 55 °C). Linearer Gradient 0,0 bis 4,25 Minuten von 10% Acetonitril zu 95% Acetonitril, von 4,25 bis 5,80 Minuten konstant 95% Acetonitril, Fluss 2,0 mL/min.

Die Retentionzeit-Indizes wurden in allen Fällen aus einer Kalibrierungsmessung einer homologen Serie von geradkettigen Alkan-2-onen mit 3 bis 16 Kohlenstoffen bestimmt, wobei der Index des ersten Alkanons auf 300, der des letzten auf 1600 gesetzt und zwischen den Werten aufeinanderfolgender Alkanone linear interpoliert wurde.

### logP-Werte

Die Bestimmung der logP-Werte erfolgte gemäß EEC Directive 79/831 Annex V.A8 durch HPLC (High Performance Liquid Chromatography) an einer Phasenumkehrsäule (C18) mit Hilfe folgender Methoden:
Der logP[a] Wert wird durch LC-UV Messung im sauren Bereich bestimmt, mit 0,9 mL/L Ameisensäure in Wasser und 1,0 mL/L Ameisensäure in Acetonitril als Eluenten (linearer Gradient von 10% Acetonitrile bis 95% Acetonitril).

Der logP[n] Wert wird durch LC-UV Messung im neutralen Bereich bestimmt, mit 79 mg/L Ammoniumcarbonat in Wasser und Acetonitril als Eluenten (linearer Gradient von 10% Acetonitril bis 95% Acetonitril).

Die Kalibrierung wurde mit einer homologen Reihe geradkettiger Alkan-2-one (mit 3 bis 16 Kohlenstoffatomen) mit bekannten logP Werten durchgeführt. Die Werte zwischen aufeinanderfolgender Alkanone werden durch lineare Regression bestimmt.

Die Messungen der ¹H-NMR Spektren wurden mit einem Bruker **Avance III 400 MHz** Spektrometer, ausgestattet mit einem 1,7 mm TCI Probenkopf, mit Tetramethylsilan als Standard (0,00 ppm) von Lösungen in den Lösungsmitteln CD₃CN, CDCl₃ oder d₆-DMSO durchgeführt. Alternativ wurde ein **Bruker Avance III 600** MHz Spektrometer ausgestattet mit einem 5 mm CPNMP Probenkopf oder ein Bruker **Avance NEO 600** MHz Spektrometer ausgestattet mit einem 5 mm TCI Probenkopf für die Messungen verwendet. In der Regel wurden die Messungen bei einer Probenkopftemperatur von 298 K durchgeführt. Sofern andere Messtemperaturen verwendet wurden, wird dies gesondert vermerkt.

Die NMR-Daten werden in klassischer Form (δ-Werte, Multiplettaufspaltung, Anzahl der H-Atome) aangegeben.

Das Lösungsmittel, in welchem das NMR-Spektrum aufgenommen wurde ist jeweils angegeben.

### Beispiel 1

### Synthese von 6-[5-(Ethylsulfanyl)-1-methyl-1H-imidazol-4-yl]-7-methyl-3-(trifluormethyl)-7H-imidazo[4,5-c]pyridazin:

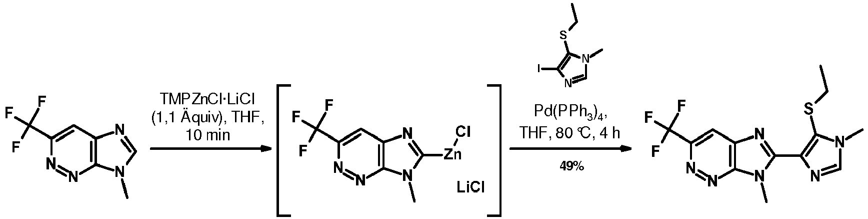

In einem trockenen, mit Argon befüllten Schlenk-Kolben, ausgestattet mit einem Magnetrührfisch und einem Septum, wurde 7-Methyl-3-(trifluormethyl)-7H-imidazo[4,5-c]pyridazin (2,00 g, 9,91 mmol) in wasserfreiem THF (15 mL) vorgelegt. Zinkchlorid-2,2,6,6-tetramethylpiperidin-1-id Lithiumchlorid Komplex (TMPZnCl·LiCl) (1,31 M in THF, 8,33 mL, 10,9 mmol) wurde tropfenweise zugegeben, und die Mischung wurde 10 min bei 25 °C gerührt. Eine Lösung von 5-(Ethylsulfanyl)-4-iod-1-methyl-1H-imidazol (2,66 g, 9,92 mmol) in wasserfreiem THF (22,5 mL) sowie Tetrakis(triphenylphosphin)palladium(0) (1,15 g, 0,991 mmol) wurden zugegeben, und anschließend wurde die Reaktionsmischung 4 Stunden bei 80 °C gerührt. Die Reaktionsmischung wurde auf 25 °C abgekühlt, mit gesättigter wässriger Ammoniumchlorid-Lösung und Natriumthiosulfat-Lösung versetzt, dreimal mit Ethylacetat extrahiert und über wasserfreiem Natriumsulfat getrocknet. Nach der Filtration wurde das Lösungsmittel im Vakuum entfernt. Das Rohprodukt wurde chromatographisch gereinigt, was 6-[5-(Ethylsulfanyl)-1-methyl-1H-imidazol-4-yl]-7-methyl-3-(trifluormethyl)-7H-imidazo[4,5-c]pyridazin (1,65 g, 49%) als weißen Feststoff ergab.

HPLC-MS: logP[a] = 2,26; logP[n] = 2,37; MH⁺: 343;

¹H-NMR (d₆-DMSO): δ 8,52 (s, 1H), 8,23 (s, 1H), 4,32 (s, 3H), 3,80 (s, 3H), 3,06 (q, 2H), 1,11 (t, 3H).

### Beispiel 2

### Synthese von 6-[2-(4-Chlorphenyl)-5-(ethylsulfanyl)-1-methyl-1H-imidazol-4-yl]-7-methyl-3-(trifluormethyl)-7H-imidazo[4,5-c]pyridazin:

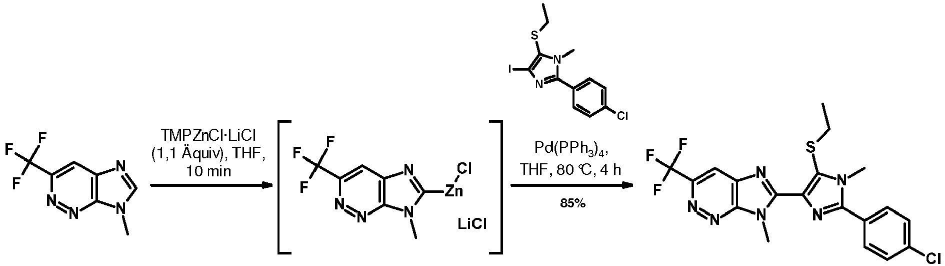

In einem trockenen, mit Argon befüllten Schlenk-Kolben, ausgestattet mit einem Magnetrührfisch und einem Septum, wurde 7-Methyl-3-(trifluormethyl)-7H-imidazo[4,5-c]pyridazin (220 mg, 1,09 mmol) in wasserfreiem THF (1,5 mL) vorgelegt. Zinkchlorid-2,2,6,6-tetramethylpiperidin-1-id Lithiumchlorid Komplex (TMPZnCl·LiCl) (1,31 M in THF, 0,914 mL, 1,09 mmol) wurde tropfenweise zugegeben, und die Mischung wurde 10 min bei 25 °C gerührt. Eine Lösung von 2-(4-Chlorphenyl)-5-(ethylsulfanyl)-4-iod-1-methyl-1H-imidazol (412 mg, 1,20 mmol) in wasserfreiem THF (2,5 mL) sowie Tetrakis(triphenylphosphin)palladium(0) (49,3 mg, 0,109 mmol) wurden zugegeben, und anschließend wurde die Reaktionsmischung 4 Stunden bei 80 °C gerührt. Die Reaktionsmischung wurde auf 25 °C abgekühlt, mit gesättigter wässriger Ammoniumchlorid-Lösung und Natriumthiosulfat-Lösung versetzt, dreimal mit Ethylacetat extrahiert und über wasserfreiem Natriumsulfat getrocknet. Nach der Filtration wurde das Lösungsmittel im Vakuum entfernt. Das Rohprodukt wurde chromatographisch gereinigt, was 6-[2-(4-Chlorphenyl)-5-(ethylsulfanyl)-1-methyl-1H-imidazol-4-yl]-7-methyl-3-(trifluormethyl)-7H-imidazo[4,5-c]pyridazin (420 mg, 85%) als weißen Feststoff ergab.

HPLC-MS: logP[a] = 4,23; logP[n] = 4,08; MH⁺: 453;

¹H-NMR (d₆-DMSO): δ 8,57 (s, 1H), 7,91 (d, 2H), 7,66 (d, 2H), 4,37 (s, 3H), 3,90 (s, 3H), 3,09 (q, 2H), 1,18 (t, 3H).

### Vergleichsbeispiel: Synthese von 6-[2-(4-Chlorphenyl)-5-(ethylsulfanyl)-1-methyl-1H-imidazol-4-yl]-7-methyl-3-(trifluormethyl)-7H-imidazo[4,5-c]pyridazin gemäß Stand der Technik (WO 2018/130443)

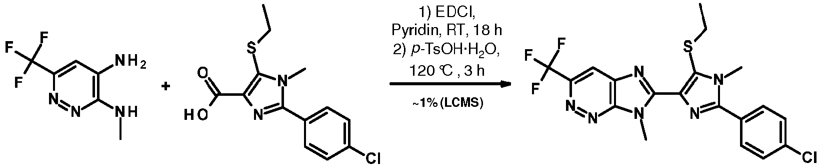

Zu einer Lösung aus 2-(4-Chlorphenyl)-5-(ethylsulfanyl)-1-methyl-1H-imidazol-4-carbonsäure (402 mg, 1,35 mmol) in Pyridin (16,1 mL) wurden N³-methyl-6-(trifluoromethyl)pyridazine-3,4-diamine (200 mg, 1,04 mmol) und 1-(3-Dimethylaminopropyl)-3-ethylcarbodiimid hydrochlorid (EDCI, 200 mg, 1,04 mmol) gegeben. Die Mischung wurde über Nacht bei 25 °C gerührt. p-Toluolsulfonsäure (179 mg, 1,04 mmol) wurde hinzugefügt und die Mischung wurde 3 Stunden bei 120 °C gerührt. Anschließend wurde das Reaktionsgemisch auf Raumtemperatur abgekühlt. Der Ansatz wurde unter Vakuum vom Lösungsmittel befreit. Das Rohprodukt enthält im HPLC-MS ca. 1% von 6-[2-(4-Chlorphenyl)-5-(ethylsulfanyl)-1-methyl-1H-imidazol-4-yl]-7-methyl-3-(trifluormethyl)-7H-imidazo[4,5-c]pyridazin.

HPLC-MS: logP[a] = 4,35; logP[n] = 4,07; MH⁺: 453;

Die Herstellung der folgenden in Tabelle 2 aufgeführten Verbindungen erfolgte analog zu 6-[2-(4-Chlorphenyl)-5-(ethylsulfanyl)-1-methyl-1H-imidazol-4-yl]-7-methyl-3-(trifluormethyl)-7H-imidazo[4,5-c]pyridazin.

**Tabelle 2:**

| Strukur | Analytik |
|---|---|
| 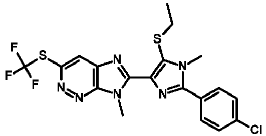 | HPLC-MS: logP[a] = 4,51; logP[n] = 4,34; MH⁺: 485; |
| | ¹H-NMR (d₆-DMSO): δ 8,48 (s, 1H), 7,90 (d, 2H), 7,66 (d, 2H), 4,33 (s, 3H), 3,89 (s, 3H), 3,09 (q, 2H), 1,18 (t, 3H). |
| 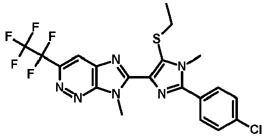 | HPLC-MS: logP[a] = 4,82; logP[n] = 4,60; MH⁺: 503; |
| | ¹H-NMR (d₆-DMSO): δ 8,59 (s, 1H), 7,91 (d, 2H), 7,66 (d, 2H), 4,37 (s, 3H), 3,90 (s, 3H), 3,10 (q, 2H), 1,18 (t, 3H). |
| 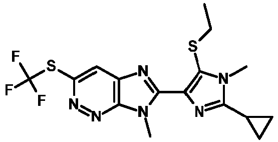 | HPLC-MS: logP[a] = 3,50; logP[n] = 3,40; MH⁺: 415; |
| | ¹H-NMR (d₆-DMSO): δ 8,405 (s, 1H), 4,24 (s, 3H), 3,82 (s, 3H), 3,00 (q, 2H), 2,20 (m, 1H), 1,11 (t, 3H), 1,05 (m, 2H), 1,01 (m, 2H). |
| 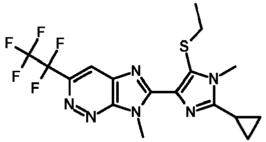 | HPLC-MS: logP[a] = 3,85; logP[n] = 3,75; MH⁺: 433; |
| | ¹H-NMR (d₆-DMSO): δ 8,51 (s, 1H), 4,28 (s, 3H), 3,83 (s, 3H), 3,02 (q, 2H), 2,21 (m, 1H), 1,12 (t, 3H), 1,05 (m, 2H), 1,01 (m, 2H). |
| 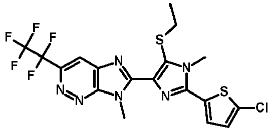 | HPLC-MS: logP[a] = 5,14; logP[n] = 4,92; MH⁺: 509; |
| | ¹H-NMR (d₆-DMSO): δ 8,59 (s, 1H), 7,62 (d, 1H), 7,31 (d, 1H), 4,35 (s, 3H), 4,01 (s, 3H), 3,07 (q, 2H), 1,16 (t, 3H). |
| 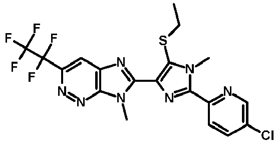 | HPLC-MS: logP[a] = 5,25; logP[n] = 4,66; MH⁺: 504; |
| | ¹H-NMR (d₆-DMSO): δ 8,80 (m, 1H), 8,60 (s, 1H), 8,30 (d, 1H), 8,16 (m, 1H), 4,41 (s, 3H), 4,22 (s, 3H), 3,115 (q, 2H), 1,16 (t, 3H). |

### Beispiel 3

### Synthese von 6-[5-(Ethylsulfanyl)-1-methyl-1H-imidazol-4-yl]-7-methyl-3-(pentafluorethyl)-7H-imidazo[4,5-c]pyridazin:

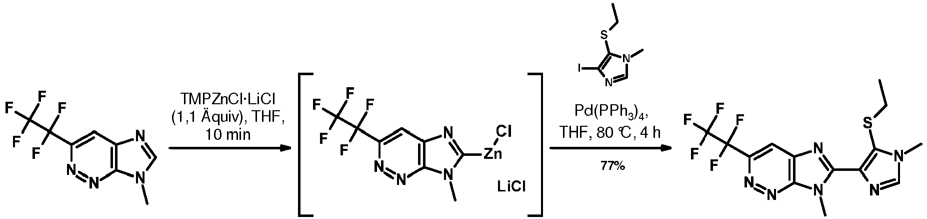

In einem trockenen, mit Argon befüllten Schlenk-Kolben, ausgestattet mit einem Magnetrührfisch und einem Septum, wurde 7-Methyl-3-(pentafluorethyl)-7H-imidazo[4,5-c]pyridazin (5,00 g, 19,8 mmol) in wasserfreiem THF (30 mL) vorgelegt. Zinkchlorid-2,2,6,6-tetramethylpiperidin-1-id Lithiumchlorid Komplex (TMPZnCl·LiCl) (1,31 M in THF, 16,7 mL, 21,8 mmol) wurde tropfenweise zugegeben, und die Mischung wurde 10 min bei 25 °C gerührt. Eine Lösung von 5-(Ethylsulfanyl)-4-iod-1-methyl-1H-imidazol (5,32 g, 19,8 mmol) in wasserfreiem THF (45 mL) sowie Tetrakis(triphenylphosphin)palladium(0) (2,29 g, 1,98 mmol) wurden zugegeben, und anschließend wurde die Reaktionsmischung 4 Stunden bei 80 °C gerührt. Die Reaktionsmischung wurde auf 25 °C abgekühlt, mit gesättigter wässriger Ammoniumchlorid-Lösung und Natriumthiosulfat-Lösung versetzt, dreimal mit Ethylacetat extrahiert und über wasserfreiem Natriumsulfat getrocknet. Nach der Filtration wurde das Lösungsmittel im Vakuum entfernt. Das Rohprodukt wurde chromatographisch gereinigt, was 6-[5-(Ethylsulfanyl)-1-methyl-1H-imidazol-4-yl]-7-methyl-3-(pentafluorethyl)-7H-imidazo[4,5-c]pyridazin (6,10 g, 77%) als weißen Feststoff ergab.

HPLC-MS: logP[a] = 2,88; logP[n] = 2,86; MH⁺: 393;

¹H-NMR (d₆-DMSO): δ 8,55 (s, 1H), 8,235 (s, 1H), 4,33 (s, 3H), 3,80 (s, 3H), 3,07 (q, 2H), 1,115 (t, 3H).

### Beispiel 4

### Synthese von 6-[5-(Ethylsulfonyl)-1-methyl-1H-imidazol-4-yl]-7-methyl-3-(pentafluorethyl)-7H-imidazo[4,5-c]pyridazin:

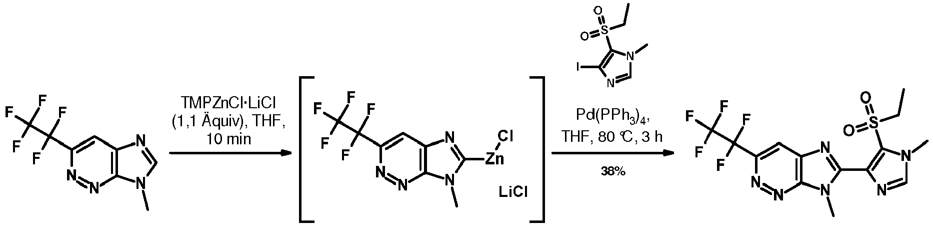

In einem trockenen, mit Argon befüllten Schlenk-Kolben, ausgestattet mit einem Magnetrührfisch und einem Septum, wurde 7-Methyl-3-(pentafluorethyl)-7H-imidazo[4,5-c]pyridazin (100 mg, 0,397 mmol) in wasserfreiem THF (1,5 mL) vorgelegt. Zinkchlorid-2,2,6,6-tetramethylpiperidin-1-id Lithiumchlorid Komplex (TMPZnCl·LiCl) (1,31 M in THF, 0,333 mL, 0,436 mmol) wurde tropfenweise zugegeben, und die Mischung wurde 10 min bei 25 °C gerührt. Eine Lösung von 5-(Ethylsulfonyl)-4-iod-1-methyl-1H-imidazol (119 mg, 0,397 mmol) in wasserfreiem THF (2,5 mL) sowie Tetrakis(triphenylphosphin)palladium(0) (45,8 mg, 0,040 mmol) wurden zugegeben, und anschließend wurde die Reaktionsmischung 3 Stunden bei 80 °C gerührt. Die Reaktionsmischung wurde auf 25 °C abgekühlt, mit gesättigter wässriger Ammoniumchlorid-Lösung und Natriumthiosulfat-Lösung versetzt, dreimal mit Ethylacetat extrahiert und über wasserfreiem Natriumsulfat getrocknet. Nach der Filtration wurde das Lösungsmittel im Vakuum entfernt. Das Rohprodukt wurde chromatographisch gereinigt, was 6-[5-(Ethylsulfonyl)-1-methyl-1H-imidazol-4-yl]-7-methyl-3-(pentafluorethyl)-7H-imidazo[4,5-c]pyridazin (68,2 mg, 38%) als weißen Feststoff ergab.

HPLC-MS: logP[a] = 2,20; logP[n] = 2,21; MH⁺: 425;

¹H-NMR (d₆-DMSO): δ 8,71 (s, 1H), 8,37 (s, 1H), 4,12 (s, 3H), 3,99 (s, 3H), 3,85 (q, 2H), 1,285 (t, 3H).

### Beispiel 5

### Synthese von 6-[2-Brom-5-(ethylsulfanyl)-1-methyl-1H-imidazol-4-yl]-7-methyl-3-(pentafluorethyl)-7H-imidazo[4,5-c]pyridazin:

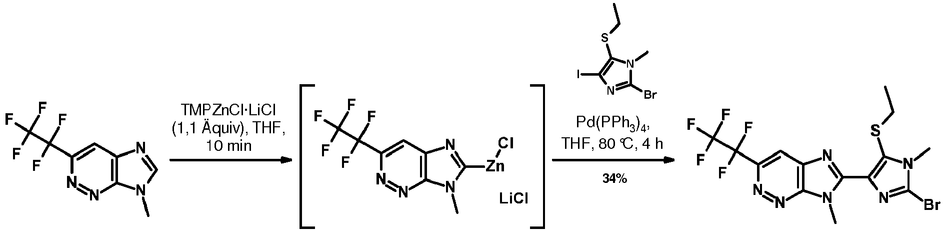

In einem trockenen, mit Argon befüllten Schlenk-Kolben, ausgestattet mit einem Magnetrührfisch und einem Septum, wurde 7-Methyl-3-(pentafluorethyl)-7H-imidazo[4,5-c]pyridazin (50,0 mg, 0,198 mmol) in wasserfreiem THF (5 mL) vorgelegt. Zinkchlorid-2,2,6,6-tetramethylpiperidin-1-id Lithiumchlorid Komplex (TMPZnCl.LiCl) (1,31 M in THF, 0,167 mL, 0,218 mmol) wurde tropfenweise zugegeben, und die Mischung wurde 10 min bei 25 °C gerührt. Eine Lösung von 2-Brom-5-(ethylsulfanyl)-4-iod-1-methyl-1H-imidazol (68,8 mg, 0,198 mmol) in wasserfreiem THF (10 mL) sowie Tetrakis(triphenylphosphin)palladium(0) (22,9 mg, 0,020 mmol) wurden zugegeben, und anschließend wurde die Reaktionsmischung 4 Stunden bei 80 °C gerührt. Die Reaktionsmischung wurde auf 25 °C abgekühlt, mit gesättigter wässriger Ammoniumchlorid-Lösung und Natriumthiosulfat-Lösung versetzt, dreimal mit Ethylacetat extrahiert und über wasserfreiem Natriumsulfat getrocknet. Nach der Filtration wurde das Lösungsmittel im Vakuum entfernt. Das Rohprodukt wurde chromatographisch gereinigt, was 6-[2-Brom-5-(ethylsulfanyl)-1-methyl-1H-imidazol-4-yl]-7-methyl-3-(pentafluorethyl)-7H-imidazo[4,5-c]pyridazin (35,8 mg, 34%) als weißen Feststoff ergab.

HPLC-MS: logP[a] = 3,76; logP[n] = 3,66; MH⁺: 471;

¹H-NMR (d₆-DMSO): δ 8,59 (s, 1H), 4,28 (s, 3H), 3,78 (s, 3H), 3,06 (q, 2H), 1,13 (t, 3H).

### Beispiel 6

### Synthese von 2-[5-(Ethylsulfanyl)-1-methyl-1H-imidazol-4-yl]-3-methyl-6-(pentafluorethyl)-3H-imidazo[4,5-c]pyridin:

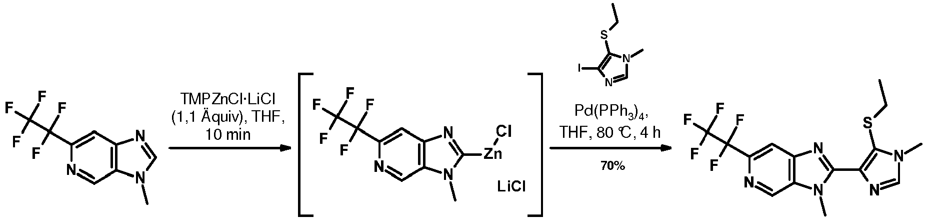

In einem trockenen, mit Argon befüllten Schlenk-Kolben, ausgestattet mit einem Magnetrührfisch und einem Septum, wurde 3-Methyl-6-(pentafluorethyl)-3H-imidazo[4,5-c]pyridazin (160 mg, 0,637 mmol) in wasserfreiem THF (5 mL) vorgelegt. Zinkchlorid-2,2,6,6-tetramethylpiperidin-1-id Lithiumchlorid Komplex (TMPZnCl·LiCl) (1,31 M in THF, 0,535 mL, 0,701 mmol) wurde tropfenweise zugegeben, und die Mischung wurde 10 min bei 25 °C gerührt. Eine Lösung von 5-(Ethylsulfanyl)-4-iod-1-methyl-1H-imidazol (171 mg, 0,637 mmol) in wasserfreiem THF (10 mL) sowie Tetrakis(triphenylphosphin)palladium(0) (73,6 mg, 0,064 mmol) wurden zugegeben, und anschließend wurde die Reaktionsmischung 4 Stunden bei 80 °C gerührt. Die Reaktionsmischung wurde auf 25 °C abgekühlt, mit gesättigter wässriger Ammoniumchlorid-Lösung und Natriumthiosulfat-Lösung versetzt, dreimal mit Ethylacetat extrahiert und über wasserfreiem Natriumsulfat getrocknet. Nach der Filtration wurde das Lösungsmittel im Vakuum entfernt. Das Rohprodukt wurde chromatographisch gereinigt, was 2-[5-(Ethylsulfanyl)-1-methyl-1H-imidazol-4-yl]-3-methyl-6-(pentafluorethyl)-3H-imidazo[4,5-c]pyridin (174 mg, 70%) als weißen Feststoff ergab.

HPLC-MS: logP[a] = 2,70; logP[n] = 2,71; MH⁺: 392;

¹H-NMR (d₆-DMSO): δ 9,15 (s, 1H), 8,18 (s, 1H), 8,16 (s, 1H), 4,20 (s, 3H), 3,78 (s, 3H), 3,02 (q, 2H), 1,09 (t, 3H).

### Beispiel 7

### Synthese von 6-Brom-2-[2-cyclopropyl-5-(ethylsulfanyl)-1-methyl-1H-imidazol-4-yl]-3-methyl-3H-imidazo[4,5-b]pyridin:

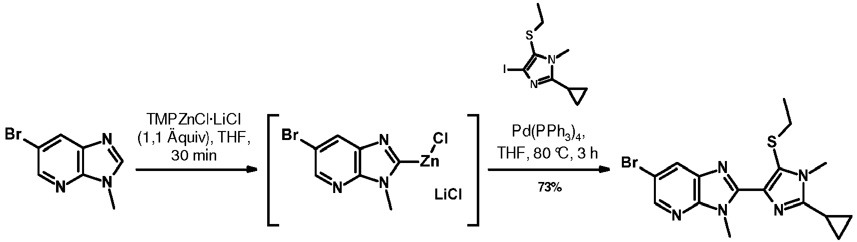

In einem trockenen, mit Argon befüllten Schlenk-Kolben, ausgestattet mit einem Magnetrührfisch und einem Septum, wurde 6-Brom-3-methyl-3H-imidazo[4,5-b]pyridin (250 mg, 1,18 mmol) in wasserfreiem THF (5 mL) vorgelegt. Zinkchlorid-2,2,6,6-tetramethylpiperidin-1-id Lithiumchlorid Komplex (TMPZnCl·LiCl) (1,31 M in THF, 0,990 mL, 1,30 mmol) wurde tropfenweise zugegeben, und die Mischung wurde 30 min bei 25 °C gerührt. Eine Lösung von 2-Cyclopropyl-5-(ethylsulfanyl)-4-iod-1-methyl-1H-imidazol (436 mg, 1,41 mmol) in wasserfreiem THF (10 mL) sowie Tetrakis(triphenylphosphin)palladium(0) (136 mg, 0,118 mmol) wurden zugegeben, und anschließend wurde die Reaktionsmischung 3 Stunden bei 80 °C gerührt. Die Reaktionsmischung wurde auf 25 °C abgekühlt, mit gesättigter wässriger Ammoniumchlorid-Lösung und Natriumthiosulfat-Lösung versetzt, dreimal mit Ethylacetat extrahiert und über wasserfreiem Natriumsulfat getrocknet. Nach der Filtration wurde das Lösungsmittel im Vakuum entfernt. Das Rohprodukt wurde chromatographisch gereinigt, was 6-Brom-2-[2-cyclopropyl-5-(ethylsulfanyl)-1-methyl-1H-imidazol-4-yl]-3-methyl-3H-imidazo[4,5-b]pyridin (350 mg, 73%) als weißen Feststoff ergab.

HPLC-MS: logP[a] = 2,92; logP[n] = 3,23; MH⁺: 392;

¹H-NMR (d₆-DMSO): δ 8,42 (d, 1H), 8,32 (d, 1H), 3,97 (s, 3H), 3,79 (s, 3H), 2,94 (q, 2H), 2,16 (m, 1H), 1,08 (t, 3H), 1,02 (m, 2H), 0,97 (m, 2H).

### Beispiel 8

### Synthese von 2-[2-Brom-5-(ethylsulfanyl)-1-methyl-1H-imidazol-4-yl]-3-methyl-6-(trifluormethyl)-3H-imidazo[4,5-c]pyridin:

### Schritt 1

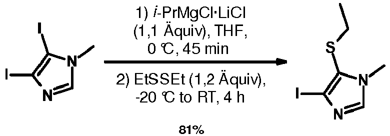

In einem trockenen, mit Argon befüllten Schlenk-Kolben, ausgestattet mit einem Magnetrührfisch und einem Septum, wurde 4,5-Diiod-1-methyl-1H-imidazol (5,00 g, 15,0 mmol) in wasserfreiem THF (35 mL) vorgelegt. Isopropylmagnesiumchlorid Lithiumchlorid Komplex (*i*-PrMgCl·LiCl) (1,3 M in THF, 12,7 mL, 16,5 mmol) wurde tropfenweise bei 0 °C zugegeben, und die Mischung wurde 45 min bei 0 °C gerührt. Diethyldisulfid (2,21 mL, 18,0 mmol) wurde tropfenweise bei -20 °C zugegeben, und anschließend wurde die Reaktionsmischung 4 Stunden bei Raumtemperatur gerührt. Die Reaktionsmischung wurde mit gesättigter wässriger Ammoniumchlorid-Lösung versetzt, mit Dichlormethan extrahiert und über wasserfreiem Natriumsulfat getrocknet. Nach der Filtration wurde das Lösungsmittel im Vakuum entfernt. Das Rohprodukt wurde chromatographisch gereinigt, was 5-(Ethylsulfanyl)-4-iod-1-methyl-1H-imidazol (2,60 g, 81%) als farbloses Öl ergab.

HPLC-MS: logP[a] = 1,61; logP[n] = 1,91; MH⁺: 269;

¹H-NMR (d₆-DMSO): δ 7,88 (s, 1H), 3,69 (s, 3H), 2,68 (q, 2H), 1,10 (t, 3H).

### Schritt 2

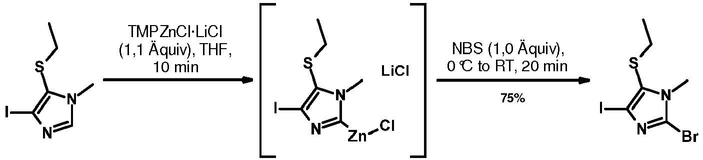

In einem trockenen, mit Argon befüllten Schlenk-Kolben, ausgestattet mit einem Magnetrührfisch und einem Septum, wurde 5-(Ethylsulfanyl)-4-iod-1-methyl-1H-imidazol (336 mg, 1,25 mmol) in wasserfreiem THF (5 mL) vorgelegt. Zinkchlorid-2,2,6,6-tetramethylpiperidin-1-id Lithiumchlorid Komplex (TMPZnCl·LiCl) (1,3 M in THF, 1,06 mL, 1,38 mmol) wurde tropfenweise zugegeben, und die Mischung wurde 10 min bei 25 °C gerührt. Eine Lösung von N-Bromsuccinimid (223 mg, 1,25 mmol) in wasserfreiem THF (7,4 mL) wurde tropfenweise bei 0 °C zugegeben und anschließend wurde die Reaktionsmischung 20 Minuten bei Raumtemperatur gerührt. Die Reaktionsmischung wurde mit gesättigter wässriger Ammoniumchlorid-Lösung und Natriumthiosulfat-Lösung versetzt, dreimal mit Ethylacetat extrahiert und über wasserfreiem Natriumsulfat getrocknet. Nach der Filtration wurde das Lösungsmittel im Vakuum entfernt. Das Rohprodukt wurde chromatographisch gereinigt, was 2-Brom-5-(ethylsulfanyl)-4-iod-1-methyl-1H-imidazol (328 mg, 75%) als weißen Feststoff ergab.

HPLC-MS: logP[a] = 2,77; logP[n] = 2,68; MH⁺: 347;

¹H-NMR (d₆-DMSO): δ 3,66 (s, 3H), 2,70 (q, 2H), 1,12 (t, 3H).

### Schritt 3

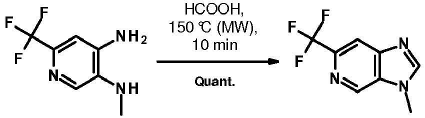

Eine Lösung von N³-Methyl-6-(trifluormethyl)pyridin-3,4-diamin (1,50 g, 7,85 mmol) in Ameisensäure (4 mL) wurde eine Stunde bei 150 °C in der Mikrowelle gerührt. Die Reaktionsmischung wurde auf 25 °C abgekühlt und das Lösungsmittel wurde im Vakuum entfernt. Das Rohprodukt wurde chromatographisch gereinigt, was 3-Methyl-6-(trifluormethyl)-3H-imidazo[4,5-c]pyridin (1,80 g, Quant.) als weißen Feststoff ergab.

HPLC-MS: logP[a] = 1,04; logP[n] = 1,10; MH⁺: 202;

¹H-NMR (d₆-DMSO): δ 9,14 (s, 1H), 8,61 (s, 1H), 8,18 (s, 1H), 4,02 (s, 3H).

### Schritt 4

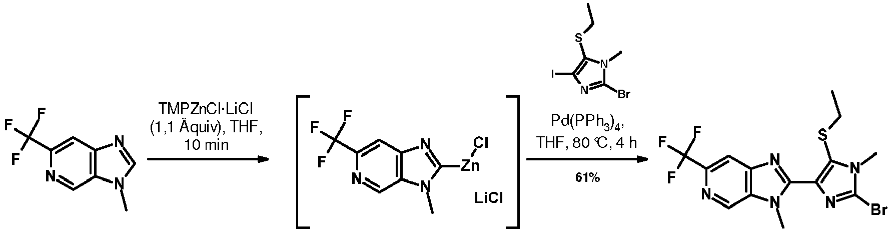

In einem trockenen, mit Argon befüllten Schlenk-Kolben, ausgestattet mit einem Magnetrührfisch und einem Septum, wurde 3-Methyl-6-(trifluormethyl)-3H-imidazo[4,5-c]pyridazin (200 mg, 0,994 mmol) in wasserfreiem THF (4 mL) vorgelegt. Zinkchlorid-2,2,6,6-tetramethylpiperidin-1-id Lithiumchlorid Komplex (TMPZnCl.LiCl) (1,18 M in THF, 0,927 mL, 1,09 mmol) wurde tropfenweise zugegeben und die Mischung wurde 10 min bei 25 °C gerührt. Eine Lösung von 2-Brom-5-(ethylsulfanyl)-4-iod-1-methyl-1H-imidazol (345 mg, 0,994 mmol) in wasserfreiem THF (9 mL) sowie Tetrakis(triphenylphosphin)palladium(0) (115 mg, 0,099 mmol) wurden zugegeben und anschließend wurde die Reaktionsmischung 4 Stunden bei 80 °C gerührt. Die Reaktionsmischung wurde auf 25 °C abgekühlt, mit gesättigter wässriger Ammoniumchlorid-Lösung und Natriumthiosulfat-Lösung versetzt, dreimal mit Ethylacetat extrahiert und über wasserfreiem Natriumsulfat getrocknet. Nach der Filtration wurde das Lösungsmittel im Vakuum entfernt. Das Rohprodukt wurde chromatographisch gereinigt, was 2-[2-Brom-5-(ethylsulfanyl)-1-methyl-1H-imidazol-4-yl]-3-methyl-6-(trifluormethyl)-3H-imidazo[4,5-c]pyridin (274 mg, 61%) als weißen Feststoff ergab.

HPLC-MS: logP[a] = 3,04; logP[n] = 2,85; MH⁺: 420;

¹H-NMR (d₆-DMSO): δ 9,15 (s, 1H), 8,18 (s, 1H), 4,15 (s, 3H), 3,76 (s, 3H), 3,00 (q, 2H), 1,11 (t, 3H).

Durch diese Methode kann 2-[2-Brom-5-(ethylsulfanyl)-1-methyl-1H-imidazol-4-yl]-3-methyl-6-(trifluormethyl)-3H-imidazo[4,5-c]pyridin aus 4,5-Diiod-1-methyl-1H-imidazol in 4 Schritten mit einer Gesamtausbeute von 37% hergestellt werden.

### Vergleichsbeispiel: Über den Weg aus dem Stand der Technik (WO 2018/130443) kann 2-[2-Brom-5-(ethylsulfanyl)-1-methyl-1H-imidazol-4-yl]-3-methyl-6-(trifluormethyl)-3H-imidazo[4,5-c]pyridin aus Ethyl-1-methyl-1H-imidazol-4-carboxylat in 4 Schritten mit einer Gesamtausbeute von 21% hergestellt werden:

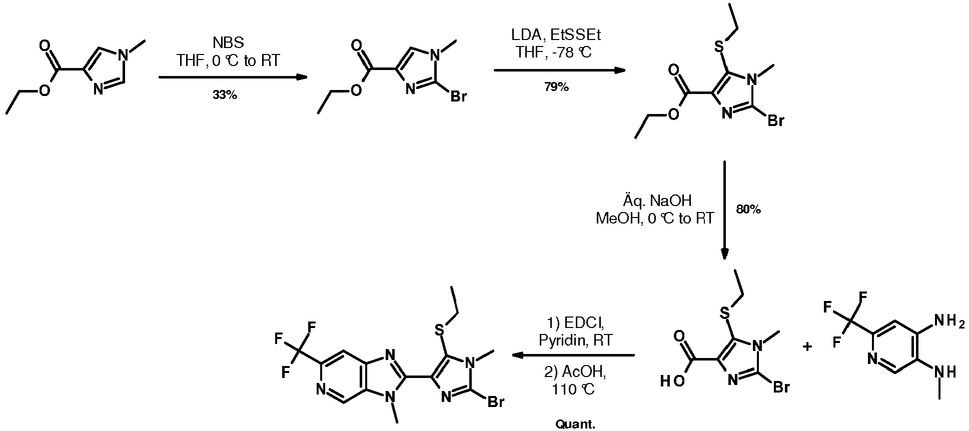

## Patentansprüche

1. Verfahren zur Herstellung von Verbindungen der Formel (II) 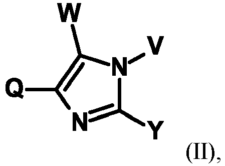
in welcher
Q für ein Strukturelement 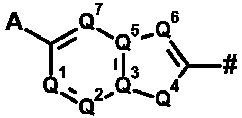
steht, wobei das Zeichen # die Bindung zum Rest des Moleküls anzeigt und
Q¹ für N oder CR⁶ steht,
Q² für N oder CR⁶ steht,
Q³ für N oder C steht,
Q⁴ für O, S, N, CR⁶ oder NR⁷ steht,
Q⁵ für N oder C steht,
Q⁶ für N oder CH steht und
Q⁷ für N oder CH steht,
wobei maximal fünf der Variablen Q¹, Q², Q³, Q⁴, Q⁵, Q⁶ und Q⁷ gleichzeitig für Stickstoff stehen und Q³ und Q⁵ nicht gleichzeitig für N stehen und
R⁶ für Wasserstoff, (C₁-C₄)Alkyl, (C₁-C₄)Halogenalkyl, (C₁-C₄)Cyanoalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht,
R⁷ für (C₁-C₄)Alkyl, (C₁-C₄)Halogenalkyl, (C₁-C₄)Cyanoalkyl, (C₁-C₄)Alkoxy-(C₁-C₄)alkyl, (C₁-C₄)Halogenalkoxy-(C₁-C₄)alkyl, (C₂-C₄)Alkenyl, (C₂-C₄)Alkenyloxy-(C₁-C₄)alkyl, (C₂-C₄)Halogenalkenyloxy-(C₁-C₄)alkyl, (C₂-C₄)Halogenalkenyl, (C₂-C₄)Cyanoalkenyl, (C₂-C₄)Alkinyl, (C₂-C₄)Alkinyloxy-(C₁-C₄)alkyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, Halogen(C₃-C₆)cycloalkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht und
A für Wasserstoff, Cyano, Halogen, (C₁-C₄)Alkyl, (C₁-C₄)Halogenalkyl, (C₂-C₄)Alkenyl, (C₂-C₄)Halogenalkenyl, (C₂-C₄)Alkinyl, (C₂-C₄)Halogenalkinyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Alkoxy, (C₁-C₄)Halogenalkoxy, (C₁-C₄)Alkoxyimino, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Alkylcarbonyl, (C₁-C₄)Halogenalkylcarbonyl, Aminocarbonyl, (C₁-C₄)Alkylaminocarbonyl, Di-(C₁-C₄)alkyl-aminocarbonyl, (C₁-C₄)Alkylsulfonylamino, (C₁-C₄)Alkylamino, Di-(C₁-C₄)Alkylamino, Aminosulfonyl, (C₁-C₄)Alkylaminosulfonyl oder Di-(C₁-C₄)alkylaminosulfonyl steht, oder
A für -O-CF₂-O- steht und gemeinsam mit Q¹ und dem Kohlenstoffatom, an das es gebunden ist, einen fünfgliedrigen Ring bildet, wobei Q¹ für Kohlenstoff steht,
W für Halogen oder S(O)ₙR⁸ steht, wobei
R⁸ für (C₁-C₆)Alkyl, (C₁-C₆)Cyanoalkyl, (C₁-C₆)Alkoxy(C₁-C₆)alkyl, (C₁-C₆)Halogenalkyl, (C₂-C₆)Alkenyl, (C₂-C₆)Halogenalkenyl, (C₂-C₆)Alkinyl, (C₂-C₆)Halogenalkinyl oder (C₃-C₈)Cycloalkyl steht und
n für 0, 1 oder 2 steht,
V für (C₁-C₆)-Alkyl, (C₁-C₆)Halogenalkyl, (C₁-C₆)Cyanoalkyl, (C₁-C₆)Alkoxy-(C₁-C₆)alkyl, (C₁-C₆)Halogenalkoxy-(C₁-C₆)alkyl, (C₂-C₆)Alkenyl, (C₂-C₆)Alkenyloxy-(C₁-C₆)alkyl, (C₂-C₆)Halogenalkenyloxy-(C₁-C₆)alkyl, (C₂-C₆)Halogenalkenyl, (C₂-C₆)Cyanoalkenyl, (C₂-C₆)Alkinyl, (C₂-C₆)Alkinyloxy-(C₁-C₆)alkyl, (C₂-C₆)Halogenalkinyloxy-(C₁-C₆)alkyl, (C₂-C₆)Halogenalkinyl, (C₂-C₆)Cyanoalkinyl, (C₃-C₈)Cycloalkyl, (C₃-C₈)Cycloalkyl-(C₃-C₈)Cycloalkyl, (C₁-C₆)Alkyl-(C₃-C₈)Cycloalkyl, Halogen(C₃-C₈)cycloalkyl, Cyano(C₃-C₈)cycloalkyl, (C₁-C₆)Alkylthio-(C₁-C₆)alkyl, (C₁-C₆)Halogenalkylthio-(C₁-C₆)alkyl, (C₁-C₆)Alkylsulfinyl-(C₁-C₆)alkyl, (C₁-C₆)Halogenalkylsulfinyl-(C₁-C₆)alkyl, (C₁-C₆)Alkylsulfonyl-(C₁-C₆)alkyl, (C₁-C₆)Halogenalkylsulfonyl-(C₁-C₆)alkyl, (C₁-C₆)Alkylcarbonyl-(C₁-C₆)alkyl, (C₁-C₆)Halogenalkylcarbonyl-(C₁-C₆)alkyl, (C₁-C₆)Alkoxycarbonyl-(C₁-C₆)alkyl oder (C₁-C₆)Halogenalkoxycarbonyl-(C₁-C₆)alkyl steht und
Y für Wasserstoff, Halogen, (C₁-C₄)Alkyl, (C₁-C₄)Halogenalkyl, (C₁-C₄)Alkoxy, (C₁-C₄)Halogenalkoxy, (C₁-C₄)Alkylthio, (C₁-C₄)Halogenalkylthio, (C₁-C₄)Alkylsulfinyl, (C₁-C₄)Halogenalkylsulfinyl, (C₁-C₄)Alkylsulfonyl, (C₁-C₄)Halogenalkylsulfonyl, SCN, (C₁-C₄)Alkylcarbonyl, (C₁-C₄)Halogenalkylcarbonyl, (C₁-C₄)Alkoxycarbonyl, (C₁-C₄)Halogenalkoxycarbonyl, Aminocarbonyl, (C₁-C₄)Alkylaminocarbonyl, Di-(C₁-C₄)alkyl-aminocarbonyl, (C₁-C₄)Halogenalkylaminocarbonyl, (C₃-C₆)Cycloalkylaminocarbonyl, Aminothiocarbonyl, (C₁-C₄)Alkylaminothiocarbonyl, Di-(C₁-C₄)alkylaminothiocarbonyl, (C₁-C₄)Halogenalkylaminothiocarbonyl, (C₃-C₆)Cycloalkylaminothiocarbonyl, Amino, (C₁-C₄)Alkylamino, (C₁-C₄)Halogenalkylamino, Di-(C₁-C₄)Alkylamino, (C₃-C₆)Cycloalkylamino, (C₁-C₄)Alkylsulfonylamino, (C₁-C₄)Alkylcarbonylamino, (C₁-C₄)Halogenalkylcarbonylamino, (C₁-C₄)Alkylcarbonyl-(C₁-C₄)alkyl-amino, (C₁-C₄)Halogenalkylcarbonyl-(C₁-C₄)alkyl-amino, (C₃-C₆)Cycloalkylcarbonylamino, (C₃-C₆)Cycloalkylcarbonyl-(C₁-C₄)alkyl-amino, (C₁-C₄)Alkylthiocarbonylamino, (C₁-C₄)Halogenalkylthiocarbonylamino, (C₁-C₄)Alkylthiocarbonyl-(C₁-C₄)alkyl-amino, (C₁-C₄)Halogenalkylthiocarbonyl-(C₁-C₄)alkyl-amino, (C₃-C₆)Cycloalkylthiocarbonylamino, (C₃-C₆)Cycloalkylthiocarbonyl-(C₁-C₄)alkyl-amino, (C₂-C₄)Alkenyl, (C₂-C₄)Halogenalkenyl, (C₂-C₄)Cyanoalkenyl, (C₃-C₆)Cycloalkyl-(C₂)alkenyl, (C₂-C₄)Alkinyl oder (C₂-C₄)Halogenalkinyl steht,
oder für jeweils gegebenenfalls einfach oder mehrfach, gleich oder verschieden substituiertes (C₃-C₆)Cycloalkyl oder (C₅-C₆)Cycloalkenyl steht, wobei als Substituenten jeweils in Frage kommen: (C₃-C₆)Cycloalkyl, (C₁-C₄)Alkyl, (C₁-C₄)Halogenalkyl, (C₁-C₄)Alkoxy, (C₁-C₄)Halogenalkoxy, Aminocarbonyl, Aminothiocarbonyl, Halogen oder Cyano,
oder für jeweils gegebenenfalls einfach oder mehrfach, gleich oder verschieden substituiertes Aryl oder Hetaryl steht, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Acetyl, Hydroxy, Amino, SCN, SF₅, Tri-(C₁-C₄)alkylsilyl, (C₃-C₆)Cycloalkyl, (C₃-C₆)Cycloalkyl-(C₃-C₆)cycloalkyl, (C₁-C₄)Alkyl-(C₃-C₆)cycloalkyl, (C₁-C₄)Halogenalkyl-(C₃-C₆)cycloalkyl, Halogen-(C₃-C₆)cycloalkyl, Cyano-(C₃-C₆)cycloalkyl, (C₁-C₄)Alkyl, (C₁-C₄)Halogenalkyl, (C₁-C₄)Cyanoalkyl, (C₁-C₄)Hydroxyalkyl, Hydroxycarbonyl-(C₁-C₄)-alkoxy, (C₁-C₄)Alkoxycarbonyl-(C₁-C₄)alkyl, (C₁-C₄)Alkoxy-(C₁-C₄)alkyl, (C₂-C₄)Alkenyl, (C₂-C₄)Halogenalkenyl, (C₂-C₄)Cyanoalkenyl, (C₃-C₆)Cycloalkyl-(C₂)alkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxycarbonyl-(C₁-C₄)alkoxy, (C₁-C₄)Alkoxy-(C₁-C₄)alkoxy, (C₁-C₄)Alkoxyimino, (C₁-C₄)Halogenalkoxyimino, (C₁-C₄)Alkylthio, (C₁-C₄)Halogenalkylthio, (C₁-C₄)Alkoxy-(C₁-C₄)alkylthio, (C₁-C₄)Alkylthio-(C₁-C₄)alkyl, (C₁-C₄)Alkylsulfinyl, (C₁-C₄)Halogenalkylsulfinyl, (C₁-C₄)Alkoxy-(C₁-C₄)alkylsulfinyl, (C₁-C₄)Alkylsulfinyl-(C₁-C₄)alkyl, (C₁-C₄)Alkylsulfonyl, (C₁-C₄)Halogenalkylsulfonyl, (C₁-C₄)Alkoxy-(C₁-C₄)alkylsulfonyl, (C₁-C₄)Alkylsulfonyl-(C₁-C₄)alkyl, (C₁-C₄)Alkylsulfonyloxy, (C₁-C₄)Halogenalkylsulfonyloxy, (C₁-C₄)Alkylcarbonyl, (C₁-C₄)Halogenalkylcarbonyl, (C₁-C₄)Alkylcarbonyloxy, (C₁-C₄)Alkoxycarbonyl, (C₁-C₄)Halogenalkoxycarbonyl, Aminocarbonyl, (C₁-C₄)Alkylaminocarbonyl, (C₁-C₄)Halogenalkylaminocarbonyl, Di-(C₁-C₄)alkyl-aminocarbonyl, (C₂-C₄)Alkenylaminocarbonyl, Di-(C₂-C₄)-alkenylaminocarbonyl, (C₃-C₆)Cycloalkylaminocarbonyl, (C₁-C₄)Alkylsulfonylamino, (C₁-C₄)Alkylamino, Di-(C₁-C₄)Alkylamino, (C₁-C₄)Halogenalkylamino, (C₃-C₆)Cycloalkylamino, Aminosulfonyl, (C₁-C₄)Alkylaminosulfonyl, Di-(C₁-C₄)alkylaminosulfonyl, (C₁-C₄)Alkylsulfoximino, Aminothiocarbonyl, (C₁-C₄)Alkylaminothiocarbonyl, Di-(C₁-C₄)alkylaminothiocarbonyl, (C₁-C₄)Halogenalkylaminothiocarbonyl, (C₃-C₆)Cycloalkylaminothiocarbonyl, (C₁-C₄)Alkylcarbonylamino, (C₁-C₄)Halogenalkylcarbonylamino, (C₁-C₄)Alkylcarbonyl-(C₁-C₄)alkyl-amino, (C₁-C₄)Halogenalkylcarbonyl-(C₁-C₄)alkyl-amino, (C₃-C₆)Cycloalkylcarbonylamino, (C₃-C₆)Cycloalkylcarbonyl-(C₁-C₄)alkyl-amino, (C₁-C₄)Alkylthiocarbonylamino, (C₁-C₄)Halogenalkylthiocarbonylamino, (C₁-C₄)Alkylthiocarbonyl-(C₁-C₄)alkyl-amino, (C₁-C₄)Halogenalkylthiocarbonyl-(C₁-C₄)alkyl-amino, (C₃-C₆)Cycloalkylthiocarbonylamino, (C₃-C₆)Cycloalkylthiocarbonyl-(C₁-C₄)alkyl-amino, Hetaryl, Oxo-Hetaryl, Halogen-Hetaryl, Halogen-Oxo-Hetaryl, Cyano-Hetaryl, Cyano-Oxo-Hetaryl, (C₁-C₄)Halogenalkyl-Hetaryl oder (C₁-C₄)Halogenalkyl-Oxo-Hetaryl,
**dadurch gekennzeichnet, dass** in einem ersten Verfahrensschritt a) eine Verbindung Q-H, in welcher Q die oben genannte Bedeutung hat,
mit einer Zink-metallorganischen Base der Struktur (NR³R⁴)-Zn-R² oder (NR³R⁴)₂-Zn, in welcher
R² für Halogen oder -O-Pivaloyl steht und
R³ und R⁴ gemeinsam eine -(CH₂)₄-, -(CH₂)₅- oder -(CH₂)₂O(CH₂)₂-Gruppe bilden, wobei jede dieser Gruppen optional durch 1, 2, 3 oder 4 R⁵-Radikale substituiert sein kann und R⁵ ausgewählt ist aus der Gruppe bestehend aus Methyl, Ethyl, n-Propyl und i-Propyl,
zu einer Verbindung der Formel (IIIa) oder der Formel (IIIb) umgesetzt wird, 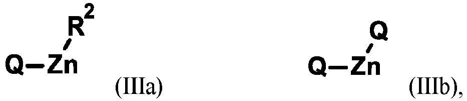
in welcher Q und R² jeweils die oben genannten Bedeutungen haben,
und diese Verbindung der Formel (IIIa) oder (IIIb) in einem zweiten Verfahrensschritt b) mit einer Verbindung der Formel (I) 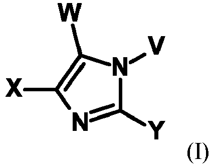
in welcher X für Halogen steht und V, W und Y jeweils die oben genannten Bedeutungen haben, in Gegenwart eines Katalysators zur Verbindung der Formel (II) umgesetzt wird.

2. Verfahren gemäß Anspruch 1, **dadurch gekennzeichnet, dass**
Q für ein Strukturelement aus der Reihe Q1 bis Q14 steht, 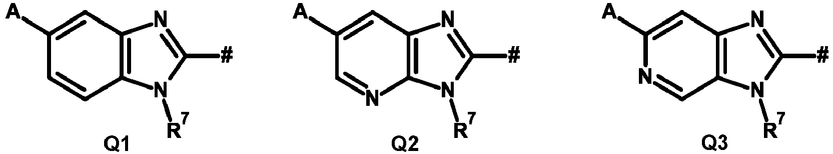 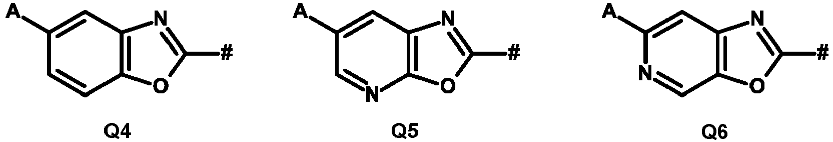 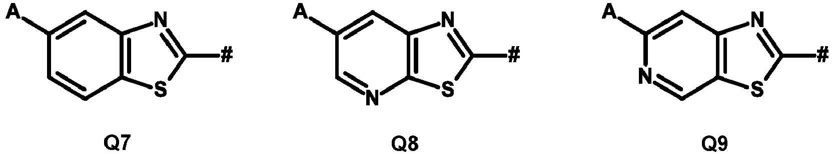 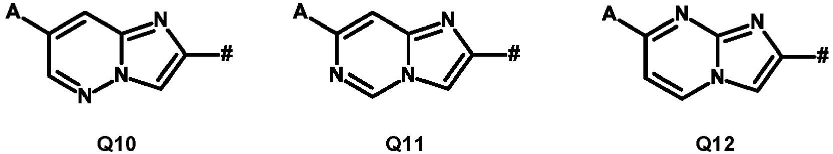 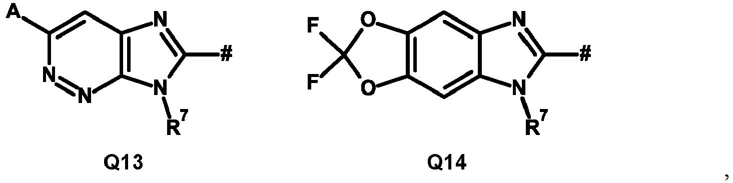 wobei
R⁷ für (C₁-C₄)Alkyl, (C₁-C₄)Halogenalkyl, (C₁-C₄)Alkoxy-(C₁-C₄)alkyl, (C₁-C₄)Halogenalkoxy-(C₁-C₄)alkyl, (C₁-C₄)Alkylthio-(C₁-C₄)alkyl, (C₁-C₄)Alkylsulfinyl-(C₁-C₄)alkyl, (C₁-C₄)Alkylsulfonyl-(C₁-C₄)alkyl oder (C₁-C₄)Alkylcarbonyl-(C₁-C₄)alkyl steht und
A für Fluor, Chlor, Brom, Fluormethyl, Difluormethyl, Trifluormethyl, Fluorethyl (CH₂CFH₂, CHFCH₃), Difluorethyl (CF₂CH₃, CH₂CHF₂, CHFCFH₂), Trifluorethyl (CH₂CF₃, CHFCHF₂, CF₂CFH₂), Tetrafluorethyl (CHFCF₃, CF₂CHF₂), Pentafluorethyl, Trifluormethoxy, Difluorchlormethoxy, Dichlorfluormethoxy, Trifluormethylthio, Trifluormethylsulfinyl oder Trifluormethylsulfonyl steht,
W für Halogen oder S(O)ₙR⁸ steht, wobei
R⁸ für (C₁-C₆)Alkyl, (C₁-C₄)Cyanoalkyl, (C₁-C₄)Alkoxy-(C₁-C₄)alkyl, (C₁-C₆)Halogenalkyl, (C₂-C₄)Alkenyl, (C₂-C₄)Halogenalkenyl, (C₂-C₄)Alkinyl, (C₂-C₄)Halogenalkinyl oder (C₃-C₆)Cycloalkyl steht und
n für 0, 1 oder 2 steht,
R² für Halogen, insbesondere Chlor, Brom oder Iod steht,
X für Halogen, insbesondere Brom oder Iod steht,
V für (C₁-C₆)-Alkyl, (C₁-C₄)Halogenalkyl, (C₁-C₄)Cyanoalkyl, (C₁-C₄)Alkoxy-(C₁-C₄)alkyl, (C₁-C₄)Halogenalkoxy-(C₁-C₄)alkyl, (C₃-C₆)Cycloalkyl, (C₃-C₆)Cycloalkyl-(C₃-C₆)Cycloalkyl, (C₁-C₄)Alkyl-(C₃-C₆)Cycloalkyl, Halogen(C₃-C₆)cycloalkyl, Cyano(C₃-C₆)cycloalkyl, (C₁-C₄)Alkylthio-(C₁-C₄)alkyl, (C₁-C₄)Halogenalkylthio-(C₁-C₄)alkyl, (C₁-C₄)Alkylsulfinyl-(C₁-C₄)alkyl, (C₁-C₄)Halogenalkylsulfinyl-(C₁-C₄)alkyl, (C₁-C₄)Alkylsulfonyl-(C₁-C₄)alkyl oder (C₁-C₄)Halogenalkylsulfonyl-(C₁-C₄)alkyl steht und
Y für Wasserstoff, Halogen, (C₁-C₄)Alkyl, (C₁-C₄)Halogenalkyl, (C₁-C₄)Alkoxy, (C₁-C₄)Halogenalkoxy, Aminocarbonyl, (C₁-C₄)Alkylaminocarbonyl, Di-(C₁-C₄)alkyl-aminocarbonyl, (C₁-C₄)Halogenalkylaminocarbonyl, (C₃-C₆)Cycloalkylaminocarbonyl, Amino, (C₁-C₄)Alkylamino, (C₁-C₄)Halogenalkylamino, Di-(C₁-C₄)Alkylamino, (C₃-C₆)Cycloalkylamino, (C₁-C₄)Alkylsulfonylamino, (C₁-C₄)Alkylcarbonylamino, (C₁-C₄)Halogenalkylcarbonylamino, (C₁-C₄)Alkylcarbonyl-(C₁-C₂)alkyl-amino, (C₁-C₄)Halogenalkylcarbonyl-(C₁-C₂)alkyl-amino, (C₃-C₆)Cycloalkylcarbonylamino, (C₃-C₆)Cycloalkylcarbonyl-(C₁-C₂)alkyl-amino, (C₂-C₄)Alkenyl, (C₂-C₄)Halogenalkenyl, (C₂-C₄)Cyanoalkenyl oder (C₃-C₆)Cycloalkyl-(C₂)alkenyl,
oder für jeweils gegebenenfalls einfach oder mehrfach, gleich oder verschieden substituiertes (C₃-C₆)Cycloalkyl oder (C₅-C₆)Cycloalkenyl steht, wobei als Substituenten jeweils in Frage kommen: (C₃-C₆)Cycloalkyl, (C₁-C₄)Alkyl, (C₁-C₄)Halogenalkyl, (C₁-C₄)Alkoxy, (C₁-C₄)Halogenalkoxy, Halogen oder Cyano,
oder für jeweils gegebenenfalls einfach oder mehrfach, gleich oder verschieden substituiertes Phenyl, Pyridyl, Pyrimidyl, Pyridazinyl, Thiophenyl, Furanyl, Pyrazolyl, Pyrrolyl, Thiazolyl, Oxazolyl oder Imidazolyl steht, wobei als Substituenten jeweils in Frage kommen: Cyano, Halogen, Nitro, Acetyl, Hydroxy, Amino, SF₅-, (C₃-C₆)Cycloalkyl, (C₁-C₄)Alkyl-(C₃-C₆)cycloalkyl, Halogen(C₃-C₆)cycloalkyl, (C₁-C₄)Alkyl, (C₁-C₄)Halogenalkyl, (C₁-C₄)Cyanoalkyl, (C₁-C₄)Hydroxyalkyl, (C₁-C₄)Alkoxy-(C₁-C₂)alkyl, (C₂-C₄)Alkenyl, (C₂-C₄)Halogenalkenyl, (C₂-C₄)Cyanoalkenyl, (C₃-C₆)Cycloalkyl-(C₂)alkenyl, (C₂-C₄)Alkinyl, (C₂-C₄)Halogenalkinyl, (C₂-C₄)Cyanoalkinyl, (C₁-C₄)Alkoxy, (C₁-C₄)Halogenalkoxy, (C₁-C₄)Cyanoalkoxy, (C₁-C₄)Alkoxy-(C₁-C₂)alkoxy, (C₁-C₄)Alkoxyimino, (C₁-C₄)Halogenalkoxyimino, (C₁-C₄)Alkylthio, (C₁-C₄)Halogenalkylthio, (C₁-C₄)Alkylthio-(C₁-C₂)alkyl, (C₁-C₄)Alkylsulfinyl, (C₁-C₄)Halogenalkylsulfinyl, (C₁-C₄)Alkylsulfonyl, (C₁-C₄)Halogenalkylsulfonyl, (C₁-C₄)Alkylsulfonyloxy, (C₁-C₄)Halogenalkylsulfonyloxy, (C₁-C₄)Alkylcarbonyl, (C₁-C₄)Halogenalkylcarbonyl, Aminocarbonyl, (C₁-C₄)Alkylaminocarbonyl, (C₁-C₄)Halogenalkylaminocarbonyl, Di-(C₁-C₄)alkyl-aminocarbonyl, (C₃-C₆)Cycloalkylaminocarbonyl, Aminothiocarbonyl, (C₁-C₄)Alkylaminothiocarbonyl, Di-(C₁-C₄)alkylaminothiocarbonyl, (C₁-C₄)Halogenalkylaminothiocarbonyl, (C₃-C₆)Cycloalkylaminothiocarbonyl, (C₁-C₄)Alkylsulfonylamino, (C₁-C₄)Alkylamino, Di-(C₁-C₄)Alkylamino, (C₁-C₄)Halogenalkylamino, (C₃-C₆)Cycloalkylamino, Aminosulfonyl, (C₁-C₄)Alkylaminosulfonyl, Di-(C₁-C₄)alkyl-aminosulfonyl, (C₁-C₄)Alkylcarbonylamino, (C₁-C₄)Halogenalkylcarbonylamino, (C₁-C₄)Alkylcarbonyl-(C₁-C₂)alkyl-amino, (C₁-C₂)Halogenalkylcarbonyl-(C₁-C₂)alkyl-amino, (C₃-C₆)Cycloalkylcarbonylamino, (C₃-C₆)Cycloalkylcarbonyl-(C₁-C₂)alkyl-amino, (C₁-C₄)Alkylthiocarbonylamino, (C₁-C₄)Halogenalkylthiocarbonylamino, (C₁-C₄)Alkylthiocarbonyl-(C₁-C₂)alkyl-amino, (C₁-C₄)Halogenalkylthiocarbonyl-(C₁-C₂)alkyl-amino, (C₃-C₆)Cycloalkylthiocarbonylamino oder (C₃-C₆)Cycloalkylthiocarbonyl-(C₁-C₂)alkyl-amino.

3. Verfahren gemäß Anspruch 1, **dadurch gekennzeichnet, dass**
Q für ein Strukturelement aus der Reihe Q2, Q3, Q4, Q10, Q11, Q13 oder Q14 steht, wobei
R⁷ für (C₁-C₄)Alkyl oder (C₁-C₄)Alkoxy-(C₁-C₄)alkyl steht und
A für Brom, Trifluormethyl, Fluorethyl (CH₂CFH₂, CHFCH₃), Difluorethyl (CF₂CH₃, CH₂CHF₂, CHFCFH₂), Trifluorethyl, (CH₂CF₃, CHFCHF₂, CF₂CFH₂), Tetrafluorethyl (CHFCF₃, CF₂CHF₂), Pentafluorethyl, Trifluormethylthio, Trifluormethylsulfinyl oder Trifluormethylsulfonyl steht,
W für Halogen oder S(O)ₙR⁸ steht, wobei
R⁸ für Methyl, Ethyl, n-Propyl oder iso-Propyl steht und
n für 0, 1 oder 2 steht,
R² für Chlor steht,
X für Brom oder Iod, insbesondere Iod steht,
V für Methyl, Ethyl, n-Propyl oder iso-Propyl steht und
Y Wasserstoff, Brom, Iod, Ethenyl, Cyclopropylethenyl, i-Propenyl, Cyclopropylethinyl, Methyl, Ethyl, Isopropyl, Cyclopropylethyl, Methoxycarbonyl, Trifluorethylaminocarbonyl, Aminocarbonyl, Methylaminocarbonyl, Dimethylaminocarbonyl, Ethylaminocarbonyl, Aminothiocarbonyl, Methylaminothiocarbonyl, Dimethylaminothiocarbonyl,
oder für jeweils gegebenenfalls einfach oder zweifach, gleich oder verschieden substituiertes Cyclopropyl, Cyclobutyl, Cyclopentyl, Cyclohexyl, Cyclopentenyl oder Cyclohexenyl steht, wobei als Substituenten jeweils in Frage kommen: Methyl, Ethyl, n-Propyl, i-Propyl, cyclo-Propyl, Difluormethyl, Trifluormethyl, Cyano, Fluor oder Chlor,
oder für jeweils gegebenenfalls einfach, zweifach oder dreifach, gleich oder verschieden substituiertes Phenyl, Pyridin-2-yl, Pyridin-3-yl, Pyridin-4-yl, Pyrimidin-5-yl, Pyridazin-3-yl, Pyridazin-4-yl, Thien-2-yl, Thien-3-yl, 1,3-Thiazol-5-yl, 1H-Imidazol-1-yl, 1H-Imidazol-2-yl, 1H-Imidazol-5-yl, 1H-Pyrazol-1-yl, 1H-Pyrazol-3-yl, 1H-Pyrazol-4-yl, 1H-Pyrazol-5-yl, 1H-Pyrrol-2-yl, 1H-Pyrrol-3-yl oder 1-Cyclohexenyl steht, wobei als Substituenten jeweils in Frage kommen: Cyano, Fluor, Chlor, Methyl, Cyclopropyl, Cyanomethyl, Cyanoisopropyl, Cyanocylopropyl, Trifluormethyl, Trifluorethyl oder Aminocarbonyl.

4. Verfahren gemäß Anspruch 1, **dadurch gekennzeichnet, dass**
Q für das Strukturelement Q2, Q3 oder Q13 steht, wobei
R⁷ für Methyl, Ethyl, n-Propyl oder iso-Propyl steht, insbesondere für Methyl und
A für Brom, Trifluormethyl, Pentafluorethyl oder Trifluormethylthio steht,
W für S(O)ₙR⁸ steht, wobei
R⁸ für Ethyl steht und
n für 0 oder 2 steht,
R² für Chlor steht,
X für Iod steht,
V für Methyl steht und
Y für Wasserstoff, Brom, Cyclopropyl, para-Chlorphenyl, 5-Chlorthien-2-yl oder 5-Chlor-2-pyridin steht.

5. Verfahren gemäß Anspruch 1, **dadurch gekennzeichnet, dass** Q für Q3 steht und R⁷, A, W, R², X, V und Y die in Anspruch 1 oder die in Anspruch 2 oder die in Anspruch 3 oder die in Anspruch 4 angegebenen Bedeutungen haben.

6. Verfahren gemäß Anspruch 1, **dadurch gekennzeichnet, dass** Q für Q13 steht und R⁷, A, W, R², X, V und Y die in Anspruch 1 oder die in Anspruch 2 oder die in Anspruch 3 oder die in Anspruch 4 angegebenen Bedeutungen haben.

7. Verfahren gemäß Anspruch 1, **dadurch gekennzeichnet, dass** W für S(O)ₙR⁸ steht und Q, n, R⁷, R⁸, A, R², X, V und Y die in Anspruch 1 oder die in Anspruch 2 oder die in Anspruch 3 oder die in Anspruch 4 angegebenen Bedeutungen haben.

8. Verfahren gemäß einem der Ansprüche 1 bis 4, **dadurch gekennzeichnet, dass** R³ und R⁴ gemeinsam eine -(CH₂)₅-Gruppe bilden, die durch 4 Methylgruppen substituiert ist.

9. Verfahren gemäß einem der Ansprüche 1 bis 8, **dadurch gekennzeichnet, dass** es sich bei der Zink-metallorganischen Base um eine Verbindung der Formel (V) handelt
(V) (TMP)ₓ ZnCl₂₋ₓ,
worin x für die Zahl 1 oder 2 steht.

10. Verfahren gemäß einem der Ansprüche 1 bis 9, **dadurch gekennzeichnet, dass** die Zinkmetallorganische Base in Verbindung mit einem Alkali- oder Erdalkalihalogenid, vorzugsweise Lithiumchlorid oder Magnesiumchlorid, vorliegt.

11. Verfahren gemäß einem der Ansprüche 1 bis 10, **dadurch gekennzeichnet, dass** die Zinkmetallorganische Base in einer Gesamtmenge von 0,5 bis 5,0 Äquivalenten, bezogen auf die Verbindung Q-H, eingesetzt wird.

12. Verfahren gemäß einem der Ansprüche 1 bis 11, **dadurch gekennzeichnet, dass** die Verbindung der Formel (I) in einer Gesamtmenge von 0,5 bis 10 Äquivalenten, bezogen auf die Verbindung Q-H, eingesetzt wird.

13. Verfahren gemäß einem der Ansprüche 1 bis 12, **dadurch gekennzeichnet, dass** es sich bei dem Katalysator um eine Palladiumverbindung handelt.

14. Verfahren gemäß einem der Ansprüche 1 bis 13, **dadurch gekennzeichnet, dass** es sich bei dem Katalysator um Tetrakis(triphenylphosphine)palladium(0) handelt.

15. Verfahren gemäß einem der Ansprüche 1 bis 14, **dadurch gekennzeichnet, dass** es in Gegenwart eines Lösungsmittels durchgeführt wird, welches ausgewählt ist aus der Gruppe bestehend aus Tetrahydrofuran (THF), 1,4-Dioxan, Diethylether, Diglyme, Methyltertbutylether (MTBE), tert-Amylmethylether (TAME), 2-Methyl-THF, Toluol, Xylole, Mesitylen, Ethylencarbonat, Propylencarbonat, N,N-Dimethylacetamid, N,N-Dimethylformamid (DMF), N-Methylpyrrolidon (NMP), N-Ethyl-2-pyrrolidon (NEP), N-Butyl-2-pyrrolidon (NBP); N,N'-Dimethylpropylenharnstoff (DMPU), Halogenkohlenwasserstoff, aromatischer Kohlenwasserstoff, Chlorkohlenwasserstoff, Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, 1,2-Dichlorbenzol, Chlortoluol, Trichlorbenzol; 4-Methoxybenzol, fluoriertes Aliphat, fluorierter Aromat, Trichlortrifluorethan, Benzotrifluorid und 4-Chlorbenzotrifluorid, oder einer Mischung aus mindestens zwei dieser Lösungsmittel untereinander.

16. Verfahren gemäß Anspruch 15, **dadurch gekennzeichnet, dass** das Lösungsmittel THF oder N,N-Dimethylformamid (DMF) ist.

17. Verfahren gemäß einem der Ansprüche 1 bis 16, **dadurch gekennzeichnet, dass** Verfahrensschritt a) bei einer Temperatur zwischen 0 °C und 80 °C durchgeführt wird.

18. Verfahren gemäß einem der Ansprüche 1 bis 16, **dadurch gekennzeichnet, dass** Verfahrensschritt b) bei einer Temperatur zwischen 40 °C und 90 °C durchgeführt wird.

## Claims

1. Process for preparing compounds of the formula (II) 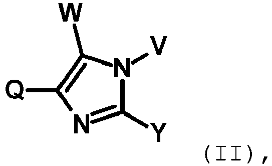
in which
Q represents a structural element 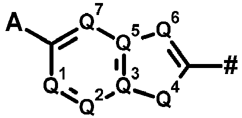
where the symbol # indicates the bond to the rest of the molecule and
Q¹ represents N or CR⁶,
Q² represents N or CR⁶,
Q³ represents N or C,
Q⁴ represents O, S, N, CR⁶ or NR⁷,
Q⁵ represents N or C,
Q⁶ represents N or CH and
Q⁷ represents N or CH,
where at most five of the variables Q¹, Q², Q³, Q⁴, Q⁵, Q⁶ and Q⁷ simultaneously represent nitrogen and Q³ and Q⁵ do not simultaneously represent N and
R⁶ represents hydrogen, (C₁-C₄)-alkyl, (C₁-C₄)-haloalkyl, (C₁-C₄)-cyanoalkyl, (C₁-C₄)-alkoxy-(C₁-C₄)-alkyl, (C₁-C₄)-haloalkoxy- (C₁-C₄)-alkyl, (C₂-C₄)-alkenyl, (C₂-C₄)-alkenyloxy- (C₁-C₄)-alkyl, (C₂-C₄)-haloalkenyloxy-(C₁-C₄)-alkyl, (C₂-C₄)-haloalkenyl, (C₂-C₄)-cyanoalkenyl, (C₂-C₄)-alkynyl, (C₂-C₄)-alkynyloxy-(C₁-C₄)-alkyl, (C₂-C₄)-haloalkynyl, (C₃-C₆)-cycloalkyl, (C₃-C₆)-cycloalkyl- (C₃-C₆)-cycloalkyl, (C₁-C₄) -alkyl- (C₃-C₆)-cycloalkyl, halo-(C₃-C₆)-cycloalkyl, (C₁-C₄) -alkylthio- (C₁-C₄) -alkyl, (C₁-C₄)-alkylsulfinyl- (C₁-C₄)-alkyl, (C₁-C₄)-alkylsulfonyl- (C₁-C₄)-alkyl or (C₁-C₄)-alkylcarbonyl- (C₁-C₄)-alkyl,
R⁷ represents (C₁-C₄) -alkyl, (C₁-C₄)-haloalkyl, (C₁-C₄)-cyanoalkyl, (C₁-C₄)-alkoxy- (C₁-C₄)-alkyl, (C₁-C₄)-haloalkoxy- (C₁-C₄)-alkyl, (C₂-C₄)-alkenyl, (C₂-C₄)-alkenyloxy- (C₁-C₄)-alkyl, (C₂-C₄)-haloalkenyloxy-(C₁-C₄) -alkyl, (C₂-C₄)-haloalkenyl, (C₂-C₄)-cyanoalkenyl, (C₂-C₄)-alkynyl, (C₂-C₄)-alkynyloxy-(C₁-C₄)-alkyl, (C₂-C₄)-haloalkynyl, (C₃-C₆)-cycloalkyl, (C₃-C₆)-cycloalkyl- (C₃-C₆)-cycloalkyl, (C₁-C₄) -alkyl- (C₃-C₆)-cycloalkyl, halo-(C₃-C₆)-cycloalkyl, (C₁-C₄)-alkylthio- (C₁-C₄)-alkyl, (C₁-C₄)-alkylsulfinyl-(C₁-C₄)-alkyl, (C₁-C₄)-alkylsulfonyl-(C₁-C₄)-alkyl or (C₁-C₄)-alkylcarbonyl- (C₁-C₄)-alkyl and
A represents hydrogen, cyano, halogen, (C₁-C₄)-alkyl, (C₁-C₄) -haloalkyl, (C₂-C₄)-alkenyl, (C₂-C₄)-haloalkenyl, (C₂-C₄)-alkynyl, (C₂-C₄)-haloalkynyl, (C₃-C₆)-cycloalkyl, (C₃-C₆)-cycloalkyl- (C₃-C₆)-cycloalkyl, (C₁-C₄)-alkyl- (C₃-C₆)-cycloalkyl, (C₁-C₄)-alkoxy, (C₁-C₄) -haloalkoxy, (C₁-C₄) -alkoxyimino, (C₁-C₄) -alkylthio, (C₁-C₄) -haloalkylthio, (C₁-C₄)-alkylsulfinyl, (C₁-C₄)-haloalkylsulfinyl, (C₁-C₄)-alkylsulfonyl, (C₁-C₄)-haloalkylsulfonyl, (C₁-C₄)-alkylsulfonyloxy, (C₁-C₄) -alkylcarbonyl, (C₁-C₄)-haloalkylcarbonyl, aminocarbonyl, (C₁-C₄)-alkylaminocarbonyl, di-(C₁-C₄)-alkylaminocarbonyl, (C₁-C₄)-alkylsulfonylamino, (C₁-C₄)-alkylamino, di-(C₁-C₄) -alkylamino, aminosulfonyl, (C₁-C₄)-alkylaminosulfonyl or di-(C₁-C₄)-alkylaminosulfonyl, or
A represents -O-CF₂-O- and, together with Q¹ and the carbon atom to which it is attached, forms a five-membered ring where Q¹ represents carbon,
W represents halogen or S(O)ₙR⁸, where
R⁸ represents (C₁-C₆)-alkyl, (C₁-C₆)-cyanoalkyl, (C₁-C₆)-alkoxy- (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl or (C₃-C₈)-cycloalkyl and
n represents 0, 1 or 2,
V represents (C₁-C₆)-alkyl, (C₁-C₆)-haloalkyl, (C₁-C₆)-cyanoalkyl, (C₁-C₆)-alkoxy- (C₁-C₆)-alkyl, (C₁-C₆)-haloalkoxy- (C₁-C₆)-alkyl, (C₂-C₆)-alkenyl, (C₂-C₆)-alkenyloxy- (C₁-C₆)-alkyl, (C₂-C₆)-haloalkenyloxy-(C₁-C₆)-alkyl, (C₂-C₆)-haloalkenyl, (C₂-C₆)-cyanoalkenyl, (C₂-C₆)-alkynyl, (C₂-C₆)-alkynyloxy- (C₁-C₆)-alkyl, (C₂-C₆)-haloalkynyloxy-(C₁-C₆)-alkyl, (C₂-C₆)-haloalkynyl, (C₂-C₆)-cyanoalkynyl, (C₃-C₈)-cycloalkyl, (C₃-C₈)-cycloalkyl- (C₃-C₈)-cycloalkyl, (C₁-C₆)-alkyl-(C₃-C₈)-cycloalkyl, halo-(C₃-C₈)-cycloalkyl, cyano-(C₃-C₈)-cycloalkyl, (C₁-C₆)-alkylthio- (C₁-C₆)-alkyl, (C₁-C₆)-haloalkylthio- (C₁-C₆)-alkyl, (C₁-C₆)-alkylsulfinyl-(C₁-C₆)-alkyl, (C₁-C₆)-haloalkylsulfinyl-(C₁-C₆)-alkyl, (C₁-C₆)-alkylsulfonyl- (C₁-C₆)-alkyl, (C₁-C₆)-haloalkylsulfonyl- (C₁-C₆)-alkyl, (C₁-C₆)-alkylcarbonyl- (C₁-C₆)-alkyl, (C₁-C₆)-haloalkylcarbonyl-(C₁-C₆)-alkyl, (C₁-C₆)-alkoxycarbonyl-(C₁-C₆)-alkyl or (C₁-C₆)-haloalkoxycarbonyl-(C₁-C₆)-alkyl and
Y represents hydrogen, halogen, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-haloalkylthio, (C₁-C₄)-alkylsulfinyl, (C₁-C₄)-haloalkylsulfinyl, (C₁-C₄)-alkylsulfonyl, (C₁-C₄)-haloalkylsulfonyl, SCN, (C₁-C₄)-alkylcarbonyl, (C₁-C₄)-haloalkylcarbonyl, (C₁-C₄)-alkoxycarbonyl, (C₁-C₄)-haloalkoxycarbonyl, aminocarbonyl, (C₁-C₄) -alkylaminocarbonyl, di-(C₁-C₄)-alkylaminocarbonyl, (C₁-C₄)-haloalkylaminocarbonyl, (C₃-C₆)-cycloalkylaminocarbonyl, aminothiocarbonyl, (C₁-C₄) -alkylaminothiocarbonyl, di-(C₁-C₄)-alkylaminothiocarbonyl, (C₁-C₄)-haloalkylaminothiocarbonyl, (C₃-C₆)-cycloalkylaminothiocarbonyl, amino, (C₁-C₄)-alkylamino, (C₁-C₄)-haloalkylamino, di-(C₁-C₄)-alkylamino, (C₃-C₆)-cycloalkylamino, (C₁-C₄)-alkylsulfonylamino, (C₁-C₄)-alkylcarbonylamino, (C₁-C₄)-haloalkylcarbonylamino, (C₁-C₄) -alkylcarbonyl- (C₁-C₄)-alkylamino, (C₁-C₄)-haloalkylcarbonyl-(C₁-C₄)-alkylamino, (C₃-C₆)-cycloalkylcarbonylamino, (C₃-C₆)-cycloalkylcarbonyl- (C₁-C₄)-alkylamino, (C₁-C₄)-alkylthiocarbonylamino, (C₁-C₄)-haloalkylthiocarbonylamino, (C₁-C₄)-alkylthiocarbonyl- (C₁-C₄)-alkylamino, (C₁-C₄)-haloalkylthiocarbonyl-(C₁-C₄)-alkylamino, (C₃-C₆)-cycloalkylthiocarbonylamino, (C₃-C₆)-cycloalkylthiocarbonyl-(C₁-C₄)-alkylamino, (C₂-C₄)-alkenyl, (C₂-C₄)-haloalkenyl, (C₂-C₄)-cyanoalkenyl, (C₃-C₆)-cycloalkyl-(C₂)-alkenyl, (C₂-C₄)-alkynyl or (C₂-C₄)-haloalkynyl,
or represents (C₃-C₆)-cycloalkyl or (C₅-C₆)-cycloalkenyl, each of which is optionally mono- or polysubstituted by identical or different substituents, possible substituents being in each case: (C₃-C₆)-cycloalkyl, (C₁-C₄)-alkyl, (C₁-C₄)-haloalkyl, (C₁-C₄)-alkoxy, (C₁-C₄)-haloalkoxy, aminocarbonyl, aminothiocarbonyl, halogen or cyano,
or represents aryl or hetaryl, each of which is optionally mono- or polysubstituted by identical or different substituents, where (in the case of hetaryl) optionally at least one carbonyl group may be present and where possible substituents in each case are as follows: cyano, carboxyl, halogen, nitro, acetyl, hydroxy, amino, SCN, SF₅, tri-(C₁-C₄)-alkylsilyl, (C₃-C₆)-cycloalkyl, (C₃-C₆)-cycloalkyl- (C₃-C₆)-cycloalkyl, (C₁-C₄) -alkyl- (C₃-C₆)-cycloalkyl, (C₁-C₄)-haloalkyl-(C₃-C₆)-cycloalkyl, halo-(C₃-C₆)-cycloalkyl, cyano-(C₃-C₆)-cycloalkyl, (C₁-C₄)-alkyl, (C₁-C₄) -haloalkyl, (C₁-C₄) -cyanoalkyl, (C₁-C₄) -hydroxyalkyl, hydroxycarbonyl- (C₁-C₄)-alkoxy, (C₁-C₄)-alkoxycarbonyl- (C₁-C₄) -alkyl, (C₁-C₄)-alkoxy-(C₁-C₄)-alkyl, (C₂-C₄)-alkenyl, (C₂-C₄)-haloalkenyl, (C₂-C₄)-cyanoalkenyl, (C₃-C₆)-cycloalkyl- (C₂)-alkenyl, (C₂-C₄)-alkynyl, (C₂-C₄)-haloalkynyl, (C₂-C₄)-cyanoalkynyl, (C₁-C₄) -alkoxy, (C₁-C₄) -haloalkoxy, (C₁-C₄)-cyanoalkoxy, (C₁-C₄)-alkoxycarbonyl-(C₁-C₄)-alkoxy, (C₁-C₄) -alkoxy- (C₁-C₄) -alkoxy, (C₁-C₄)-alkoxyimino, (C₁-C₄) -haloalkoxyimino, (C₁-C₄)-alkylthio, (C₁-C₄) -haloalkylthio, (C₁-C₄)-alkoxy-(C₁-C₄)-alkylthio, (C₁-C₄) -alkylthio- (C₁-C₄) -alkyl, (C₁-C₄)-alkylsulfinyl, (C₁-C₄) -haloalkylsulfinyl, (C₁-C₄)-alkoxy-(C₁-C₄)-alkylsulfinyl, (C₁-C₄)-alkylsulfinyl-(C₁-C₄) -alkyl, (C₁-C₄) -alkylsulfonyl, (C₁-C₄)-haloalkylsulfonyl, (C₁-C₄)-alkoxy-(C₁-C₄)-alkylsulfonyl, (C₁-C₄)-alkylsulfonyl-(C₁-C₄)-alkyl, (C₁-C₄) -alkylsulfonyloxy, (C₁-C₄)-haloalkylsulfonyloxy, (C₁-C₄)-alkylcarbonyl, (C₁-C₄)-haloalkylcarbonyl, (C₁-C₄)-alkylcarbonyloxy, (C₁-C₄)-alkoxycarbonyl, (C₁-C₄)-haloalkoxycarbonyl, aminocarbonyl, (C₁-C₄)-alkylaminocarbonyl, (C₁-C₄)-haloalkylaminocarbonyl, di-(C₁-C₄)-alkylaminocarbonyl, (C₂-C₄)-alkenylaminocarbonyl, di-(C₂-C₄)-alkenylaminocarbonyl, (C₃-C₆)-cycloalkylaminocarbonyl, (C₁-C₄)-alkylsulfonylamino, (C₁-C₄) -alkylamino, di-(C₁-C₄) -alkylamino, (C₁-C₄)-haloalkylamino, (C₃-C₆)-cycloalkylamino, aminosulfonyl, (C₁-C₄)-alkylaminosulfonyl, di-(C₁-C₄)-alkylaminosulfonyl, (C₁-C₄)-alkylsulfoximino, aminothiocarbonyl, (C₁-C₄)-alkylaminothiocarbonyl, di-(C₁-C₄) -alkylaminothiocarbonyl, (C₁-C₄)-haloalkylaminothiocarbonyl, (C₃-C₆)-cycloalkylaminothiocarbonyl, (C₁-C₄)-alkylcarbonylamino, (C₁-C₄)-haloalkylcarbonylamino, (C₁-C₄) -alkylcarbonyl- (C₁-C₄) -alkylamino, (C₁-C₄)-haloalkylcarbonyl-(C₁-C₄) -alkylamino, (C₃-C₆)-cycloalkylcarbonylamino, (C₃-C₆)-cycloalkylcarbonyl-(C₁-C₄) -alkylamino, (C₁-C₄) -alkylthiocarbonylamino, (C₁-C₄) -haloalkylthiocarbonylamino, (C₁-C₄)-alkylthiocarbonyl- (C₁-C₄) -alkylamino, (C₁-C₄)-haloalkylthiocarbonyl-(C₁-C₄)-alkylamino, (C₃-C₆)-cycloalkylthiocarbonylamino, (C₃-C₆)-cycloalkylthiocarbonyl-(C₁-C₄)-alkylamino, hetaryl, oxohetaryl, halohetaryl, halooxohetaryl, cyanohetaryl, cyanooxohetaryl, (C₁-C₄)-haloalkylhetaryl or (C₁-C₄)-haloalkyloxohetaryl,
**characterized in that**, in a first process step a), a compound Q-H in which Q is as defined above is reacted with an organozinc base of the structure (NR³R⁴)-Zn-R² or (NR³R⁴)₂-Zn in which
R² represents halogen or -O-pivaloyl and
R³ and R⁴ together form a -(CH₂)₄-, -(CH₂)₅- or - (CH₂)₂O(CH₂)₂- group, where each of these groups may optionally be substituted by 1, 2, 3 or 4 R⁵ radicals and R⁵ is selected from the group consisting of methyl, ethyl, n-propyl and i-propyl,
to give a compound of the formula (IIIa) or the formula (IIIb) 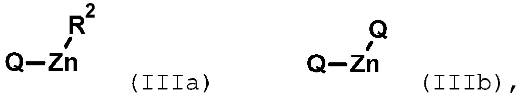
in which Q and R² each have the definitions given above, and this compound of the formula (IIIa) or (IIIb) is reacted in a second process step b) with a compound of the formula (I) 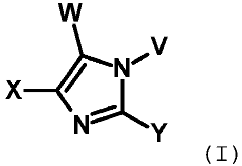
in which X represents halogen and V, W and Y each have the meanings mentioned above, in the presence of a catalyst, to give the compound of the formula (II).

2. Process according to Claim 1, **characterized in that**
Q represents a structural element from the group of Q1 to Q14, 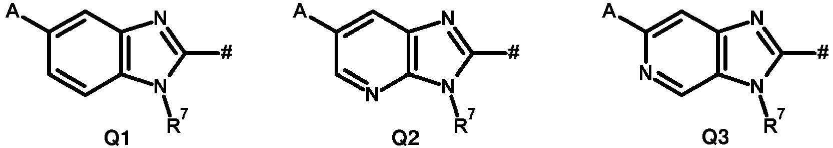 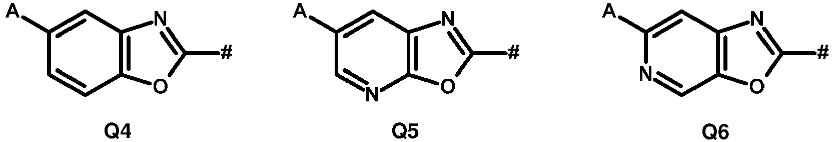 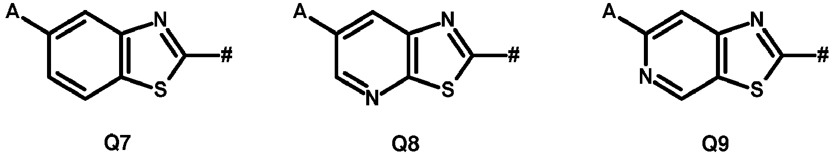 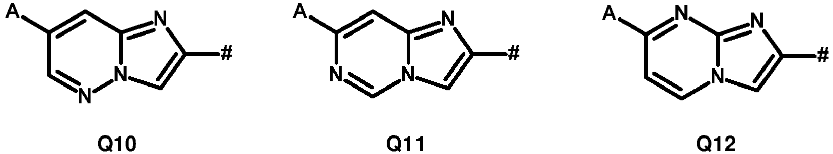 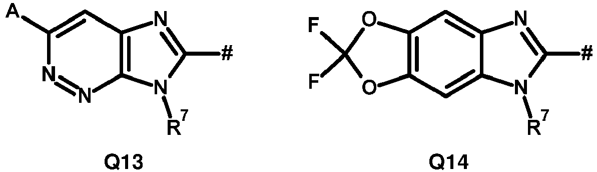
, where
R⁷ represents (C₁-C₄) -alkyl, (C₁-C₄) -haloalkyl, (C₁-C₄)-alkoxy- (C₁-C₄) -alkyl, (C₁-C₄)-haloalkoxy-(C₁-C₄)-alkyl, (C₁-C₄) -alkylthio- (C₁-C₄) -alkyl, (C₁-C₄)-alkylsulfinyl-(C₁-C₄)-alkyl, (C₁-C₄)-alkylsulfonyl-(C₁-C₄) -alkyl or (C₁-C₄) -alkylcarbonyl- (C₁-C₄) -alkyl and
A represents fluorine, chlorine, bromine, fluoromethyl, difluoromethyl, trifluoromethyl, fluoroethyl (CH₂CFH₂, CHFCH₃) , difluoroethyl (CF₂CH₃, CH₂CHF₂, CHFCFH₂), trifluoroethyl (CH₂CF₃, CHFCHF₂, CF₂CFH₂), tetrafluoroethyl (CHFCF₃, CF₂CHF₂), pentafluoroethyl, trifluoromethoxy, difluorochloromethoxy, dichlorofluoromethoxy, trifluoromethylthio, trifluoromethylsulfinyl or trifluoromethylsulfonyl,
W represents halogen or S(O)ₙR⁸, where
R⁸ represents (C₁-C₆)-alkyl, (C₁-C₄) -cyanoalkyl, (C₁-C₄)-alkoxy- (C₁-C₄) -alkyl, (C₁-C₆)-haloalkyl, (C₂-C₄)-alkenyl, (C₂-C₄)-haloalkenyl, (C₂-C₄)-alkynyl, (C₂-C₄)-haloalkynyl or (C₃-C₆)-cycloalkyl and
n represents 0, 1 or 2,
R² represents halogen, in particular chlorine, bromine or iodine,
X represents halogen, in particular bromine or iodine,
V represents (C₁-C₆)-alkyl, (C₁-C₄)-haloalkyl, (C₁-C₄)-cyanoalkyl, (C₁-C₄)-alkoxy- (C₁-C₄)-alkyl, (C₁-C₄)-haloalkoxy- (C₁-C₄) -alkyl, (C₃-C₆)-cycloalkyl, (C₃-C₆)-cycloalkyl- (C₃-C₆)-cycloalkyl, (C₁-C₄)-alkyl-(C₃-C₆)-cycloalkyl, halo- (C₃-C₆)-cycloalkyl, cyano-(C₃-C₆)-cycloalkyl, (C₁-C₄)-alkylthio- (C₁-C₄)-alkyl, (C₁-C₄)-haloalkylthio-(C₁-C₄)-alkyl, (C₁-C₄)-alkylsulfinyl-(C₁-C₄) -alkyl, (C₁-C₄)-haloalkylsulfinyl-(C₁-C₄)-alkyl, (C₁-C₄)-alkylsulfonyl-(C₁-C₄)-alkyl or (C₁-C₄)-haloalkylsulfonyl-(C₁-C₄)-alkyl and
Y represents hydrogen, halogen, (C₁-C₄)-alkyl, (C₁-C₄)-haloalkyl, (C₁-C₄)-alkoxy, (C₁-C₄)-haloalkoxy, aminocarbonyl, (C₁-C₄) -alkylaminocarbonyl, di-(C₁-C₄)-alkylaminocarbonyl, (C₁-C₄)-haloalkylaminocarbonyl, (C₃-C₆)-cycloalkylaminocarbonyl, amino, (C₁-C₄)-alkylamino, (C₁-C₄)-haloalkylamino, di-(C₁-C₄)-alkylamino, (C₃-C₆)-cycloalkylamino, (C₁-C₄)-alkylsulfonylamino, (C₁-C₄)-alkylcarbonylamino, (C₁-C₄)-haloalkylcarbonylamino, (C₁-C₄)-alkylcarbonyl-(C₁-C₂)-alkylamino, (C₁-C₄)-haloalkylcarbonyl-(C₁-C₂)-alkylamino, (C₃-C₆)-cycloalkylcarbonylamino, (C₃-C₆)-cycloalkylcarbonyl- (C₁-C₂)-alkylamino, (C₂-C₄)-alkenyl, (C₂-C₄)-haloalkenyl, (C₂-C₄)-cyanoalkenyl or (C₃-C₆)-cycloalkyl- (C₂)-alkenyl,
or represents (C₃-C₆)-cycloalkyl or (C₅-C₆)-cycloalkenyl, each of which is optionally mono- or polysubstituted by identical or different substituents, possible substituents being in each case: (C₃-C₆)-cycloalkyl, (C₁-C₄) -alkyl, (C₁-C₄)-haloalkyl, (C₁-C₄) -alkoxy, (C₁-C₄) -haloalkoxy, halogen or cyano,
or represents phenyl, pyridyl, pyrimidyl, pyridazinyl, thiophenyl, furanyl, pyrazolyl, pyrrolyl, thiazolyl, oxazolyl or imidazolyl, each of which is optionally mono- or polysubstituted by identical or different substituents, possible substituents being in each case: cyano, halogen, nitro, acetyl, hydroxy, amino, SF₅-, (C₃-C₆)-cycloalkyl, (C₁-C₄) -alkyl- (C₃-C₆)-cycloalkyl, halo-(C₃-C₆)-cycloalkyl, (C₁-C₄) -alkyl, (C₁-C₄) -haloalkyl, (C₁-C₄) -cyanoalkyl, (C₁-C₄) -hydroxyalkyl, (C₁-C₄)-alkoxy-(C₁-C₂)-alkyl, (C₂-C₄)-alkenyl, (C₂-C₄)-haloalkenyl, (C₂-C₄)-cyanoalkenyl, (C₃-C₆)-cycloalkyl-(C₂)-alkenyl, (C₂-C₄)-alkynyl, (C₂-C₄)-haloalkynyl, (C₂-C₄)-cyanoalkynyl, (C₁-C₄) -alkoxy, (C₁-C₄)-haloalkoxy, (C₁-C₄) -cyanoalkoxy, (C₁-C₄) -alkoxy- (C₁-C₂)-alkoxy, (C₁-C₄) -alkoxyimino, (C₁-C₄)-haloalkoxyimino, (C₁-C₄)-alkylthio, (C₁-C₄)-haloalkylthio, (C₁-C₄) -alkylthio- (C₁-C₂)-alkyl, (C₁-C₄)-alkylsulfinyl, (C₁-C₄) -haloalkylsulfinyl, (C₁-C₄)-alkylsulfonyl, (C₁-C₄)-haloalkylsulfonyl, (C₁-C₄)-alkylsulfonyloxy, (C₁-C₄)-haloalkylsulfonyloxy, (C₁-C₄)-alkylcarbonyl, (C₁-C₄)-haloalkylcarbonyl, aminocarbonyl, (C₁-C₄)-alkylaminocarbonyl, (C₁-C₄)-haloalkylaminocarbonyl, di-(C₁-C₄)-alkylaminocarbonyl, (C₃-C₆)-cycloalkylaminocarbonyl, aminothiocarbonyl, (C₁-C₄)-alkylaminothiocarbonyl, di-(C₁-C₄) -alkylaminothiocarbonyl, (C₁-C₄)-haloalkylaminothiocarbonyl, (C₃-C₆)-cycloalkylaminothiocarbonyl, (C₁-C₄)-alkylsulfonylamino, (C₁-C₄) -alkylamino, di-(C₁-C₄)-alkylamino, (C₁-C₄) -haloalkylamino, (C₃-C₆)-cycloalkylamino, aminosulfonyl, (C₁-C₄)-alkylaminosulfonyl, di-(C₁-C₄)-alkylaminosulfonyl, (C₁-C₄) -alkylcarbonylamino, (C₁-C₄)-haloalkylcarbonylamino, (C₁-C₄)-alkylcarbonyl-(C₁-C₂)-alkylamino, (C₁-C₂)-haloalkylcarbonyl-(C₁-C₂)-alkylamino, (C₃-C₆)-cycloalkylcarbonylamino, (C₃-C₆)-cycloalkylcarbonyl- (C₁-C₂)-alkylamino, (C₁-C₄)-alkylthiocarbonylamino, (C₁-C₄)-haloalkylthiocarbonylamino, (C₁-C₄)-alkylthiocarbonyl- (C₁-C₂)-alkylamino, (C₁-C₄)-haloalkylthiocarbonyl-(C₁-C₂)-alkylamino, (C₃-C₆)-cycloalkylthiocarbonylamino or (C₃-C₆)-cycloalkylthiocarbonyl-(C₁-C₂)-alkylamino.

3. Process according to Claim 1, **characterized in that**
Q represents a structural element from the group of Q2, Q3, Q4, Q10, Q11, Q13 or Q14, where
R⁷ represents (C₁-C₄) -alkyl or (C₁-C₄) -alkoxy- (C₁-C₄)-alkyl and
A represents bromine, trifluoromethyl, fluoroethyl (CH₂CFH₂, CHFCH₃), difluoroethyl (CF₂CH₃, CH₂CHF₂, CHFCFH₂), trifluoroethyl, (CH₂CF₃, CHFCHF₂, CF₂CFH₂), tetrafluoroethyl (CHFCF₃, CF₂CHF₂), pentafluoroethyl, trifluoromethylthio, trifluoromethylsulfinyl or trifluoromethylsulfonyl,
W represents halogen or S(O)ₙR⁸, where
R⁸ represents methyl, ethyl, n-propyl or isopropyl and
n represents 0, 1 or 2,
R² represents chlorine,
X represents bromine or iodine, in particular iodine,
V represents methyl, ethyl, n-propyl or isopropyl and
Y represents hydrogen, bromine, iodine, ethenyl, cyclopropylethenyl, isopropenyl, cyclopropylethynyl, methyl, ethyl, isopropyl, cyclopropylethyl, methoxycarbonyl, trifluoroethylaminocarbonyl, aminocarbonyl, methylaminocarbonyl, dimethylaminocarbonyl, ethylaminocarbonyl, aminothiocarbonyl, methylaminothiocarbonyl, dimethylaminothiocarbonyl,
or represents cyclopropyl, cyclobutyl, cyclopentyl, cyclohexyl, cyclopentenyl or cyclohexenyl, each of which is optionally mono- or disubstituted by identical or different substituents, possible substituents being in each case: methyl, ethyl, n-propyl, isopropyl, cyclopropyl, difluoromethyl, trifluoromethyl, cyano, fluorine or chlorine,
or represents phenyl, pyridin-2-yl, pyridin-3-yl, pyridin-4-yl, pyrimidin-5-yl, pyridazin-3-yl, pyridazin-4-yl, thien-2-yl, thien-3-yl, 1,3-thiazol-5-yl, 1H-imidazol-1-yl, 1H-imidazol-2-yl, 1H-imidazol-5-yl, 1H-pyrazol-1-yl, 1H-pyrazol-3-yl, 1H-pyrazol-4-yl, 1H-pyrazol-5-yl, 1H-pyrrol-2-yl, 1H-pyrrol-3-yl or 1-cyclohexenyl, each of which is optionally mono-, di- or trisubstituted by identical or different substituents, possible substituents being in each case: cyano, fluorine, chlorine, methyl, cyclopropyl, cyanomethyl, cyanoisopropyl, cyanocyclopropyl, trifluoromethyl, trifluoroethyl or aminocarbonyl.

4. Process according to Claim 1, **characterized in that**
Q represents the structural element Q2, Q3 or Q13, where
R⁷ represents methyl, ethyl, n-propyl or isopropyl, in particular methyl, and
A represents bromine, trifluoromethyl, pentafluoroethyl or trifluoromethylthio,
W represents S(O)ₙR⁸, where
R⁸ represents ethyl and
n represents 0 or 2,
R² represents chlorine,
X represents iodine,
V represents methyl and
Y represents hydrogen, bromine, cyclopropyl, para-chlorophenyl, 5-chlorothien-2-yl or 5-chloro-2-pyridine.

5. Process according to Claim 1, **characterized in that** Q represents Q3 and R⁷, A, W, R², X, V and Y have the meanings given in Claim 1 or in Claim 2 or in Claim 3 or in Claim 4.

6. Process according to Claim 1, **characterized in that** Q represents Q13 and R⁷, A, W, R², X, V and Y have the meanings given in Claim 1 or in Claim 2 or in Claim 3 or in Claim 4.

7. Process according to Claim 1, **characterized in that** W represents S(O)ₙR⁸ and Q, n, R⁷, R⁸, A, R², X, V and Y have the meanings given in Claim 1 or in Claim 2 or in Claim 3 or in Claim 4.

8. Process according to any of Claims 1 to 4,
**characterized in that**
R³ and R⁴ together form a -(CH₂)₅- group substituted by 4 methyl groups.

9. Process according to any of Claims 1 to 8, **characterized in that** the organozinc base is a compound of the formula (V)
(V) (TMP)ₓ ZnCl₂₋ₓ ,
in which x represents the number 1 or 2.

10. Process according to any of Claims 1 to 9, **characterized in that** the organozinc base is present in conjunction with an alkali metal halide or alkaline earth metal halide, preferably lithium chloride or magnesium chloride.

11. Process according to any of Claims 1 to 10, **characterized in that** the organozinc base is used in a total amount of 0.5 to 5.0 equivalents, based on the compound Q-H.

12. Process according to any of Claims 1 to 11, **characterized in that** the compound of the formula (I) is used in a total amount of 0.5 to 10 equivalents, based on the compound Q-H.

13. Process according to any of Claims 1 to 12, **characterized in that** the catalyst is a palladium compound.

14. Process according to any of Claims 1 to 13, **characterized in that** the catalyst is tetrakis(triphenylphosphine)palladium(0).

15. Process according to any of Claims 1 to 14, **characterized in that** it is carried out in the presence of a solvent selected from the group consisting of tetrahydrofuran (THF), 1,4-dioxane, diethyl ether, diglyme, methyl tert-butyl ether (MTBE), tert-amyl methyl ether (TAME), 2-methyl-THF, toluene, xylenes, mesitylene, ethylene carbonate, propylene carbonate, N,N-dimethylacetamide, N,N-dimethylformamide (DMF), N-methylpyrrolidone (NMP), N-ethyl-2-pyrrolidone (NEP), N-butyl-2-pyrrolidone (NBP); N,N'-dimethylpropyleneurea (DMPU), halohydrocarbon, aromatic hydrocarbon, chlorohydrocarbon, tetrachloroethylene, tetrachloroethane, dichloropropane, methylene chloride, dichlorobutane, chloroform, carbon tetrachloride, trichloroethane, trichloroethylene, pentachloroethane, difluorobenzene, 1,2-dichloroethane, chlorobenzene, bromobenzene, dichlorobenzene, 1,2-dichlorobenzene, chlorotoluene, trichlorobenzene; 4-methoxybenzene, fluorinated aliphatic, fluorinated aromatic, trichlorotrifluoroethane, benzotrifluoride and 4-chlorobenzotrifluoride, or a mixture of at least two of these solvents with one another.

16. Process according to Claim 15, **characterized in that** the solvent is THF or N,N-dimethylformamide (DMF).

17. Process according to any of Claims 1 to 16, **characterized in that** process step a) is carried out at a temperature between 0°C and 80°C.

18. Process according to any of Claims 1 to 16, **characterized in that** process step b) is carried out at a temperature between 40°C and 90°C.

## Revendications

1. Procédé pour la préparation de composés de formule (II) 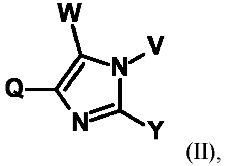
dans laquelle
Q représente un élément structural 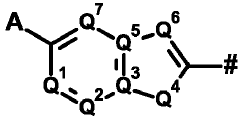
où le symbole # montre la liaison au reste de la molécule et
Q¹ représente N ou CR⁶,
Q² représente N ou CR⁶,
Q³ représente N ou C,
Q⁴ représente O, S, N, CR⁶ ou NR⁷,
Q⁵ représente N ou C,
Q⁶ représente N ou CH et
Q⁷ représente N ou CH,
au maximum cinq des variables Q¹, Q², Q³, Q⁴, Q⁵, Q⁶ et Q⁷ représentant simultanément azote et Q³ et Q⁵ ne représentant pas simultanément N et
R⁶ représentant hydrogène, (C₁-C₄) alkyle, (C₁-C₄)halogénoalkyle, (C₁-C₄) cyanoalkyle, (C₁-C₄)alcoxy- (C₁-C₄) alkyle, (C₁-C₄)halogénoalcoxy-(C₁-C₄)alkyle, (C₂-C₄)alcényle, (C₂-C₄)alcényloxy-(C₁-C₄)alkyle, (C₂-C₄)halogénoalcényloxy-(C₁-C₄) alkyle, (C₂-C₄)halogénoalcényle, (C₂-C₄)cyanoalcényle, (C₂-C₄)alcynyle, (C₂-C₄)alcynyloxy- (C₂-C₄)alkyle, (C₂-C₄)halogénoalcynyle, (C₃-C₆)cycloalkyle, (C₃-C₆)cycloalkyl-(C₃-C₆)cycloalkyle, (C₁-C₄) alkyl- (C₃-C₆)cycloalkyle, halogène (C₃-C₆)cycloalkyle, (C₁-C₄)alkylthio-(C₁-C₄) alkyle, (C₁-C₄)alkylsulfinyl-(C₁-C₄)alkyle, (C₁-C₄) alkylsulfonyl-(C₁-C₄) alkyle ou (C₁-C₄)alkylcarbonyl-(C₁-C₄) alkyle,
R⁷ représentant (C₁-C₄) alkyle, (C₁-C₄) halogénoalkyle, (C₁-C₄) cyanoalkyle, (C₁-C₄) alcoxy- (C₁-C₄) alkyle, (C₁-C₄)halogénoalcoxy-(C₁-C₄) alkyle, (C₂-C₄)alcényle, (C₂-C₄)alcényloxy- (C₁-C₄) alkyle, (C₂-C₄)halogénoalcényloxy-(C₁-C₄)alkyle, (C₂-C₄)halogénoalcényle, (C₂-C₄)cyanoalcényle, (C₂-C₄)alcynyle, (C₂-C₄)alcynyloxy- (C₁-C₄) alkyle, (C₂-C₄)halogénoalcynyle, (C₃-C₆)cycloalkyle, (C₃-C₆)cycloalkyl-(C₃-C₆)cycloalkyle, (C₁-C₄)alkyl-(C₃-C₆)cycloalkyle, halogène (C₃-C₆)cycloalkyle, (C₁-C₄)alkylthio-(C₁-C₄) alkyle, (C₁-C₄)alkylsulfinyl-(C₁-C₄)alkyle, (C₁-C₄) alkylsulfonyl-(C₁-C₄) alkyle ou (C₁-C₄)alkylcarbonyl-(C₁-C₄) alkyle et
A représente hydrogène, cyano, halogène, (C₁-C₄)alkyle, (C₁-C₄) halogénoalkyle, (C₂-C₄)alcényle, (C₂-C₄)halogénoalcényle, (C₂-C₄)alcynyle, (C₂-C₄)halogénoalcynyle, (C₃-C₆)cycloalkyle, (C₃-C₆)cycloalkyl-(C₃-C₆)cycloalkyle, (C₁-C₄)alkyl-(C₃-C₆)cycloalkyle, (C₁-C₄) alcoxy, (C₁-C₄)halogénoalcoxy, (C₁-C₄) alcoxyimino, (C₁-C₄)alkylthio, (C₁-C₄) halogénoalkylthio, (C₁-C₄)alkylsulfinyle, (C₁-C₄)halogénoalkylsulfinyle, (C₁-C₄) alkylsulfonyle, (C₁-C₄)halogénoalkylsulfonyle, (C₁-C₄)alkylsulfonyloxy, (C₁-C₄) alkylcarbonyle, (C₁-C₄)halogénoalkylcarbonyle, aminocarbonyle, (C₁-C₄)alkylaminocarbonyle, di-(C₁-C₄)alkylaminocarbonyle, (C₁-C₄)alkylsulfonylamino, (C₁-C₄)alkylamino, di-(C₁-C₄)alkylamino, aminosulfonyle, (C₁-C₄)alkylaminosulfonyle ou di-(C₁-C₄)alkylaminosulfonyle ou
A représente -O-CF₂-O- et forme, conjointement avec Q¹ et l'atome de carbone auquel il est lié, un cycle à cinq chaînons, Q¹ représentant carbone,
W représente halogène ou S(O)ₙR⁸,
R⁸ représentant (C₁-C₆)alkyle, (C₁-C₆)cyanoalkyle, (C₁-C₆)alcoxy(C₁-C₆)alkyle, (C₁-C₆)halogénoalkyle, (C₂-C₆)alcényle, (C₂-C₆)halogénoalcényle, (C₂-C₆)alcynyle, (C₂-C₆)halogénoalcynyle ou (C₃-C₈)cycloalkyle et
n représentant 0, 1 ou 2,
V représente (C₁-C₆)-alkyle, (C₁-C₆)halogénoalkyle, (C₁-C₆)cyanoalkyle, (C₁-C₆)alcoxy-(C₁-C₆)alkyle, (C₁-C₆)halogénoalcoxy-(C₁-C₆)alkyle, (C₂-C₆)alcényle, (C₂-C₆)alcényloxy- (C₁-C₆)alkyle, (C₂-C₆)halogénoalcényloxy-(C₁-C₆)alkyle, (C₂-C₆)halogénoalcényle, (C₂-C₆)cyanoalcényle, (C₂-C₆)alcynyle, (C₂-C₆)alcynyloxy-(C₁-C₆)alkyle, (C₂-C₆)halogénoalcynyloxy-(C₁-C₆)alkyle, (C₂-C₆)halogénoalcynyle, (C₂-C₆)cyanoalcynyle, (C₃-C₈)cycloalkyle, (C₃-C₈)cycloalkyl- (C₃-C₈)cycloalkyle, (C₁-C₆)alkyl-(C₃-C₈)cycloalkyle, halogéno (C₃-C₈)cycloalkyle, cyano(C₃-C₈)cycloalkyle, (C₁-C₆)alkylthio-(C₁-C₆)alkyle, (C₁-C₆)halogénoalkylthio-(C₁-C₆)alkyle, (C₁-C₆)alkylsulfinyl-(C₁-C₆)alkyle, (C₁-C₆)halogénoalkylsulfinyl-(C₁-C₆)alkyle, (C₁-C₆)alkylsulfonyl-(C₁-C₆)alkyle, (C₁-C₆)halogénoalkylsulfonyl-(C₁-C₆)alkyle, (C₁-C₆)alkylcarbonyl-(C₁-C₆)alkyle, (C₁-C₆)halogénoalkylcarbonyl-(C₁-C₆)alkyle, (C₁-C₆)alcoxycarbonyl-(C₁-C₆)alkyle ou (C₁-C₆)halogénoalcoxycarbonyl-(C₁-C₆)alkyle et
Y représente hydrogène, halogène, (C₁-C₄)alkyle, (C₁-C₄)halogénoalkyle, (C₁-C₄)alcoxy, (C₁-C₄)halogénoalcoxy, (C₁-C₄)alkylthio, (C₁-C₄)halogénoalkylthio, (C₁-C₄)alkylsulfinyle, (C₁-C₄)halogénoalkylsulfinyle, (C₁-C₄)alkylsulfonyle, (C₁-C₄)halogénoalkylsulfonyle, SCN, (C₁-C₄)alkylcarbonyle, (C₁-C₄)halogénoalkylcarbonyle, (C₁-C₄)alcoxycarbonyle, (C₁-C₄)halogénoalcoxycarbonyle, aminocarbonyle, (C₁-C₄)alkylaminocarbonyle, di-(C₁-C₄)alkylaminocarbonyle, (C₁-C₄)halogénoalkylaminocarbonyle, (C₃-C₆)cycloalkylaminocarbonyle, aminothiocarbonyle, (C₁-C₄)alkylaminothiocarbonyle, di-(C₁-C₄)alkylaminothiocarbonyle, (C₁-C₄)halogénoalkylaminothiocarbonyle, (C₃-C₆)cycloalkylaminothiocarbonyle, amino, (C₁-C₄)alkylamino, (C₁-C₄)halogénoalkylamino, di-(C₁-C₄)alkylamino, (C₃-C₆)cycloalkylamino, (C₁-C₄)alkylsulfonylamino, (C₁-C₄)alkylcarbonylamino, (C₁-C₄)halogénoalkylcarbonylamino, (C₁-C₄)alkylcarbonyl-(C₁-C₄)alkylamino, (C₁-C₄)halogénoalkylcarbonyl-(C₁-C₄)alkylamino, (C₃-C₆)cycloalkylcarbonylamino, (C₃-C₆)cycloalkylcarbonyl-(C₁-C₄)alkylamino, (C₁-C₄)alkylthiocarbonylamino, (C₁-C₄)halogénoalkylthiocarbonylamino, (C₁-C₄)alkylthiocarbonyl-(C₁-C₄)alkylamino, (C₁-C₄)halogénoalkylthiocarbonyl-(C₁-C₄)alkylamino, (C₃-C₆)cycloalkylthiocarbonylamino, (C₃-C₆)cycloalkylthiocarbonyl-(C₁-C₄)alkylamino, (C₂-C₄)alcényle, (C₂-C₄)halogénoalcényle, (C₂-C₄)cyanoalcényle, (C₃-C₆)cycloalkyl- (C₂)alcényle, (C₂-C₄)alcynyle ou (C₂-C₄)halogénoalcynyle,
ou représente (C₃-C₆)cycloalkyle ou (C₅-C₆)cycloalcényle à chaque fois le cas échéant monosubstitué ou polysubstitué, de manière identique ou différente, les substituants qui entrent à chaque fois en ligne de compte étant : (C₃-C₆)cycloalkyle, (C₁-C₄)alkyle, (C₁-C₄)halogénoalkyle, (C₁-C₄)alcoxy, (C₁-C₄)halogénoalcoxy, aminocarbonyle, aminothiocarbonyle, halogéno ou cyano,
ou représente aryle ou hétéroaryle, à chaque fois le cas échéant monosubstitué ou polysubstitué, de manière identique ou différente, où (dans le cas de hétéroaryle), le cas échéant au moins un groupe carbonyle peut être contenu et où les substituants qui entrent à chaque fois en ligne de compte sont : cyano, carboxyle, halogène, nitro, acétyle, hydroxy, amino, SCN, SF₅, tri-(C₁-C₄)alkylsilyle, (C₃-C₆)cycloalkyle, (C₃-C₆)cycloalkyl- (C₃-C₆)cycloalkyle, (C₁-C₄)alkyl-(C₃-C₆)cycloalkyle, (C₁-C₄)halogénoalkyl-(C₃-C₆)cycloalkyle, halogéno-(C₃-C₆)cycloalkyle, cyano- (C₃-C₆)cycloalkyle, (C₁-C₄)alkyle, (C₁-C₄)halogénoalkyle, (C₁-C₄)cyanoalkyle, (C₁-C₄)hydroxyalkyle, hydroxycarbonyl-(C₁-C₄)-alcoxy, (C₁-C₄)alcoxycarbonyl-(C₁-C₄)alkyle, (C₁-C₄)alcoxy- (C₁-C₄)alkyle, (C₂-C₄)alcényle, (C₂-C₄)halogénoalcényle, (C₂-C₄)cyanoalcényle, (C₃-C₆)cycloalkyl-(C₂)alcényle, (C₂-C₄)alcynyle, (C₂-C₄)halogénoalcynyle, (C₂-C₄)cyanoalcynyle, (C₁-C₄)alcoxy, (C₁-C₄)halogénoalcoxy, (C₁-C₄)cyanoalcoxy, (C₁-C₄)alcoxycarbonyl-(C₁-C₄)alcoxy, (C₁-C₄)alcoxy- (C₁-C₄)alcoxy, (C₁-C₄)alcoxyimino, (C₁-C₄)halogénoalcoxyimino, (C₁-C₄)alkylthio, (C₁-C₄)halogénoalkylthio, (C₁-C₄)alcoxy- (C₁-C₄)alkylthio, (C₁-C₄)alkylthio-(C₁-C₄)alkyle, (C₁-C₄)alkylsulfinyle, (C₁-C₄)halogénoalkylsulfinyle, (C₁-C₄)alcoxy- (C₁-C₄)alkylsulfinyle, (C₁-C₄)alkylsulfinyl-(C₁-C₄)alkyle, (C₁-C₄)alkylsulfonyle, (C₁-C₄)halogénoalkylsulfonyle, (C₁-C₄)alcoxy- (C₁-C₄)alkylsulfonyle, (C₁-C₄)alkylsulfonyl-(C₁-C₄)alkyle, (C₁-C₄)alkylsulfonyloxy, (C₁-C₄)halogénoalkylsulfonyloxy, (C₁-C₄)alkylcarbonyle, (C₁-C₄)halogénoalkylcarbonyle, (C₁-C₄)alkylcarbonyloxy, (C₁-C₄)alcoxycarbonyle, (C₁-C₄)halogénoalcoxycarbonyle, aminocarbonyle, (C₁-C₄)alkylaminocarbonyle, (C₁-C₄)halogénoalkylaminocarbonyle, di-(C₁-C₄)alkylaminocarbonyle, (C₂-C₄)alcénylaminocarbonyle, di-(C₂-C₄)-alcénylaminocarbonyle, (C₃-C₆)cycloalkylaminocarbonyle, (C₁-C₄)alkylsulfonylamino, (C₁-C₄)alkylamino, di-(C₁-C₄)alkylamino, (C₁-C₄)halogénoalkylamino, (C₃-C₆)cycloalkylamino, aminosulfonyle, (C₁-C₄)alkylaminosulfonyle, di-(C₁-C₄)alkylaminosulfonyle, (C₁-C₄)alkylsulfoximino, aminothiocarbonyle, (C₁-C₄)alkylaminothiocarbonyle, di-(C₁-C₄)alkylaminothiocarbonyle, (C₁-C₄)halogénoalkylaminothiocarbonyle, (C₃-C₆)cycloalkylaminothiocarbonyle, (C₁-C₄)alkylcarbonylamino, (C₁-C₄)halogénoalkylcarbonylamino, (C₁-C₄)alkylcarbonyl-(C₁-C₄)alkylamino, (C₁-C₄)halogénoalkylcarbonyl-(C₁-C₄)alkylamino, (C₃-C₆)cycloalkylcarbonylamino, (C₃-C₆)cycloalkylcarbonyl-(C₁-C₄)alkylamino, (C₁-C₄)alkylthiocarbonylamino, (C₁-C₄)halogénoalkylthiocarbonylamino, (C₁-C₄)alkylthiocarbonyl-(C₁-C₄)alkylamino, (C₁-C₄)halogénoalkylthiocarbonyl-(C₁-C₄)alkylamino, (C₃-C₆)cycloalkylthiocarbonylamino, (C₃-C₆)cycloalkylthiocarbonyl-(C₁-C₄)alkylamino, hétéroaryle, oxohétéroaryle, halogénohétéroaryle, halogéno-oxohétéroaryle, cyanohétéroaryle, cyano-oxohétéroaryle, (C₁-C₄)halogénoalkylhétéroaryle ou (C₁-C₄)halogénoalkyloxohétéroaryle,
**caractérisé en ce qu'**on transforme, dans une première étape de procédé a), un composé Q-H, dans lequel Q présente la signification susmentionnée,
avec une base organométallique de zinc de structure (NR³R⁴)-Zn-R² ou (NR³R⁴)₂-Zn, dans laquelle
R² représente halogène ou -O-pivaloyle et
R³ et R⁴ représentent, conjointement, un groupe -(CH₂)₄-, -(CH₂)₅- ou -(CH₂)₂O(CH₂)₂-, chacun de ces groupes pouvant éventuellement être substitué par 1, 2, 3 ou 4 radicaux R⁵ et R⁵ étant choisi dans le groupe constitué par méthyle, éthyle, n-propyle et i-propyle, en un composé de formule (IIIa) ou un composé de formule (IIIb), 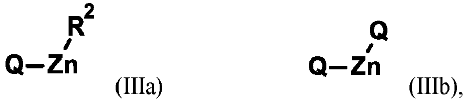
dans lesquelles Q et R² ont à chaque fois les significations susmentionnées,
et on transforme ce composé de formule (IIIa) ou (IIIb) dans une deuxième étape de procédé b) avec un composé de formule (I) 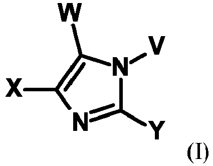
dans laquelle X représente halogène et V, W et Y présentent à chaque fois les significations susmentionnées, en présence d'un catalyseur, en composé de formule (II).

2. Procédé selon la revendication 1, **caractérisé en ce que** Q représente un élément structural de la série Q1 à Q14, 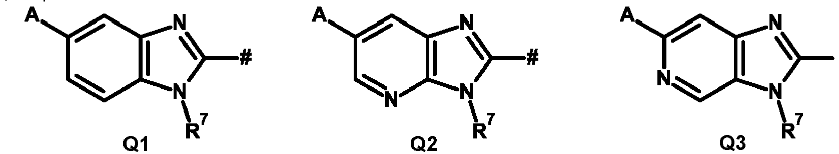 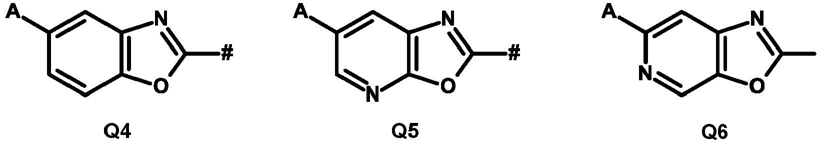 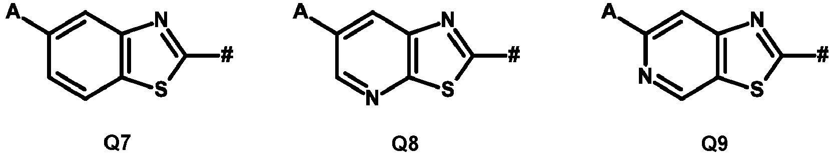 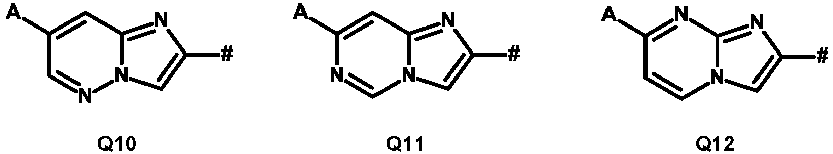 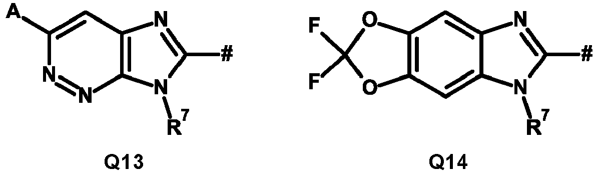 où
R⁷ représente (C₁-C₄)alkyle, (C₁-C₄)halogénoalkyle, (C₁-C₄)alcoxy- (C₁-C₄)alkyle, (C₁-C₄)halogénoalcoxy-(C₁-C₄)alkyle, (C₁-C₄)alkylthio-(C₁-C₄)alkyle, (C₁-C₄)alkylsulfinyl-(C₁-C₄)alkyle, (C₁-C₄)alkylsulfonyl-(C₁-C₄)alkyle ou (C₁-C₄)alkylcarbonyl-(C₁-C₄)alkyle et
A représente fluor, chlore, brome, fluorométhyle, difluorométhyle, trifluorométhyle, fluoroéthyle (CH₂CFH₂, CHFCH₃), difluoroéthyle (CF₂CH₃, CH₂CHF₂, CHFCFH₂), trifluoroéthyle (CH₂CF₃, CHFCHF₂, CF₂CFH₂), tétrafluoroéthyle (CHFCF₃, CF₂CHF₂), pentafluoroéthyle, trifluorométhoxy, difluorochlorométhoxy, dichlorofluorométhoxy, trifluorométhylthio, trifluorométhylsulfinyle ou trifluorométhylsulfonyle,
W représente halogène ou S(O)ₙR⁸,
R⁸ représentant (C₁-C₆)alkyle, (C₁-C₄)cyanoalkyle, (C₁-C₄)alcoxy- (C₁-C₄)alkyle, (C₁-C₆)halogénoalkyle, (C₂-C₄)alcényle, (C₂-C₄)halogénoalcényle, (C₂-C₄)alcynyle, (C₂-C₄)halogénoalcynyle ou (C₃-C₆)cycloalkyle et
n représentant 0, 1 ou 2,
R² représente halogène, en particulier chlore, brome ou iode,
X représente halogène, en particulier brome ou iode,
V représente (C₁-C₆)alkyle, (C₁-C₄)halogénoalkyle, (C₁-C₄)cyanoalkyle, (C₁-C₄)alcoxy- (C₁-C₄)alkyle, (C₁-C₄)halogénoalcoxy-(C₁-C₄)alkyle, (C₃-C₆)cycloalkyle, (C₃-C₆)cycloalkyl- (C₃-C₆)cycloalkyle, (C₁-C₄)alkyl-(C₃-C₆)cycloalkyle, halogéno (C₃-C₆)cycloalkyle, cyano(C₃-C₆)cycloalkyle, (C₁-C₄)alkylthio-(C₁-C₄)alkyle, (C₁-C₄)halogénoalkylthio-(C₁-C₄)alkyle, (C₁-C₄)alkylsulfinyl-(C₁-C₄)alkyle, (C₁-C₄)halogénoalkylsulfinyl-(C₁-C₄)alkyle, (C₁-C₄)alkylsulfonyl-(C₁-C₄)alkyle ou (C₁-C₄)halogénoalkylsulfonyl-(C₁-C₄)alkyle et
Y représente hydrogène, halogène, (C₁-C₄)alkyle, (C₁-C₄)halogénoalkyle, (C₁-C₄)-alcoxy, (C₁-C₄)halogénoalcoxy, aminocarbonyle, (C₁-C₄)alkylaminocarbonyle, di-(C₁-C₄)alkylaminocarbonyle, (C₁-C₄)halogénoalkylaminocarbonyle, (C₃-C₆)cycloalkylaminocarbonyle, amino, (C₁-C₄)alkylamino, (C₁-C₄)halogénoalkylamino, di-(C₁-C₄)alkylamino, (C₃-C₆)cycloalkylamino, (C₁-C₄)alkylsulfonylamino, (C₁-C₄)alkylcarbonylamino, (C₁-C₄)halogénoalkylcarbonylamino, (C₁-C₄)alkylcarbonyl-(C₁-C₂)alkylamino, (C₁-C₄)halogénoalkylcarbonyl-(C₁-C₂)alkylamino, (C₃-C₆)cycloalkylcarbonylamino, (C₃-C₆)cycloalkylcarbonyl-(C₁-C₂)alkylamino, (C₂-C₄)alcényle, (C₂-C₄)halogénoalcényle, (C₂-C₄)cyanoalcényle ou (C₃-C₆)cycloalkyl- (C₂)alcényle,
ou représente (C₃-C₆)cycloalkyle ou (C₅-C₆)cycloalcényle à chaque fois le cas échéant monosubstitué ou polysubstitué, de manière identique ou différente, les substituants qui entrent à chaque fois en ligne de compte étant : (C₃-C₆)cycloalkyle, (C₁-C₄)alkyle, (C₁-C₄)halogénoalkyle, (C₁-C₄)alcoxy, (C₁-C₄)halogénoalcoxy, halogène ou cyano,
ou représente phényle, pyridyle, pyrimidyle, pyridazinyle, thiophényle, furannyle, pyrazolyle, pyrrolyle, thiazolyle, oxazolyle ou imidazolyle à chaque fois le cas échéant monosubstitué ou polysubstitué, de manière identique ou différente, les substituants qui entrent à chaque fois en ligne de compte étant : cyano, halogène, nitro, acétyle, hydroxy, amino, SF₅-, (C₃-C₆)cycloalkyle, (C₁-C₄)alkyl- (C₃-C₆)cycloalkyle, halogéno(C₃-C₆)cycloalkyle, (C₁-C₄)alkyle, (C₁-C₄)halogénoalkyle, (C₁-C₄)cyanoalkyle, (C₁-C₄)hydroxyalkyle, (C₁-C₄)alcoxy- (C₁-C₂)alkyle, (C₂-C₄)alcényle, (C₂-C₄)halogénoalcényle, (C₂-C₄)cyanoalcényle, (C₃-C₆)cycloalkyl- (C₂)alcényle, (C₂-C₄)alcynyle, (C₂-C₄)halogénoalcynyle, (C₂-C₄)cyanoalcynyle, (C₁-C₄)alcoxy, (C₁-C₄)halogénoalcoxy, (C₁-C₄)cyanoalcoxy, (C₁-C₄)alcoxy- (C₁-C₂)alcoxy, (C₁-C₄)alcoxyimino, (C₁-C₄)halogénoalcoxyimino, (C₁-C₄)alkylthio, (C₁-C₄)halogénoalkylthio, (C₁-C₄)alkylthio-(C₁-C₂)alkyle, (C₁-C₄)alkylsulfinyle, (C₁-C₄)halogénoalkylsulfinyle, (C₁-C₄)alkylsulfonyle, (C₁-C₄)halogénoalkylsulfonyle, (C₁-C₄)alkylsulfonyloxy, (C₁-C₄)halogénoalkylsulfonyloxy, (C₁-C₄)alkylcarbonyle, (C₁-C₄)halogénoalkylcarbonyle, aminocarbonyle, (C₁-C₄)alkylaminocarbonyle, (C₁-C₄)halogénoalkylaminocarbonyle, di-(C₁-C₄)alkylaminocarbonyle, (C₃-C₆)cycloalkylaminocarbonyle, aminothiocarbonyle, (C₁-C₄)alkylaminothiocarbonyle, di-(C₁-C₄)alkylaminothiocarbonyle, (C₁-C₄)halogénoalkylaminothiocarbonyle, (C₃-C₆)cycloalkylaminothiocarbonyle, (C₁-C₄)alkylsulfonylamino, (C₁-C₄)alkylamino, di-(C₁-C₄)alkylamino, (C₁-C₄)halogénoalkylamino, (C₃-C₆)cycloalkylamino, aminosulfonyle, (C₁-C₄)alkylaminosulfonyle, di-(C₁-C₄)alkylaminosulfonyle, (C₁-C₄)alkylcarbonylamino, (C₁-C₄)halogénoalkylcarbonylamino, (C₁-C₄)alkylcarbonyl-(C₁-C₂)alkylamino, (C₁-C₂)halogénoalkylcarbonyl-(C₁-C₂)alkylamino, (C₃-C₆)cycloalkylcarbonylamino, (C₃-C₆)cycloalkylcarbonyl-(C₁-C₂)alkylamino, (C₁-C₄)alkylthiocarbonylamino, (C₁-C₄)halogénoalkylthiocarbonylamino, (C₁-C₄)alkylthiocarbonyl-(C₁-C₂)alkylamino, (C₁-C₄)halogénoalkylthiocarbonyl-(C₁-C₂)alkylamino, (C₃-C₆)cycloalkylthiocarbonylamino ou (C₃-C₆)cycloalkylthiocarbonyl-(C₁-C₂)alkylamino.

3. Procédé selon la revendication 1, **caractérisé en ce que** Q représente un élément structural de la série Q2, Q3, Q4, Q10, Q11, Q13 ou Q14, où
R⁷ représente (C₁-C₄)alkyle ou (C₁-C₄)alcoxy- (C₁-C₄)alkyle et
A représente brome, trifluorométhyle, fluoroéthyle (CH₂CFH₂, CHFCH₃), difluoroéthyle (CF₂CH₃, CH₂CHF₂, CHFCFH₂), trifluoroéthyle (CH₂CF₃, CHFCHF₂, CF₂CFH₂), tétrafluoroéthyle (CHFCF₃, CF₂CHF₂), pentafluoroéthyle, trifluorométhylthio, trifluorométhylsulfinyle ou trifluorométhylsulfonyle,
W représente halogène ou S(O)ₙR⁸, où
R⁸ représente méthyle, éthyle, n-propyle ou iso-propyle et
n représente 0, 1 ou 2,
R² représente chlore,
X représente brome ou iode, en particulier iode,
V représente méthyle, éthyle, n-propyle ou iso-propyle et
Y représente hydrogène, brome, iode, éthényle, cyclopropyléthényle, i-propényle, cyclopropyléthynyle, méthyle, éthyle, isopropyle, cyclopropyléthyle, méthoxycarbonyle, trifluoroéthylaminocarbonyle, aminocarbonyle, méthylaminocarbonyle, diméthylaminocarbonyle, éthylaminocarbonyle, aminothiocarbonyle, méthylaminothiocarbonyle, diméthylaminothiocarbonyle,
ou représente cyclopropyle, cyclobutyle, cyclopentyle, cyclohexyle, cyclopentényle ou cyclohexényle à chaque fois le cas échéant monosubstitué ou disubstitué, de manière identique ou différente, les substituants qui entrent à chaque fois en ligne de compte étant : méthyle, éthyle, n-propyle, i-propyle, cyclopropyle, difluorométhyle, trifluorométhyle, cyano, fluor ou chlore,
ou représente phényle, pyridin-2-yle, pyridin-3-yle, pyridin-4-yle, pyrimidine-5-yle, pyridazin-3-yle, pyridazin-4-yle, thién-2-yle, thién-3-yle, 1,3-thiazol-5-yle, 1H-imidazol-1-yle, 1H-imidazol-2-yle, 1H-imidazol-5-yle, 1H-pyrazol-1-yle, 1H-pyrazol-3-yle, 1H-pyrazol-4-yle, 1H-pyrazol-5-yle, 1H-pyrrol-2-yle, 1H-pyrrol-3-yle ou 1-cyclohexényle à chaque fois le cas échéant monosubstitué, disubstitué ou trisubstitué, de manière identique ou différente, les substituants qui entrent à chaque fois en ligne de compte étant : cyano, fluor, chlore, méthyle, cyclopropyle, cyanométhyle, cyano-isopropyle, cyanocyclopropyle, trifluorométhyle, trifluoroéthyle ou aminocarbonyle.

4. Procédé selon la revendication 1, **caractérisé en ce que** Q représente l'élément structural Q2, Q3 ou Q13, où
R⁷ représente méthyle, éthyle, n-propyle ou iso-propyle, en particulier méthyle et
A représente brome, trifluorométhyle, pentafluoroéthyle ou trifluorométhylthio,
W représente S(O)ₙR⁸, où
R⁸ représente éthyle et
n représente 0 ou 2,
R² représente chlore,
X représente iode,
V représente méthyle et
Y représente hydrogène, brome, cyclopropyle, para-chlorophényle, 5-chlorothién-2-yle ou 5-chloro-2-pyridine.

5. Procédé selon la revendication 1, **caractérisé en ce que** Q représente Q3 et R⁷, A, W, R², X, V et Y présentent les significations indiquées dans la revendication 1 ou dans la revendication 2 ou dans la revendication 3 ou dans la revendication 4.

6. Procédé selon la revendication 1, **caractérisé en ce que** Q représente Q13 et R⁷, A, W, R², X, V et Y présentent les significations indiquées dans la revendication 1 ou dans la revendication 2 ou dans la revendication 3 ou dans la revendication 4.

7. Procédé selon la revendication 1, **caractérisé en ce que** W représente S(O)ₙR⁸ et Q, n, R⁷, R⁸, A, R², X, V et Y présentent les significations indiquées dans la revendication 1 ou dans la revendication 2 ou dans la revendication 3 ou dans la revendication 4.

8. Procédé selon l'une quelconque des revendications 1 à 4, **caractérisé en ce que** R³ et R⁴ forment conjointement un groupe -(CH₂)₅-, qui est substitué par 4 groupes méthyle.

9. Procédé selon l'une quelconque des revendications 1 à 8, **caractérisé en ce qu'**il s'agit, pour la base organométallique de zinc, d'un composé de formule (V)
(V) (TMP)ₓZnCl₂₋ₓ,
dans laquelle x représente le nombre 1 ou 2.

10. Procédé selon l'une quelconque des revendications 1 à 9, **caractérisé en ce que** la base organométallique de zinc se trouve en association avec un halogénure de métal alcalin ou alcalino-terreux, de préférence le chlorure de lithium ou le chlorure de magnésium.

11. Procédé selon l'une quelconque des revendications 1 à 10, **caractérisé en ce que** la base organométallique de zinc est utilisée en une quantité totale de 0,5 à 5,0 équivalents, par rapport au composé Q-H.

12. Procédé selon l'une quelconque des revendications 1 à 11, **caractérisé en ce que** le composé de formule (I) est utilisé en une quantité totale de 0,5 à 10 équivalents, par rapport au composé Q-H.

13. Procédé selon l'une quelconque des revendications 1 à 12, **caractérisé en ce qu'**il s'agit, pour le catalyseur, d'un composé du palladium.

14. Procédé selon l'une quelconque des revendications 1 à 13, **caractérisé**
**en ce qu'**il s'agit, pour le catalyseur, de tétrakis(triphénylphosphine)-palladium(0).

15. Procédé selon l'une quelconque des revendications 1 à 14, **caractérisé en ce qu'**il est réalisé en présence d'un solvant, qui est choisi dans le groupe constitué par le tétrahydrofuranne (THF), le 1,4-dioxane, le diéthyléther, le diglyme, le méthyl-tert-butyléther (MTBE), le tert-amylméthyléther (TAME), le 2-méthyl-THF, le toluène, les xylènes, le mésitylène, le carbonate d'éthylène, le carbonate de propylène, le N,N-diméthylacétamide, le N,N-diméthylformamide (DMF), la N-méthylpyrrolidone (NMP), la N-éthyl-2-pyrrolidone (NEP), N-butyl-2-pyrrolidone (NBP) ; la N,N'-diméthylpropylène-urée (DMPU), les hydrocarbures halogénés, les hydrocarbures aromatiques, les hydrocarbures chlorés, le tétrachloroéthylène, le tétrachloroéthane, le dichloropropane, le chlorure de méthylène, le dichlorobutane, le chloroforme, le tétrachlorure de carbone, le trichloroéthane, le trichloroéthylène, le pentachloroéthane, le difluorobenzène, le 1,2-dichloroéthane, le chlorobenzène, le bromobenzène, le dichlorobenzène, le 1,2-dichlorobenzène, le chlorotoluène, le trichlorobenzène ; le 4-méthoxybenzène, un aliphate fluoré, un aromate fluoré, le trichlorotrifluoroéthane, le benzotrifluorure et le 4-chlorobenzotrifluorure ou un mélange d'au moins deux de ces solvants l'un avec l'autre.

16. Procédé selon la revendication 15, **caractérisé en ce que** le solvant est le THF ou le N,N-diméthylformamide (DMF).

17. Procédé selon l'une quelconque des revendications 1 à 16, **caractérisé en ce que** l'étape de procédé a) est réalisée à une température entre 0 et 80°C.

18. Procédé selon l'une quelconque des revendications 1 à 16, **caractérisé en ce que** l'étape de procédé b) est réalisée à une température entre 40 et 90°C.
